# EUROPEAN PATENT APPLICATION

(11) **EP 4 438 053 A2**
(43) Date of publication of application: **02.10.2024**
(21) Application number: 24186643.3
(22) Date of filing: 17.09.2020
(51) Int. Cl.: A61K 38/00

(54) **AN ANAPLASTIC LYMPHOMA KINASE (ALK) CANCER VACCINE AND METHODS OF USE**

(30) Priority: 18.09.2019 US 201962902096 P
(62) Divisional of application: 20864504.4
(71) Applicant: Children's Medical Center Corporation, Boston, MA 02115 (US)
(72) Inventor: CHIARLE, Roberto, Boston, MA, 02115 (US); BLASCO-PATINO, Rafael, Boston, MA, 02115 (US)
(74) Representative: Simmons & Simmons LLP (Munich)

(57) **Abstract**

Provided herein are isolated anaplastic lymphoma kinase (ALK) peptides that are fragments of the cytoplasmic portion of an ALK protein shared by cancers having an ALK rearrangement and cancers expressing the ALK protein, that bind a human leukocyte antigen (HLA), and elicit an immune response against one or more ALK-positive cancers. Also provided are isolated ALK peptides that are modified with an amphiphilic conjugate to increase T-cell expansion and greatly enhance anti-tumor efficacy. The invention also provides polynucleotides encoding isolated ALK peptides, vaccines comprising an isolated ALK peptide or polynucleotide, immunogenic compositions thereof, and kits for administering the same. Methods of treatment and methods of generating an immune response in a subject by administering the ALK-specific peptide antigens, immunogens, vaccines, or immunogenic compositions thereof are provided.

## Description

### CROSS REFERENCE TO RELATED APPLICATIONS

This application is an International PCT Application which claims priority to and the benefit of U.S. Provisional Application No. 62/902,096, filed September 18, 2019, the entire contents of which are hereby incorporated by reference in its entirety.

### BACKGROUND OF THE INVENTION

Anaplastic lymphoma kinase (ALK) is a receptor tyrosine kinase in the insulin receptor superfamily, which plays an important role in the development of the brain and nervous system. ALK is processed into peptides by the proteasome, transported to the endoplasmic reticulum by transporters associated with antigen processing-1 and -2 (TAP1 and TAP2), and binds to the HLA class I molecules. ALK is minimally expressed by normal tissues during adulthood. However, ALK is aberrantly expressed by tumors, such as non-small cell lung cancer (NSCLC), anaplastic large cell lymphoma (ALCL) and neuroblastoma. More rarely, ALK is expressed by B-cell lymphoma, thyroid cancer, colon cancer, breast cancer, inflammatory myofibroblastic tumors (IMT), renal carcinoma, esophageal cancer, and melanoma. Thus, ALK is an ideal shared antigen across different type of cancers. ALK may become oncogenic by forming a fusion gene with other genes, by gaining additional gene copies, or by genetic mutations. For example, a 2;5 chromosomal translocation creates a fusion gene consisting of the *ALK* gene and the *nucleophosmin* (*NPM*) gene. The NPM-ALK translocation is associated with approximately 60% anaplastic large-cell lymphomas (ALCLs).

Lung cancer is the most common cause of cancer-related death worldwide, and the annual incidence of ALK-positive non-small cell lung cancers (NSCLCs) in the U.S. is about 8,000 cases. EMI,4-ALK positive lung cancer is a primary malignant lung tumor where the *echinoderm microtubule-associate protein-like 4 (EML4)* gene is fused to the *ALK* gene. About 3-7% of NSCLCs harbor an EML4-ALK-rearrangement in the *ALK* gene.

Several ALK tyrosine kinase inhibitors (TKIs) are available for the treatment of ALK-rearranged NSCLC, including crizotinib, ceritinib, alectinib, brigatinib, and lorlatinib. Unfortunately, resistance to these drugs occurs within 1-2 years through a variety of mechanisms. Once patients develop resistance to available ALK inhibitors, they are typically treated with cytotoxic chemotherapy rather than immunotherapy since response rates to PD-1 pathway inhibitors in this population are very low. Although PD-1 inhibitors such as pembrolizumab (Keytruda^{®}) and nivolumab (Opdivo^{®}) have revolutionized the treatment of lung cancer in general, particularly in smoking-associated cancers, most patients with ALK-positive lung cancer do not respond to these immunotherapies. Thus, the development of additional ALK-targeted therapy in ALK-positive NSCLCs and other tumors is clearly a need.

### INCORPORATION BY REFERENCE

All publications, patents, and patent applications mentioned in this specification are herein incorporated by reference to the same extent as if each individual publication, patent, or patent application was specifically and individually indicated to be incorporated by reference. Absent any indication otherwise, publications, patents, and patent applications mentioned in this specification are incorporated herein by reference in their entireties

### SUMMARY OF THE INVENTION

As described below, the present invention features isolated anaplastic lymphoma kinase (ALK) peptides that are fragments of the cytoplasmic portion of an ALK protein shared by cancers having an ALK rearrangement and cancers expressing the ALK protein, that bind a human leukocyte antigen (HLA), and elicit an immune response against one or more ALK-positive cancers. The isolated ALK peptides may be modified with an amphiphilic conjugate to increase T-cell expansion and greatly enhance anti-tumor efficacy. Also provided are polynucleotides encoding isolated ALK peptides, vaccines comprising an isolated ALK peptide or polynucleotide, immunogenic compositions thereof, and kits for administering the same. Methods of treatment and methods of generating an immune response in a subject by administering the ALK-specific peptide antigens, immunogens, vaccines, or immunogenic compositions thereof are provided.

In one aspect, the invention provides an isolated anaplastic lymphoma kinase (ALK) peptide wherein the peptide: i) is a fragment of the cytoplasmic portion of an ALK protein shared by cancers having an ALK rearrangement and cancers expressing the ALK protein; ii) is capable of binding a human leukocyte antigen (HLA); and iii) is capable of generating an immune response against one or more ALK-positive cancers, wherein the peptide does not include the amino acid sequence AMLDLLHVA. In some embodiments, the ALK peptide is capable of generating an immune response against one or more ALK-positive cancers, wherein the ALK peptide comprises the amino acid sequence RPRPSQPSSL. In some embodiments, the ALK peptide is capable of generating an immune response against one or more ALK-positive cancers, wherein the ALK peptide comprises the amino acid sequence IVRCIGVSL. In some embodiments, the ALK peptide is capable of generating an immune response against one or more ALK-positive cancers, wherein the ALK peptide comprises the amino acid sequence VPRKNITLI. In some embodiments, the ALK peptide is capable of generating an immune response against one or more ALK-positive cancers, wherein the ALK peptide comprises the amino acid sequence TAAEVSVRV.

In some embodiments, the peptide comprises an amino acid sequence that has at least about 95% identity to a sequence listed in Table 3, Table 4, or Table 5. In some embodiments, the peptide comprises or consists of an amino acid sequence selected from those listed in Table 3, Table 4, or Table 5. In some embodiments, the peptide comprises an amino acid sequence that has at least about 95% identity to an amino acid sequence selected from the following: RPRPSQPSSL; IVRCIGVSL; VPRKNITLI; or TAAEVSVRV.

In some embodiments, the ALK antigen is conjugated to an amphiphile. In some embodiments, the amphiphile is N-hydroxy succinimidyl ester-end-functionalized poly(ethylene glycol)-lipid (NHS-PEG2KDa-DSPE). In some embodiments, the one or more ALK-positive cancers is selected from the group consisting of non-small cell lung cancer (NSCLC), anaplastic large cell lymphoma (ALCL), neuroblastoma, B-cell lymphoma, thyroid cancer, colon cancer, breast cancer, inflammatory myofibroblastic tumors (IMT), renal carcinoma, esophageal cancer, and melanoma. In some embodiments, the one or more ALK-positive cancers is non-small cell lung cancer (NSCLC). In some embodiments, the one or more ALK-positive cancers is anaplastic large cell lymphoma (ALCL).

In some embodiments, the HLA is encoded by a HLA class I allele. In some embodiments, the HLA class I allele is selected from those listed in Table 1, Table 2, or Table 3. In some embodiments, the HLA class I allele is HLA A*02:01, HLA A*68:02, HLA B*07:02, or HLA C*07:02. In some embodiments, the ALK protein is a full-length human ALK protein. In some embodiments, the ALK rearrangement is a nucleophosmin-ALK rearrangement (NPM-ALK) or an echinoderm microtubule-associate protein-like 4-ALK rearrangement (EMI,4-ALK).

In one aspect, the invention provides a polynucleotide encoding for an ALK peptide. In another aspect, the invention provides a vaccine comprising a polynucleotide encoding for an ALK peptide. In yet another aspect, the invention provides a vaccine comprising an ALK peptide.

In one aspect, the invention provides a vaccine comprising two or more isolated synthetic anaplastic lymphoma kinase (ALK) peptides capable of generating an immune response against one or more ALK-positive cancers, wherein the two or more isolated ALK peptides are selected from those listed in Table 3, Table 4, or Table 5. In one embodiment, the two or more ALK peptides comprise two or more amino acid sequences selected from the following: AMLDLLHVA; RPRPSQPSSL; IVRCIGVSL; VPRKNITLI; and/or TAAEVSVRV. In some embodiments, the two or more ALK peptides comprise at least one of the following combinations: AMLDLLHVA and RPRPSQPSSL; AMLDLLHVA and IVRCIGVSL; AMLDLLHVA and VPRKNITLI; AMLDLLHVA and TAAEVSVRV; RPRPSQPSSL and IVRCIGVSL; RPRPSQPSSL and VPRKNITLI; RPRPSQPSSL and TAAEVSVRV; IVRCIGVSL and VPRKNITLI; IVRCIGVSL and TAAEVSVRV; VPRKNITLI and TAAEVSVRV; AMLDLLHVA and RPRPSQPSSL and IVRCIGVSL; AMLDLLHVA and RPRPSQPSSL and VPRKNITLI; AMLDLLHVA and RPRPSQPSSL and TAAEVSVRV; AMLDLLHVA and IVRCIGVSL and VPRKNITLI; AMLDLLHVA and IVRCIGVSL and TAAEVSVRV; AMLDLLHVA and VPRKNITLI and TAAEVSVRV; RPRPSQPSSL and IVRCIGVSL and VPRKNITLI; RPRPSQPSSL; IVRCIGVSL and TAAEVSVRV; RPRPSQPSSL and VPRKNITLI and TAAEVSVRV; IVRCIGVSL and VPRKNITLI and TAAEVSVRV; AMLDLLHVA and RPRPSQPSSL and IVRCIGVSL and VPRKNITLI; AMLDLLHVA and RPRPSQPSSL and IVRCIGVSL and TAAEVSVRV; AMLDLLHVA and IVRCIGVSL and VPRKNITLI and TAAEVSVRV; AMLDLLHVA and RPRPSQPSSL and VPRKNITLI and TAAEVSVRV; RPRPSQPSSL and IVRCIGVSL and VPRKNITLI and TAAEVSVRV; and/or AMLDLLHVA and RPRPSQPSSL and IVRCIGVSL and VPRKNITLI and TAAEVSVRV.

In another embodiment, at least one of the two or more ALK peptides is conjugated to an amphiphile. In another embodiment, the amphiphile is N-hydroxy succinimidyl ester-end-functionalized poly(ethylene glycol)-lipid (NHS-PEG2KDa-DSPE).

In one aspect, the invention provides an immunogenic composition comprising a vaccine and a pharmaceutically acceptable carrier, diluent, or excipient. In one embodiment, the immunogenic composition further comprises an adjuvant. In some embodiments, the adjuvant is a synthetic complex of carboxymethylcellulose, polyinosinic-polycytidylic acid, and poly-L-lysine double-stranded RNA (poly ICLC) or CpG oligonucleotides. In some embodiments, the adjuvant is conjugated to an amphiphile. In some embodiments, the amphiphile is N-hydroxy succinimidyl ester-end-functionalized poly(ethylene glycol)-lipid (NHS-PEG2KDa-DSPE).

In one aspect, the invention provides a method of treating an ALK-positive cancer in a subject by administering to the subject an effective amount of an ALK peptide, vaccine, or immunogenic composition. In another aspect, the invention provides a method of treating a subject with one or more an ALK-positive cancers by administering to the subject an effective amount of an ALK peptide, vaccine, or immunogenic composition. In some embodiments, the methods of treating further comprise administering, simultaneously or sequentially, to the subject an effective amount of one or more of an ALK inhibitor, immune checkpoint inhibitor, and/or tyrosine kinase inhibitor (TKI). In some embodiments, the immune checkpoint inhibitor is selected from one or more of a programmed cell death protein 1 (PD-1) inhibitor, programmed death-ligand 1 (PD-L1) inhibitor, or cytotoxic T-lymphocyte-associated antigen-4 (CTLA-4) inhibitor. In some embodiments, the PD-1 inhibitor is an anti-PD1 antibody. In some embodiments, the ALK inhibitor is lorlatinib. In some embodiments, an anti-PD1 antibody is administered in combination with lorlatinib. In some embodiments, an adjuvant is concomitantly administered to the subject.

In some embodiments, the ALK-positive cancer is selected from the group consisting of non-small cell lung cancer (NSCLC), anaplastic large cell lymphoma (ALCL), neuroblastoma, B-cell lymphoma, thyroid cancer, colon cancer, breast cancer, inflammatory myofibroblastic tumors (IMT), renal carcinoma, esophageal cancer, and melanoma. In some embodiments, the ALK-positive cancer is non-small cell lung cancer (NSCLC). In some embodiments, the ALK-positive cancer is anaplastic large cell lymphoma (ALCL).

In some embodiments, the subject had at least one prior treatment with at least one tyrosine kinase inhibitor (TKI). In some embodiments, the step of administering comprises one or more doses of the ALK peptide, the vaccine, or the immunogenic composition. In some embodiments, the step of administering comprises at least six (6) prime doses and at least two (2) booster doses of the ALK peptide, the vaccine, or the immunogenic composition.

In one aspect, the invention provides a method of generating an immune response in a subject by administering to the subject an effective amount of an ALK peptide, vaccine, or immunogenic composition. In some embodiments, the immune response produces T-lymphocytes.

In another aspect, the invention provides a kit comprising an agent for administration to a subject with one or more ALK-positive cancers, wherein the agent comprises an ALK peptide, vaccine, or immunogenic composition.

Compositions and articles defined by the invention were isolated or otherwise manufactured in connection with the examples provided below. Other features and advantages of the invention will be apparent from the detailed description, and from the claims.

### Definitions

Unless defined otherwise, all technical and scientific terms used herein have the meaning commonly understood by a person skilled in the art to which this invention pertains or relates. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); Benjamin Lewin, Genes V, published by Oxford University Press, 1994 (ISBN 0-19-854287-9); Kendrew et al. (eds.); The Encyclopedia of Molecular Biology, published by Blackwell Science Ltd., 1994 (ISBN 0-632-02182-9); Molecular Biology and Biotechnology: a Comprehensive Desk Reference, Robert A. Meyers (ed.), published by VCH Publishers, Inc., 1995 (ISBN 1-56081-569-8); and Hale & Marham, The Harper Collins Dictionary of Biology (1991). As used herein, the following terms have the meanings ascribed to them below, unless specified otherwise.

By "adjuvant" is meant a substance or vehicle that non-specifically enhances the immune response to an antigen. Adjuvants may include a suspension of minerals (e.g., alum, aluminum hydroxide, or phosphate) on which antigen is adsorbed; or water-in-oil emulsion in which antigen solution is emulsified in mineral oil *(e.g.,* Freund's incomplete adjuvant), sometimes with the inclusion of killed mycobacteria (Freund's complete adjuvant) to further enhance antigenicity. Immunostimulatory oligonucleotides (such as those including a CpG motif) can also be used as adjuvants (*see, e.g.,* U.S. Patent Nos. 6,194,388; 6,207,646; 6,214,806; 6,218,371; 6,239,116; 6,339,068; 6,406,705; and 6,429,199). Adjuvants also include biological molecules, such as costimulatory molecules. Exemplary biological adjuvants include, without limitation, interleukin-1 (IL-2), the protein memory T-cell attractant "Regulated on Activation, Normal T Expressed and Secreted" (RANTES), granulocyte-macrophage-colony stimulating factor (GM-CSF), tumor necrosis factor-alpha (TNF-α), interferon-gamma (IFN-γ), granulocyte-colony stimulation factor (G-CSF), lymphocyte function-associated antigen 3 (LFA-3, also called CD58), cluster of differentiation antigen 72 (CD72), (a negative regulator of B-cell responsiveness), peripheral membrane protein, B7-1 (B7-1, also called CD80), peripheral membrane protein, B7-2 (B7-2, also called CD86), the TNF ligand superfamily member 4 ligand (OX40L) or the type 2 transmembrane glycoprotein receptor belonging to the TNF superfamily (4-1BBL). In some embodiments, the adjuvant may be conjugated to an amphiphile as described in H. Liu et al., Structure-based programming of lymph-node targeting in molecular vaccines. Nature 507, 5199522 (2014). In some embodiments, the amphiphile conjugated to the adjuvant is N-hydroxy succinimidyl ester-end-functionalized poly(ethylene glycol)-lipid (NHS-PEG2KDa-DSPE).

By "administer" is meant giving, supplying, or dispensing a composition, agent, therapeutic and the like to a subject, or applying or bringing the composition and the like into contact with the subject. Administering or administration may be accomplished by any of a number of routes, such as, for example, without limitation, topical, oral, subcutaneous, intramuscular, intraperitoneal, intravenous (IV), injection, intrathecal, intramuscular, dermal, intradermal, intracranial, inhalation, rectal, intravaginal, or intraocular.

By "agent" is meant any small molecule chemical compound, antibody, nucleic acid molecule, peptide, polypeptide, or fragments thereof.

By "ALK polypeptide" or "ALK peptide" is meant a protein or fragment thereof having an amino acid sequence that is at least 85%, at least 90%, at least 95%, at least 98%, at least 99%, or 100% identical to an anaplastic lymphoma kinase (ALK) amino acid sequence associated with GenBank Accession No.: BAD92714.1, ACY79563, or ACI47591, which is capable of inducing an ALK-specific immune response in an immunized subject. In some embodiments, the ALK polypeptide is at least 85%, at least 90%, at least 95%, at least 98%, at least 99%, or 100% identical to the ALK protein in *Homo Sapiens.* An exemplary ALK full-length amino acid sequence from *Homo Sapiens* is provided below (ALK cytoplasmic portion in bold font):

TASSGGMGAIGLLWLLPLLLSTAAVGSGMGTGQRAGSPAAGPPLQPREPLSYSRLQRKSLAVDF
VVPSLFRVYARDLLLPPSSSELKAGRPEARGSLALDCAPLLRLLGPAPGVSWTAGSPAPAEART
LSRVLKGGSVRKLRRAKQLVLELGEEAILEGCVGPPGEAAVGLLQFNLSELFSWWIRQGEGRLR
IRLMPEKKASEVGREGRLSAAIRASQPRLLFQIFGTGHSSLESPTNMPSPSPDYFTWNLTWIMK
DSFPFLSHRSRYGLECSFDFPCELEYSPPLHDLRNQSWSWRRIPSEEASQMDLLDGPGAERSKE
MPRGSFLLLNTSADSKHTILSPWMRSSSEHCTLAVSVHRHLQPSGRYIAQLLPHNEAAREILLM
PTPGKHGWTVLQGRIGRPDNPFRVALEYISSGNRSLSAVDFFALKNCSEGTSPGSKMALQSSFT
CWNGTVLQLGQACDFHQDCAQGEDESQMCRKLPVGFYCNFEDGFCGWTQGTLSPHTPQWQVRTL
KDARFQDHQDHALLLSTTDVPASESATVTSATFPAPIKSSPCELRMSWLIRGVLRGNVSLVLVE
NKTGKEQGRMVWHVAAYEGLSLWQWMVLPLLDVSDRFWLQMVAWWGQGSRAIVAFDNISISLDC
YLTISGEDKILQNTAPKSRNLFERNPNKELKPGENSPRQTPIFDPTVHWLFTTCGASGPHGPTQ
AQCNNAYQNSNLSVEVGSEGPLKGIQIWKVPATDTYSISGYGAAGGKGGKNTMMRSHGVSVLGI
FNLEKDDMLYILVGQQGEDACPSTNQLIQKVCIGENNVIEEEIRVNRSVHEWAGGGGGGGATY
VFKMKDGVPVPLIIAAGGGGRAYGAKTDTFHPERLENNSSVLGLNGNSGAAGGGGGWNDNTSLL
WAGKSLQEGATGGHSCPQAMKKWGWETRGGFGGGGGGCSSGGGGGGYIGGNAASNNDPEMDGED
GVSFISPLGILYTPALKVMEGHGEVNIKHYLNCSHCEVDECHMDPESHKVICFCDHGTVLAEDG
VSCIVSPTPEPHLPLSLILSVVTSALVAALVLAFSGIMIVYRRKHQELQAMQMELQSPEYKLSK
LRTSTIMTDYNPNYCFAGKTSSISDLKEVPRKNITLIRGLGHGAFGEVYEGQVSGMPNDPSPLQ

VAVKTLPEVCSEQDELDFLMEALIISKFNHQNIVRCIGVSLQSLPRFILLELMAGGDLKSFLRE
TRPRPSQPSSLAMLDLLHVARDIACGCQYLEENHFIHRDIAARNCLLTCPGPGRVAKIGDFGMA
RDIYRASYYRKGGCAMLPVKWMPPEAFMEGIFTSKTDTWSFGVLLWEIFSLGYMPYPSKSNQEV
LEFVTSGGRMDPPKNCPGPVYRIMTQCWQHQPEDRPNFAIILERIEYCTQDPDVINTALPIEYG
PLVEEEEKVPVRPKDPEGVPPLLVSQQAKREEERSPAAPPPLPTTSSGKAAKKPTAAEISVRVP
RGPAVEGGHVNMAFSQSNPPSELHKVHGSRNKPTSLWNPTYGSWFTEKPTKKNNPIAKKEPHDR
GNLGLEGSCTVPPNVATGRLPGASLLLEPSSLTANMKEVPLFRLRHFPCGNVNYGYQQQGLPLE
AATAPGAGHYEDTILKSKNSMNQPGP

An exemplary full-length ALK amino acid sequence from *Homo Sapiens* is provided below:

G A A V V A A G T S R R L C S E G R G
A P R C F P A A L W S A T Q S R G R * *
W V R R G R Q D F G R P C P E R P Q L L
P P G P L Q C L R T L R S R G A G E S K
D A A N L R S A G A G I H A Q K F S R Q
T V R S L P A A E R * L E G A Q D G S L
R P R F P P R P G R R A W R S Q K E R K
R R P G Q R A A A G S R R S Q P * K L Q
R L E A A P R G D R P Q L R L R G A G E
D G T Q L P P P F N H S S S S V P S A A
S Y R R G R G T R R G E R E A Q G P S Q
* A Q C A * V S L D S P L S F Q V C F I
* T P A R L R A V G G K Q E T C A H A Q
S S G D Q V E G A A G Y Q G L F R A S S
H L G E S E G * G W A R R A V * T A S S
G G M G A I G L L W L L P L L L S T A A
V G S G M G T G Q R A G S P A A G P P L
Q P R E P L S Y S R L Q R K S L A V D F
V V P S L F R V Y A R D L L L P P S S S
E L K A G R P E A R G S L A L D C A P L
L R L L G P A P G V S W T A G S P A P A
E A R T L S R V L K G G S V R K L R R A
K Q L V L E L G E E A I L E G C V G P P
G E A A V G L L Q F N L S E L F S W W I
R Q G E G R L R I R L M P E K K A S E V
G R E G R L S A A I R A S Q P R L L F Q
I F G T G H S S L E S P T N M P S P S P
D Y F T W N L T W I M K D S F P F L S H
R S R Y G L E C S F D F P C E L E Y S P

P L H D L R N Q S W S W R R I P S E E A
S Q M D L L D G P G A E R S K E M P R G
S F L L L N T S A D S K H T I L S P W M
R S S S E H C T L A V S V H R H L Q P S
G R Y I A Q L L P H N E A A R E I L L M
P T P G K H G W T V L Q G R I G R P D N
P F R V A L E Y I S S G N R S L S A V D
F F A L K N C S E G T S P G S K M A L Q
S S F T C W N G T V L Q L G Q A C D F H
Q D C A Q G E D E S Q M C R K L P V G F
Y C N F E D G F C G W T Q G T L S P H T
P Q W Q V R T L K D A R F Q D H Q D H A
L L L S T T D V P A S E S A T V T S A T
F P A P I K S S P C E L R M S W L I R G
V L R G N V S L V L V E N K T G K E Q G
R M V W H V A A Y E G L S L W Q W M V L
P L L D V S D R F W L Q M V A W W G Q G
S R A I V A F D N I S I S L D C Y L T I
S G E D K I L Q N T A P K S R N L F E R
N P N K E L K P G E N S P R Q T P I F D
P T V H W L F T T C G A S G P H G P T Q
A Q C N N A Y Q N S N L S V E V G S E G
P L K G I Q I W K V P A T D T Y S I S G
Y G A A G G K G G K N T M M R S H G V S
V L G I F N L E K D D M L Y I L V G Q Q
G E D A C P S T N Q L I Q K V C I G E N
N V I E E E I R V N R S V H E W A G G G
G G G G G A T Y V F K M K D G V P V P L
I I A A G G G G R A Y G A K T D T F H P
E R L E N N S S V L G L N G N S G A A G
G G G G W N D N T S L L W A G K S L Q E
G A T G G H S C P Q A M K K W G W E T R
G G F G G G G G G C S S G G G G G G Y I
G G N A A S N N D P E M D G E D G V S F
I S P L G I L Y T P A L K V M E G H G E
V N I K H Y L N C S H C E V D E C H M D
P E S H K V I C F C D H G T V L A E D G
V S C I V S P T P E P H L P L S L I L S
V V T S A L V A A L V L A F S G I M I V
Y R R K H Q E L Q A M Q M E L Q S P E Y
K L S K L R T S T I M T D Y N P N Y C F

A G K T S S I S D L K E V P R K N I T L
I R G L G H G A F G E V Y E G Q V S G M
P N D P S P L Q V A V K T L P E V C S E
Q D E L D F L M E A L I I S K F N H Q N
I V R C I G V S L Q S L P R F I L L E L
M A G G D L K S F L R E T R P R P S Q P
S S L A M L D L L H V A R D I A C G C Q
Y L E E N H F I H R D I A A R N C L L T
C P G P G R V A K I G D F G M A R D I Y
R A S Y Y R K G G C A M L P V K W M P P
E A F M E G I F T S K T D T W S F G V L
L W E I F S L G Y M P Y P S K S N Q E V
L E F V T S G G R M D P P K N C P G P V
Y R I M T Q C W Q H Q P E D R P N F A I
I L E R I E Y C T Q D P D V I N T A L P
I E Y G P L V E E E E K V P V R P K D P
E G V P P L L V S Q Q A K R E E E R S P
A A P P P L P T T S S G K A A K K P T A
A E I S V R V P R G P A V E G G H V N M
A F S Q S N P P S E L H K V H G S R N K
P T S L W N P T Y G S W F T E K P T K K
N N P I A K K E P H D R G N L G L E G S
C T V P P N V A T G R L P G A S L L L E
P S S L T A N M K E V P L F R L R H F P
C G N V N Y G Y Q Q Q G L P L E A A T A
P G A G H Y E D T I L K S K N S M N Q P
G P * A R S H T H F S S L G S L R P W R
R E R Q W L L H K P E T K C H V L F C A
N L F * S T T K K A V F * K C F R K V L
S M G S S Y S F E R R K Y H K N E * * I
Q G P D V V A * G F Y A C L L Y T S L C
F F Q I V C A L L Q C S Q N * L L L C F
I V G V I D V S L P C * C G H E P F E G
R G N G N K G V I C N D *

An exemplary ALK polypeptide sequence is provided at UniProt Accession No. >sp|Q9UM73|ALK_HUMAN ALK tyrosine kinase receptor OS=Homo sapiens OX=9606 GN=ALK PE=1 SV=3

MGAIGLLWLLPLLLSTAAVGSGMGTGQRAGSPAAGPPLQPREPLSYSRLQRKSLAVDFVV
PSLFRVYARDLLLPPSSSELKAGRPEARGSLALDCAPLLRLLGPAPGVSWTAGSPAPAEA

RTLSRVLKGGSVRKLRRAKQLVLELGEEAILEGCVGPPGEAAVGLLQFNLSELFSWWIRQ
GEGRLRIRLMPEKKASEVGREGRLSAAIRASQPRLLFQIFGTGHSSLESPTNMPSPSPDY
FTWNLTWIMKDSFPFLSHRSRYGLECSFDFPCELEYSPPLHDLRNQSWSWRRIPSEEASQ
MDLLDGPGAERSKEMPRGSFLLLNTSADSKHTILSPWMRSSSEHCTLAVSVHRHLQPSGR
YIAQLLPHNEAAREILLMPTPGKHGWTVLQGRIGRPDNPFRVALEYISSGNRSLSAVDFF
ALKNCSEGTSPGSKMALQSSFTCWNGTVLQLGQACDFHQDCAQGEDESQMCRKLPVGFYC
NFEDGFCGWTQGTLSPHTPQWQVRTLKDARFQDHQDHALLLSTTDVPASESATVTSATFP
APIKSSPCELRMSWLIRGVLRGNVSLVLVENKTGKEQGRMVWHVAAYEGLSLWQWMVLPL
LDVSDRFWLQMVAWWGQGSRAIVAFDNISISLDCYLTISGEDKILQNTAPKSRNLFERNP
NKELKPGENSPRQTPIFDPTVHWLFTTCGASGPHGPTQAQCNNAYQNSNLSVEVGSEGPL
KGIQIWKVPATDTYSISGYGAAGGKGGKNTMMRSHGVSVLGIFNLEKDDMLYILVGQQGE
DACPSTNQLIQKVCIGENNVIEEEIRVNRSVHEWAGGGGGGGATYVFKMKDGVPVPLII
AAGGGGRAYGAKTDTFHPERLENNSSVLGLNGNSGAAGGGGGWNDNTSLLWAGKSLQEGA
TGGHSCPQAMKKWGWETRGGFGGGGGGCSSGGGGGGYIGGNAASNNDPEMDGEDGVSFIS
PLGILYTPALKVMEGHGEVNIKHYLNCSHCEVDECHMDPESHKVICFCDHGTVLAEDGVS
CIVSPTPEPHLPLSLILSVVTSALVAALVLAFSGIMIVYRRKHQELQAMQMELQSPEYKL
SKLRTSTIMTDYNPNYCFAGKTSSISDLKEVPRKNITLIRGLGHGAFGEVYEGQVSGMPN
DPSPLQVAVKTLPEVCSEQDELDFLMEALIISKFNHQNIVRCIGVSLQSLPRFILLELMA
GGDLKSFLRETRPRPSQPSSLAMLDLLHVARDIACGCQYLEENHFIHRDIAARNCLLTCP
GPGRVAKIGDFGMARDIYRASYYRKGGCAMLPVKWMPPEAFMEGIFTSKTDTWSFGVLLW
EIFSLGYMPYPSKSNQEVLEFVTSGGRMDPPKNCPGPVYRIMTQCWQHQPEDRPNFAIIL
ERIEYCTQDPDVINTALPIEYGPLVEEEEKVPVRPKDPEGVPPLLVSQQAKREEERSPAA
PPPLPTTSSGKAAKKPTAAEISVRVPRGPAVEGGHVNMAFSQSNPPSELHKVHGSRNKPT
SLWNPTYGSWFTEKPTKKNNPIAKKEPHDRGNLGLEGSCTVPPNVATGRLPGASLLLEPS
SLTANMKEVPLFRLRHFPCGNVNYGYQQQGLPLEAATAPGAGHYEDTILKSKNSMNQPGP

Exemplary ALK peptide amino acid sequences are provided in **Table 3, Table 4,** and **Table 5.**

An exemplary ALK peptide amino sequence is as follows: RPRPSQPSSL .

An exemplary ALK peptide amino sequence is as follows: IVRCIGVSL .

An exemplary ALK peptide amino sequence is as follows: VPRKNITLI.

An exemplary ALK peptide amino sequence is as follows: TAAEVSVRV.

An exemplary ALK peptide amino sequence is as follows: AMLDLLHVA.

By "ALK polynucleotide" is meant any nucleic acid molecule encoding an ALK polypeptide or fragment thereof (antigen or antigen protein). An exemplary full-length ALK nucleic acid sequence from *Homo Sapiens* is provided below:

2 gggcggcagcggtggtagcagctggtacctcccgccgcctctgttcggagggtcgcggg 61
62 gcaccgaggtgctttccggccgccctctggtcggccacccaaagccgcgggcgctgatga 121
122 tgggtgaggagggggcggcaagatttcgggcgcccctgccctgaacgccctcagctgctg 181
182 ccgccggggccgctccagtgcctgcgaactctgaggagccgaggcgccggtgagagcaag 241
242 gacgctgcaaacttgcgcagcgcgggggctgggattcacgcccagaagttcagcaggcag 301
302 acagtccgaagccttcccgcagcggagagatagcttgagggtgcgcaagacggcagcctc 361
362 cgccctcggttcccgcccagaccgggcagaagagcttggaggagccaaaaggaacgcaaa 421
422 aggcggccaggacagcgtgcagcagctgggagccgccgttctcagccttaaaagttgcag 481
482 agattggaggctgccccgagaggggacagaccccagctccgactgcggggggcaggagag 541
542 gacggtacccaactgccacctcccttcaaccatagtagttcctctgtaccgagcgcagcg 601
602 agctacagacgggggcgcggcactcggcgcggagagcgggaggctcaaggtcccagccag 661
662 tgagcccagtgtgcttgagtgtctctggactcgcccctgagcttccaggtctgtttcatt 721
722 tagactcctgctcgcctccgtgcagttgggggaaagcaagagacttgcgcgcacgcacag 781
782 tcctctggagatcaggtggaaggagccgctgggtaccaaggactgttcagagcctcttcc 841
842 catctcggggagagcgaagggtgaggctgggcccggagagcagtgtaaacggcctcctcc 901
902 ggcgggatgggagccatcgggctcctgtggctcctgccgctgctgctttccacggcagct 961
962 gtgggctccgggatggggaccggccagcgcgcgggctccccagctgcggggccgccgctg 1021
1022 cagccccgggagccactcagctactcgcgcctgcagaggaagagtctggcagttgacttc 1081
1082 gtggtgccctcgctcttccgtgtctacgcccgggacctactgctgccaccatcctcctcg 1141
1142 gagctgaaggctggcaggcccgaggcccgcggctcgctagctctggactgcgccccgctg 1201
1202 ctcaggttgctggggccggcgccgggggtctcctggaccgccggttcaccagccccggca 1261
1262 gaggcccggacgctgtccagggtgctgaagggcggctccgtgcgcaagctccggcgtgcc 1321
1322 aagcagttggtgctggagctgggcgaggaggcgatcttggagggttgcgtcgggcccccc 1381
1382 ggggaggcggctgtggggctgctccagttcaatctcagcgagctgttcagttggtggatt 1441
1442 cgccaaggcgaagggcgactgaggatccgcctgatgcccgagaagaaggcgtcggaagtg 1501
1502 ggcagagagggaaggctgtccgcggcaattcgcgcctcccagccccgccttctcttccag 1561
1562 atcttcgggactggtcatagctccttggaatcaccaacaaacatgccttctccttctcct 1621
1622 gattattttacatggaatctcacctggataatgaaagactccttccctttcctgtctcat 1681
1682 cgcagccgatatggtctggagtgcagctttgacttcccctgtgagctggagtattcccct 1741
1742 ccactgcatgacctcaggaaccagagctggtcctggcgccgcatcccctccgaggaggcc 1801
1802 tcccagatggacttgctggatgggcctggggcagagcgttctaaggagatgcccagaggc 1861
1862 tcctttctccttctcaacacctcagctgactccaagcacaccatcctgagtccgtggatg 1921
1922 aggagcagcagtgagcactgcacactggccgtctcggtgcacaggcacctgcagccctct 1981
1982 ggaaggtacattgcccagctgctgccccacaacgaggctgcaagagagatcctcctgatg 2041
2042 cccactccagggaagcatggttggacagtgctccagggaagaatcgggcgtccagacaac 2101
2102 ccatttcgagtggccctggaatacatctccagtggaaaccgcagcttgtctgcagtggac 2161
2162 ttctttgccctgaagaactgcagtgaaggaacatccccaggctccaagatggccctgcag 2221

2222 agctccttcacttgttggaatgggacagtcctccagcttgggcaggcctgtgacttccac 2281
2282 caggactgtgcccagggagaagatgagagccagatgtgccggaaactgcctgtgggtttt 2341
2342 tactgcaactttgaagatggcttctgtggctggacccaaggcacactgtcaccccacact 2401
2402 cctcaatggcaggtcaggaccctaaaggatgcccggttccaggaccaccaagaccatgct 2461
2462 ctattgctcagtaccactgatgtccccgcttctgaaagtgctacagtgaccagtgctacg 2521
2522 tttcctgcaccgatcaagagctctccatgtgagctccgaatgtcctggctcattcgtgga 2581
2582 gtcttgaggggaaacgtgtccttggtgctagtggagaacaaaaccgggaaggagcaaggc 2641
2642 aggatggtctggcatgtcgccgcctatgaaggcttgagcctgtggcagtggatggtgttg 2701
2702 cctctcctcgatgtgtctgacaggttctggctgcagatggtcgcatggtggggacaagga 2761
2762 tccagagccatcgtggcttttgacaatatctccatcagcctggactgctacctcaccatt 2821
2822 agcggagaggacaagatcctgcagaatacagcacccaaatcaagaaacctgtttgagaga 2881
2882 aacccaaacaaggagctgaaacccggggaaaattcaccaagacagacccccatctttgac 2941
2942 cctacagttcattggctgttcaccacatgtggggccagcgggccccatggccccacccag 3001
3002 gcacagtgcaacaacgcctaccagaactccaacctgagcgtggaggtggggagcgagggc 3061
3062 cccctgaaaggcatccagatctggaaggtgccagccaccgacacctacagcatctcgggc 3121
3122 tacggagctgctggcgggaaaggcgggaagaacaccatgatgcggtcccacggcgtgtct 3181
3182 gtgctgggcatcttcaacctggagaaggatgacatgctgtacatcctggttgggcagcag 3241
3242 ggagaggacgcctgccccagtacaaaccagttaatccagaaagtctgcattggagagaac 3301
3302 aatgtgatagaagaagaaatccgtgtgaacagaagcgtgcatgagtgggcaggaggcgga 3361
3362 ggaggagggggtggagccacctacgtatttaagatgaaggatggagtgccggtgcccctg 3421
3422 atcattgcagccggaggtggtggcagggcctacggggccaagacagacacgttccaccca 3481
3482 gagagactggagaataactcctcggttctagggctaaacggcaattccggagccgcaggt 3541
3542 ggtggaggtggctggaatgataacacttccttgctctgggccggaaaatctttgcaggag 3601
3602 ggtgccaccggaggacattcctgcccccaggccatgaagaagtgggggtgggagacaaga 3661
3662 gggggtttcggagggggtggaggggggtgctcctcaggtggaggaggcggaggatatata 3721
3722 ggcggcaatgcagcctcaaacaatgaccccgaaatggatggggaagatggggtttccttc 3781
3782 atcagtccactgggcatcctgtacaccccagctttaaaagtgatggaaggccacggggaa 3841
3842 gtgaatattaagcattatctaaactgcagtcactgtgaggtagacgaatgtcacatggac 3901
3902 cctgaaagccacaaggtcatctgcttctgtgaccacgggacggtgctggctgaggatggc 3961
3962 gtctcctgcattgtgtcacccaccccggagccacacctgccactctcgctgatcctctct 4021
4022 gtggtgacctctgccctcgtggccgccctggtcctggctttctccggcatcatgattgtg 4081
4082 taccgccggaagcaccaggagctgcaagccatgcagatggagctgcagagccctgagtac 4141
4142 aagctgagcaagctccgcacctcgaccatcatgaccgactacaaccccaactactgcttt 4201
4202 gctggcaagacctcctccatcagtgacctgaaggaggtgccgcggaaaaacatcaccctc 4261
4262 attcggggtctgggccatggcgcctttggggaggtgtatgaaggccaggtgtccggaatg 4321
4322 cccaacgacccaagccccctgcaagtggctgtgaagacgctgcctgaagtgtgctctgaa 4381
4382 caggacgaactggatttcctcatggaagccctgatcatcagcaaattcaaccaccagaac 4441
4442 attgttcgctgcattggggtgagcctgcaatccctgccccggttcatcctgctggagctc 4501
4502 atggcggggggagacctcaagtccttcctccgagagacccgccctcgcccgagccagccc 4561
4562 tcctccctggccatgctggaccttctgcacgtggctcgggacattgcctgtggctgtcag 4621
4622 tatttggaggaaaaccacttcatccaccgagacattgctgccagaaactgcctcttgacc 4681

4682 tgtccaggccctggaagagtggccaagattggagacttcgggatggcccgagacatctac 4741
4742 agggcgagctactatagaaagggaggctgtgccatgctgccagttaagtggatgccccca 4801
4802 gaggccttcatggaaggaatattcacttctaaaacagacacatggtcctttggagtgctg 4861
4862 ctatgggaaatcttttctcttggatatatgccataccccagcaaaagcaaccaggaagtt 4921
4922 ctggagtttgtcaccagtggaggccggatggacccacccaagaactgccctgggcctgta 4981
4982 taccggataatgactcagtgctggcaacatcagcctgaagacaggcccaactttgccatc 5041
5042 attttggagaggattgaatactgcacccaggacccggatgtaatcaacaccgctttgccg 5101
5102 atagaatatggtccacttgtggaagaggaagagaaagtgcctgtgaggcccaaggaccct 5161
5162 gagggggttcctcctctcctggtctctcaacaggcaaaacgggaggaggagcgcagccca 5221
5222 gctgccccaccacctctgcctaccacctcctctggcaaggctgcaaagaaacccacagct 5281
5282 gcagagatctctgttcgagtccctagagggccggccgtggaagggggacacgtgaatatg 5341
5342 gcattctctcagtccaaccctccttcggagttgcacaaggtccacggatccagaaacaag 5401
5402 cccaccagcttgtggaacccaacgtacggctcctggtttacagagaaacccaccaaaaag 5461
5462 aataatcctatagcaaagaaggagccacacgacaggggtaacctggggctggagggaagc 5521
5522 tgtactgtcccacctaacgttgcaactgggagacttccgggggcctcactgctcctagag 5581
5582 ccctcttcgctgactgccaatatgaaggaggtacctctgttcaggctacgtcacttccct 5641
5642 tgtgggaatgtcaattacggctaccagcaacagggcttgcccttagaagccgctactgcc 5701
5702 cctggagctggtcattacgaggataccattctgaaaagcaagaatagcatgaaccagcct 5761
5762 gggccctgagctcggtcgcacactcacttctcttccttgggatccctaagaccgtggagg 5821
5822 agagagaggcaatggctccttcacaaaccagagaccaaatgtcacgttttgttttgtgcc 5881
5882 aacctattttgaagtaccaccaaaaaagctgtattttgaaaatgctttagaaaggttttg 5941
5942 agcatgggttcatcctattctttcgaaagaagaaaatatcataaaaatgagtgataaata 6001
6002 caaggcccagatgtggttgcataaggtttttatgcatgtttgttgtatacttccttatgc 6061
6062 ttctttcaaattgtgtgtgctctgcttcaatgtagtcagaattagctgcttctatgtttc 6121
6122 atagttggggtcatagatgtttccttgccttgttgatgtggacatgagccatttgagggg 6181
6182 agagggaacggaaataaaggagttatttgtaatgactaaaa 6222

By "alteration" is meant a change (increase or decrease) in the expression levels or activity of a gene or polypeptide as detected by standard art known methods such as those described herein. As used herein, an alteration includes a 5% change in expression levels, a 10% change in expression levels, preferably a 25% change, more preferably a 40% change, and most preferably a 50% or greater change in expression levels.

By "ameliorate" is meant decrease, reduce, delay diminish, suppress, attenuate, arrest, or stabilize the development or progression of a disease or pathological condition.

By "antibody" is meant an immunoglobulin (Ig) molecule produced by B lymphoid cells and having a specific amino acid sequence. Antibodies are evoked or elicited in subjects (humans or other animals or mammals) following exposure to a specific antigen (immunogen). A subject capable of generating antibodies/immunoglobulins (*i.e.,* an immune response) directed against a specific antigen/immunogen is said to be immunocompetent. Antibodies are characterized by reacting specifically with (*e*.*g*., binding to) an antigen or immunogen in some demonstrable way, antibody and antigen/immunogen each being defined in terms of the other.

"Eliciting an antibody response" refers to the ability of an antigen, immunogen or other molecule to induce the production of antibodies. Antibodies are of different classes, *e.g.,* IgM, IgG, IgA, IgE, IgD and subtypes or subclasses, *e*.*g*., IgG1, IgG2, IgG2a, IgG2b, IgG3, IgG4. An antibody/immunoglobulin response elicited in a subject can neutralize a pathogenic (*e*.*g*., disease-causing) agent by binding to epitopes (antigenic determinants) on the agent and blocking or inhibiting the activity of the agent, and/or by forming a binding complex with the agent that is cleared from the system of the subject, *e*.*g*., via the liver.

By "amphiphile" is meant a chemical compound possessing both hydrophilic and lipophilic properties. Such a compound is called amphiphilic or amphipathic. The amphiphile may be conjugated or linked to an antigen or adjuvant cargo by a solubility-promoting polar polymer chain. In some embodiments, the amphiphile is conjugated or linked to an adjuvant. In some embodiments, the adjuvant is Freund's adjuvant. In some embodiments, the amphiphile is conjugated or linked to an ALK antigen or immunogen. In some embodiments, the amphiphile is a lipophilic albumin-binding tail. In some embodiments, the amphiphile is N-hydroxy succinimidyl ester-end-functionalized poly(ethylene glycol)-lipid (NHS-PEG2KDa-DSPE).

By "antigen" is meant an agent (*e*.*g*., polypeptide, peptide) that can stimulate an immune response in an animal, including compositions that are injected or absorbed into an animal. An antigen reacts with the products of specific humoral or cellular immunity, including those induced by heterologous immunogens. In some embodiments of the disclosed compositions and methods, the antigen is an ALK protein or an antibody-binding portion thereof. An antigen that elicits or stimulates an immune response in a subject is termed an "immunogen."

A "codon-optimized" nucleic acid (polynucleotide) refers to a nucleic acid sequence that has been altered such that the codons are optimal for expression in a particular system (such as a particular species of group of species). For example, a nucleic acid sequence can be optimized for expression in mammalian cells. Codon optimization does not alter the amino acid sequence of the encoded protein.

In this disclosure, "comprises," "comprising," "containing" and "having" and the like can have the meaning ascribed to them in U.S. Patent law and can mean "includes," "including," and the like; "consisting essentially of" or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments.

"Detect" refers to identifying the presence, absence or amount of an analyte, compound, agent, or substance to be detected.

By "detectable label" is meant a composition that, when linked to a molecule of interest, renders the latter detectable, *e*.*g*., via spectroscopic, photochemical, biochemical, immunochemical, or chemical means. Nonlimiting examples of useful detectable labels include radioactive isotopes, magnetic beads, metallic beads, colloidal particles, fluorescent dyes, electron-dense reagents, enzymes (for example, as commonly used in an ELISA), biotin, digoxigenin, or haptens.

By "disease" is meant any condition, disorder, or pathology that damages or interferes with the normal function of a cell, tissue, or organ. Examples of diseases include those caused by oncogenic ALK gene fusions, rearrangements, duplications or mutations (*e*.*g*., ALK-positive cancers). In some embodiments, the cancer is an ALK-positive cancer. By "ALK-positive cancer" is meant a cancer or tumor that expresses the ALK protein. Nonlimiting examples of ALK-positive cancers include non-small cell lung cancer (NSCLC), anaplastic large cell lymphoma (ALCL), neuroblastoma, B-cell lymphoma, thyroid cancer, colon cancer, breast cancer, inflammatory myofibroblastic tumors (IMT), renal carcinoma, esophageal cancer, and melanoma. The ALK-positive cancer may be caused by an oncogenic *ALK* gene that either forms a fusion gene with other genes, gains additional gene copies, or is genetically mutated. In some embodiments, the ALK-positive cancer is caused by an *ALK* fusion gene encoding an ALK fusion protein. In some embodiments, the ALK-positive cancer is caused by a fusion between the *ALK* gene and the *nucleophosmin* (*NPM*) gene encoding a NPM-ALK fusion protein. In some embodiments, the ALK-positive cancer is caused by a fusion between the *ALK* gene and the *echinoderm microtubule-associated protein-like 4* (*EML4*) gene encoding an ELM4-ALK fusion protein.

By "effective amount" is meant the amount of an active therapeutic agent, composition, compound, biologic (*e*.*g*., a vaccine or therapeutic peptide, polypeptide, or polynucleotide) required to ameliorate, reduce, delay, improve, abrogate, diminish, or eliminate the symptoms and/or effects of a disease, condition, or pathology relative to an untreated patient. In some embodiments, an effective amount of an ALK peptide is the amount required to induce an ALK-specific immune response in a subject immunized with the peptide. The effective amount of an immunogen or a composition comprising an immunogen, as used to practice the methods of therapeutic treatment of a disease, condition, or pathology, varies depending upon the manner of administration, the age, body weight, and general health of the subject. Ultimately, the attending physician or veterinarian will decide the appropriate amount and dosage regimen. Such amount is referred to as an "effective" amount.

The inventions herein provide a number of targets that are useful for the development of highly specific drugs to treat a disease or disorder characterized by the methods delineated herein. In addition, the methods of the invention provide a facile means to identify therapies that are safe for use in subjects. In addition, the methods of the invention provide a route for analyzing virtually any number of compounds for effects on a disease described herein with high-volume throughput, high sensitivity, and low complexity.

A "therapeutically effective amount" refers to a quantity of a specified agent sufficient to achieve a desired effect in a subject being treated with that agent. For example, this may be the amount of an ALK-specific antigen, immunogen, immunogenic composition or vaccine useful for eliciting an immune response in a subject, treating and/or for preventing a disease caused by oncogenic ALK gene fusions, rearrangements, duplications or mutations (*e*.*g*., ALK-positive cancers). Ideally, in the context of the present disclosure, a therapeutically effective amount of an ALK-specific vaccine or immunogenic composition is an amount sufficient to prevent, ameliorate, reduce, delay and/or treat a disease caused by oncogenic ALK gene fusions, rearrangements, duplications or mutations (*e*.*g*., ALK-positive cancers) in a subject without causing a substantial cytotoxic effect in the subject. The effective amount of an ALK-specific vaccine or immunogenic composition useful for preventing, delaying, ameliorating, reducing, and/or treating a disease caused by oncogenic ALK gene fusions, rearrangements, duplications or mutations (*e*.*g*., ALK-positive cancers) in a subject depends on, for example, the subject being treated, the manner of administration of the therapeutic composition and other factors, as noted *supra.*

By "fragment" is meant a portion of a polypeptide or nucleic acid molecule. This portion contains, preferably, at least 10%, 20%, 30%, 40%, 50%, 60%, 70%, 80%, or 90% of the entire length of the reference nucleic acid molecule or polypeptide. A fragment may contain 3, 4, 5, 6, 7, 8, 9, 10, 20, 30, 40, 50, 60, 70, 80, 90, or 100, 200, 300, 400, 500, 600, 700, 800, 900, or 1000 nucleotides or amino acids. A portion or fragment of a polypeptide may be a peptide. In the case of an antibody or immunoglobulin fragment, the fragment typically binds to the target antigen.

By "fusion protein" is meant a protein generated by expression of a nucleic acid (polynucleotide) sequence engineered from nucleic acid sequences encoding at least a portion of two different (heterologous) proteins or peptides. To create a fusion protein, the nucleic acid sequences must be in the same open reading frame and contain no internal stop codons. One protein can be located at the amino-terminal (N-terminal) portion of the fusion protein or at the carboxy-terminal (C-terminal) protein thus forming an amino-terminal fusion protein or a carboxy-terminal fusion protein, respectively.

For example, a fusion protein includes an ALK protein fused to a heterologous protein. In some embodiments, the fusion protein is an ALK protein fused to a nucleophosmin (NPM) protein. In some embodiments, the NPM-ALK fusion protein is at least 85%, at least 90%, at least 95%, at least 98%, at least 99%, or 100% identical to a NPM-ALK fusion protein in *Homo Sapiens.* In some embodiments, the NPM-ALK fusion protein is at least 85%, at least 90%, at least 95%, at least 98%, at least 99%, or 100% identical to an exemplary NPM-ALK fusion protein amino acid sequence from *Homo Sapiens* as provided below (ALK cytoplasmic portion in bold font):

In some embodiments, the NPM-ALK fusion protein is at least 85%, at least 90%, at least 95%, at least 98%, at least 99%, or 100% identical to an exemplary NPM-ALK fusion protein amino acid sequence from *Homo Sapiens* (GenBank: AAA58698.1) as provided below:

1 medsmdmdms plrpqnylfg celkadkdyh fkvdndeneh qlslrtvslg agakdelhiv

61 eaeamnyegs pikvtlatlk msvqptvslg gfeitppvvl rlkcgsgpvh isgqhlvvyr

121 rkhqelqamq melqspeykl sklrtstimt dynpnycfag ktssisdlke vprknitlir

181 glghgafgev yegqvsgmpn dpsplqvavk tlpevcseqd eldflmeali iskfnhqniv

241 rcigvslqsl prfillelma ggdlksflre trprpsqpss lamldllhva rdiacgcqyl

301 eenhfihrdi aarnclltcp gpgrvakigd fgmardiyra syyrkggcam lpvkwmppea

361 fmegiftskt dtwsfgvllw eifslgympy psksnqevle fvtsggrmdp pkncpgpvyr

421 imtqcwqhqp edrpnfaiil erieyctqdp dvintalpie ygplveeeek vpvrpkdpeg

481 vppllvsqqa kreeerspaa ppplpttssg kaakkptaae vsvrvprgpa vegghvnmaf

541 sqsnppselh kvhgsrnkpt slwnptygsw ftekptkknn piakkephdr gnlglegsct

601 vppnvatgrl pgasllleps sltanmkevp lfrlrhfpcg nvnygyqqqg lpleaatapg

661 aghyedtilk sknsmnqpgp

In some embodiments, the NPM-ALK fusion protein is at least 85%, at least 90%, at least 95%, at least 98%, at least 99%, or 100% identical to an exemplary NPM-ALK fusion protein amino acid sequence from *Homo Sapiens* as provided below:

M E D S M D M D M S P L R P Q N Y L F G
C E L K A D K D Y H F K V D N D E N E H
Q L S L R T V S L G A G A K D E L H I V
E A E A M N Y E G S P I K V T L A T L K
M S V Q P T V S L G G F E I T P P V V L
R L K C G S G P V H I S G Q H L V V Y R
R K H Q E L Q A M Q M E L Q S P E Y K L
S K L R T S T I M T D Y N P N Y C F A G
K T S S I S D L K E V P R K N I T L I R
G L G H G A F G E V Y E G Q V S G M P N
D P S P L Q V A V K T L P E V C S E Q D
E L D F L M E A L I I S K F N H Q N I V
R C I G V S L Q S L P R F I L L E L M A
G G D L K S F L R E T R P R P S Q P S S
L A M L D L L H V A R D I A C G C Q Y L
E E N H F I H R D I A A R N C L L T C P
G P G R V A K I G D F G M A R D I Y R A
S Y Y R K G G C A M L P V K W M P P E A
F M E G I F T S K T D T W S F G V L L W
E I F S L G Y M P Y P S K S N Q E V L E
F V T S G G R M D P P K N C P G P V Y R
I M T Q C W Q H Q P E D R P N F A I I L
E R I E Y C T Q D P D V I N T A L P I E
Y G P L V E E E K V P V R P K D P E G
V P P L L V S Q Q A K R E E E R S P A A
P P P L P T T S S G K A A K K P T A A E
V S V R V P R G P A V E G G H V N M A F
S Q S N P P S E L H K V H G S R N K P T
S L W N P T Y G S W F T E K P T K K N N
P I A K K E P H D R G N L G L E G S C T
V P P N V A T G R L P G A S L L L E P S
S L T A N M K E V P L F R L R H F P C G
N V N Y G Y Q Q Q G L P L E A A T A P G
A G H Y E D T I L K S K N S M N Q P G P
*

In some embodiments, the NPM-ALK fusion protein is encoded by a nucleic acid sequence that is at least 85%, at least 90%, at least 95%, at least 98%, at least 99%, or 100% identical to the exemplary nucleic acid sequence from *Homo Sapiens* as provided below:

1 atggaagattcgatggacatggacatgagccccctgaggccccagaactatcttttcggt 60

61 tgtgaactaaaggccgacaaagattatcactttaaggtggataatgatgaaaatgagcac 120

121 cagttatctttaagaacggtcagtttaggggctggtgcaaaggatgagttgcacattgtt 180

181 gaagcagaggcaatgaattacgaaggcagtccaattaaagtaacactggcaactttgaaa 240
241 atgtctgtacagccaacggtttcccttgggggctttgaaataacaccaccagtggtctta 300
301 aggttgaagtgtggttcagggccagtgcatattagtggacagcacttagtagtgtaccgc 360
361 cggaagcaccaggagctgcaagccatgcagatggagctgcagagccctgagtacaagctg 420
421 agcaagctccgcacctcgaccatcatgaccgactacaaccccaactactgctttgctggc 480
481 aagacctcctccatcagtgacctgaaggaggtgccgcggaaaaacatcaccctcattcgg 540
541 ggtctgggccatggcgcctttggggaggtgtatgaaggccaggtgtccggaatgcccaac 600
601 gacccaagccccctgcaagtggctgtgaagacgctgcctgaagtgtgctctgaacaggac 660
661 gaactggatttcctcatggaagccctgatcatcagcaaattcaaccaccagaacattgtt 720
721 cgctgcattggggtgagcctgcaatccctgccccggttcatcctgctggagctcatggcgg 780
781 ggggagacctcaagtccttcctccgagagacccgccctcgcccgagccagccctcctcc 840
841 ctggccatgctggaccttctgcacgtggctcgggacattgcctgtggctgtcagtatttg 900
901 gaggaaaaccacttcatccaccgagacattgctgccagaaactgcctcttgacctgtcca 960
961 ggccctggaagagtggccaagattggagacttcgggatggcccgagacatctacagggcg 1020
1021 agctactatagaaagggaggctgtgccatgctgccagttaagtggatgcccccagaggcc 1080
1081 ttcatggaaggaatattcacttctaaaacagacacatggtcctttggagtgctgctatgg 1140
1141 gaaatcttttctcttggatatatgccataccccagcaaaagcaaccaggaagttctggag 1200
1201 tttgtcaccagtggaggccggatggacccacccaagaactgccctgggcctgtataccgg 1260
1261 ataatgactcagtgctggcaacatcagcctgaagacaggcccaactttgccatcattttg 1320
1321 gagaggattgaatactgcacccaggacccggatgtaatcaacaccgctttgccgatagaa 1380
1381 tatggtccacttgtggaagaggaagagaaagtgcctgtgaggcccaaggaccctgagggg 1440
1441 gttcctcctctcctggtctctcaacaggcaaaacgggaggaggagcgcagcccagctgcc 1500
1501 ccaccacctctgcctaccacctcctctggcaaggctgcaaagaaacccacagctgcagag 1560
1561 gtctctgttcgagtccctagagggccggccgtggaagggggacacgtgaatatggcattc 1620
1621 tctcagtccaaccctccttcggagttgcacaaggtccacggatccagaaacaagcccacc 1680
1681 agcttgtggaacccaacgtacggctcctggtttacagagaaacccaccaaaaagaataat 1740
1741 cctatagcaaagaaggagccacacgacaggggtaacctggggctggagggaagctgtact 1800
1801 gtcccacctaacgttgcaactgggagacttccgggggcctcactgctcctagagccctct 1860
1861 tcgctgactgccaatatgaaggaggtacctctgttcaggctacgtcacttcccttgtggg 1920
1921 aatgtcaattacggctaccagcaacagggcttgcccttagaagccgctactgcccctgga 1980
1981 gctggtcattacgaggataccattctgaaaagcaagaatagcatgaaccagcctgggccc 2040
2041 tga 2043

In some embodiments, the fusion protein is an ALK protein fused to an echinoderm microtubule-associated protein-like 4 (EML4) protein. In some embodiments, the ELM4-ALK fusion protein is at least 85%, at least 90%, at least 95%, at least 98%, at least 99%, or 100% identical to a ELM4-ALK fusion protein in *Homo Sapiens* or a variant thereof. In some embodiments, the ELM4-ALK fusion protein is at least 85%, at least 90%, at least 95%, at least 98%, at least 99%, or 100% identical to an exemplary ELM4-ALK fusion protein amino acid sequence from *Homo Sapiens* (GenBank: BAM37627.1) as provided below:

1 
61 
121 
181 
241 
301 
361 
421 
481 
541 
601 
661 
721 
781 
841 
901 
961 
1021 
1081 

In some embodiments, the ELM4-ALK fusion protein is encoded by a nucleic acid sequence that is at least 85%, at least 90%, at least 95%, at least 98%, at least 99%, or 100% identical to an exemplary nucleic acid sequence from *Homo Sapiens* (GenBank: AB274722.1) as provided below:

1 ggcggcgcgg cgcggcgctc gcggctgctg cctgggaggg aggccgggca ggcggctgag

61 cggcgcggct ctcaacgtga cggggaagtg gttcgggcgg ccgcggctta ctaccccagg

121 gcgaacggac ggacgacgga ggcgggagcc ggtagccgag ccgggcgacc tagagaacga

181 gcgggtcagg ctcagcgtcg gccactctgt cggtccgctg aatgaagtgc ccgcccctct

241 gagcccggag cccggcgctt tccccgcaag atggacggtt tcgccggcag tctcgatgat

301 agtatttctg ctgcaagtac ttctgatgtt caagatcgcc tgtcagctct tgagtcacga

361 gttcagcaac aagaagatga aatcactgtg ctaaaggcgg ctttggctga tgttttgagg

421 cgtcttgcaa tctctgaaga tcatgtggcc tcagtgaaaa aatcagtctc aagtaaaggc

481 caaccaagcc ctcgagcagt tattcccatg tcctgtataa ccaatggaag tggtgcaaac

541 agaaaaccaa gtcataccag tgctgtctca attgcaggaa aagaaactct ttcatctgct

601 gctaaaagtg gtacagaaaa aaagaaagaa aaaccacaag gacagagaga aaaaaaagag

661 gaatctcatt ctaatgatca aagtccacaa attcgagcat caccttctcc ccagccctct

721 tcacaacctc tccaaataca cagacaaact ccagaaagca agaatgctac tcccaccaaa

781 agcataaaac gaccatcacc agctgaaaag tcacataatt cttgggaaaa ttcagatgat

841 agccgtaata aattgtcgaa aataccttca acacccaaat taataccaaa agttaccaaa

901 actgcagaca agcataaaga tgtcatcatc aaccaagaag gagaatatat taaaatgttt

961 atgcgcggtc ggccaattac catgttcatt ccttccgatg ttgacaacta tgatgacatc

1021 agaacggaac tgcctcctga gaagctcaaa ctggagtggg catatggtta tcgaggaaag

1081 gactgtagag ctaatgttta ccttcttccg accggggaaa tagtttattt cattgcatca

1141 gtagtagtac tatttaatta tgaggagaga actcagcgac actacctggg ccatacagac

1201 tgtgtgaaat gccttgctat acatcctgac aaaattagga ttgcaactgg acagatagct

1261 ggcgtggata aagatggaag gcctctacaa ccccacgtca gagtgtggga ttctgttact

1321 ctatccacac tgcagattat tggacttggc acttttgagc gtggagtagg atgcctggat

1381 ttttcaaaag cagattcagg tgttcattta tgtgttattg atgactccaa tgagcatatg

1441 cttactgtat gggactggca gaagaaagca aaaggagcag aaataaagac aacaaatgaa

1501 gttgttttgg ctgtggagtt tcacccaaca gatgcaaata ccataattac atgcggtaaa

1561 tctcatattt tcttctggac ctggagcggc aattcactaa caagaaaaca gggaattttt

1621 gggaaatatg aaaagccaaa atttgtgcag tgtttagcat tcttggggaa tggagatgtt

1681 cttactggag actcaggtgg agtcatgctt atatggagca aaactactgt agagcccaca

1741 cctgggaaag gacctaaagt gtaccgccgg aagcaccagg agctgcaagc catgcagatg

1801 gagctgcaga gccctgagta caagctgagc aagctccgca cctcgaccat catgaccgac

1861 tacaacccca actactgctt tgctggcaag acctcctcca tcagtgacct gaaggaggtg

1921 ccgcggaaaa acatcaccct cattcggggt ctgggccatg gagcctttgg ggaggtgtat

1981 gaaggccagg tgtccggaat gcccaacgac ccaagccccc tgcaagtggc tgtgaagacg

2041 ctgcctgaag tgtgctctga acaggacgaa ctggatttcc tcatggaagc cctgatcatc

2101 agcaaattca accaccagaa cattgttcgc tgcattgggg tgagcctgca atccctgccc

2161 cggttcatcc tgctggagct catggcgggg ggagacctca agtccttcct ccgagagacc

2221 cgccctcgcc cgagccagcc ctcctccctg gccatgctgg accttctgca cgtggctcgg

2281 gacattgcct gtggctgtca gtatttggag gaaaaccact tcatccaccg agacattgct

2341 gccagaaact gcctcttgac ctgtccaggc cctggaagag tggccaagat tggagacttc

2401 gggatggccc gagacatcta cagggcgagc tactatagaa agggaggctg tgccatgctg

2461 ccagttaagt ggatgccccc agaggccttc atggaaggaa tattcacttc taaaacagac

2521 acatggtcct ttggagtgct gctatgggaa atcttttctc ttggatatat gccatacccc

2581 agcaaaagca accaggaagt tctggagttt gtcaccagtg gaggccggat ggacccaccc

2641 aagaactgcc ctgggcctgt ataccggata atgactcagt gctggcaaca tcagcctgaa

2701 gacaggccca actttgccat cattttggag aggattgaat actgcaccca ggacccggat

2761 gtaatcaaca ccgctttgcc gatagaatat ggtccacttg tggaagagga agagaaagtg

2821 cctgtgaggc ccaaggaccc tgagggggtt cctcctctcc tggtctctca acaggcaaaa

2881 cgggaggagg agcgcagccc agctgcccca ccacctctgc ctaccacctc ctctggcaag

2941 gctgcaaaga aacccacagc tgcagaggtc tctgttcgag tccctagagg gccggccgtg

3001 gaagggggac acgtgaatat ggcattctct cagtccaacc ctccttcgga gttgcacagg

3061 gtccacggat ccagaaacaa gcccaccagc ttgtggaacc caacgtacgg ctcctggttt

3121 acagagaaac ccaccaaaaa gaataatcct atagcaaaga aggagccaca cgagaggggt

3181 aacctggggc tggagggaag ctgtactgtc ccacctaacg ttgcaactgg gagacttccg

3241 ggggcctcac tgctcctaga gccctcttcg ctgactgcca atatgaagga ggtacctctg

3301 ttcaggctac gtcacttccc ttgtgggaat gtcaattacg gctaccagca acagggcttg

3361 cccttagaag ccgctactgc ccctggagct ggtcattacg aggataccat tctgaaaagc

3421 aagaatagca tgaaccagcc tgggccctga gctcggtcac acactcactt ctcttccttg

3481 ggatccctaa gaccgtggag gagagagagg caatcaatgg ctccttcaca aaccagagac

3541 caaatgtcac gttttgtttt gtgccaacct attttgaagt accaccaaaa aagctgtatt

3601 ttgaaaatgc tttagaaagg ttttgagcat gggttcatcc tattctttcg aaagaagaaa

3661 atatcataaa aatgagtgat aaatacaagg cccagatgtg gttgcataag gtttttatgc

3721 atgtttgttg tatacttcct tatgcttctt ttaaattgtg tgtgctctgc ttcaatgtag

3781 tcagaattag ctgcttctat gtttcatagt tggggtcata gatgtttcct tgccttgttg

3841 atgtggacat gagccatttg aggggagagg gaacggaaat aaaggagtta tttgtaatga

3901 aaaaaaaaaa aaaaaaaaaa aaaaaa

In some embodiments, the ELM4-ALK fusion protein is at least 85%, at least 90%, at least 95%, at least 98%, at least 99%, or 100% identical to an exemplary ELM4-ALK variant 1 fusion protein amino acid sequence from *Homo Sapiens* (GenBank: BAF73611.1) as provided below:

1 
61 
121 

181 
241 
301 
361 
421 
481 
541 
601 
661 
721 
781 
841 
901 
961 
1021 

By "genetic vaccine" is meant an immunogenic composition comprising a polynucleotide encoding an antigen.

"Hybridization" means hydrogen bonding, which may be Watson-Crick, Hoogsteen, or reversed Hoogsteen hydrogen bonding, between complementary nucleobases. For example, in DNA, adenine and thymine, and cytosine and guanine, are, respectively, complementary nucleobases that pair through the formation of hydrogen bonds. By "hybridize" is meant pairing to form a double-stranded molecule between complementary polynucleotide sequences (*e*.*g*., a gene), or portions thereof, under various conditions of stringency. (*See, e.g.,* Wahl, G. M. and S. L. Berger, (1987), Methods Enzymol., 152:399; Kimmel, A. R., (1987), Methods Enzymol. 152:507).

By way of example, stringent salt concentration will ordinarily be less than about 750 mM NaCl and 75 mM trisodium citrate, preferably less than about 500 mM NaCl and 50 mM trisodium citrate, and more preferably less than about 250 mM NaCl and 25 mM trisodium citrate. Low stringency hybridization can be obtained in the absence of organic solvent, *e*.*g*., formamide, while high stringency hybridization can be obtained in the presence of at least about 35% formamide, and more preferably at least about 50% formamide. Stringent temperature conditions will ordinarily include temperatures of at least about 30°C, more preferably of at least about 37°C, and most preferably of at least about 42°C. Varying additional parameters, such as hybridization time, the concentration of detergent, *e*.*g*., sodium dodecyl sulfate (SDS), and the inclusion or exclusion of carrier DNA, are well known to those skilled in the art. Various levels of stringency are accomplished by combining these various conditions as needed. In a preferred: embodiment, hybridization will occur at 30°C in 750 mM NaCl, 75 mM trisodium citrate, and 1% SDS. In a more preferred embodiment, hybridization will occur at 37°C in 500 mM NaCl, 50 mM trisodium citrate, 1% SDS, 35% formamide, and 100 µg/ml denatured salmon sperm DNA (ssDNA). In a most preferred embodiment, hybridization will occur at 42°C in 250 mM NaCl, 25 mM trisodium citrate, 1% SDS, 50% formamide, and 200 µg/ml ssDNA. Useful variations on these conditions will be apparent to those skilled in the art.

For most applications, washing steps that follow hybridization will also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25°C, more preferably of at least about 42°C, and even more preferably of at least about 68°C. In a preferred embodiment, wash steps will occur at 25°C in 30 mM NaCl, 3 mM trisodium citrate, and 0.1% SDS. In a more preferred embodiment, wash steps will occur at 42 C in 15 mM NaCl, 1.5 mM trisodium citrate, and 0.1% SDS. In a more preferred embodiment, wash steps will occur at 68°C in 15 mM NaCl, 1.5 mM trisodium citrate, and 0.1% SDS. Additional variations on these conditions will be apparent to those skilled in the art. Hybridization techniques are well known to those skilled in the art and are described, for example, in Benton and Davis (Science 196:180, 1977); Grunstein and Hogness (Proc. Natl. Acad. Sci., USA 72:3961, 1975); Ausubel et al. (Current Protocols in Molecular Biology, Wiley Interscience, New York, 2001); Berger and Kimmel (Guide to Molecular Cloning Techniques, 1987, Academic Press, New York); and Sambrook et al., Molecular Cloning: A Laboratory Manual, Cold Spring Harbor Laboratory Press, New York.

By "immunogen" is meant agent which is capable, under appropriate conditions, of eliciting or stimulating an immune response, such as the production a T-cell response, in an animal, including compositions that are injected or absorbed into an animal. As used herein, an "immunogenic composition" is a composition comprising an immunogen (such as an ALK polypeptide) or a vaccine comprising an immunogen (such as an ALK polypeptide). As will be appreciated by the skilled person in the art, if administered to a subject in need prior to the subject's contracting disease or experiencing full-blown disease, an immunogenic composition can be prophylactic and result in the subject's eliciting an immune response, *e.g.,* a cellular immune response, to protect against disease, or to prevent more severe disease or condition, and/or the symptoms thereof. If administered to a subject in need following the subject's contracting disease, an immunogenic composition can be therapeutic and result in the subject's eliciting an immune response, *e.g.,* a cellular immune response, to treat the disease, *e*.*g*., by reducing, diminishing, abrogating, ameliorating, or eliminating the disease, and/or the symptoms thereof. In some embodiments, the immune response is a B-cell response, which results in the production of antibodies, *e*.*g*., neutralizing antibodies, directed against the immunogen or immunogenic composition comprising the antigen or antigen sequence. In some embodiments, the immune response is a T-cell response, which results in the production of T-lymphocytes. In a manner similar to the foregoing, in some embodiments, an immunogenic composition or vaccine can be prophylactic. In some embodiments, an immunogenic composition or vaccine can be therapeutic. In some embodiments, the disease is caused by oncogenic ALK gene fusions, rearrangements, duplications or mutations (*e*.*g*., ALK-positive cancers). In some embodiments, the cancer is an ALK-positive cancer. In some embodiments, the ALK-positive cancer is non-small cell lung cancer (NSCLC), anaplastic large cell lymphoma (ALCL), neuroblastoma, B-cell lymphoma, thyroid cancer, colon cancer, breast cancer, inflammatory myofibroblastic tumors (IMT), renal carcinoma, esophageal cancer, melanoma, or a combination thereof.

The term "immune response" is meant any response mediated by an immunoresponsive cell. In one example of an immune response, leukocytes are recruited to carry out a variety of different specific functions in response to exposure to an antigen (*e.g.,* a foreign entity). Immune responses are multifactorial processes that differ depending on the type of cells involved. Immune responses include cell-mediated responses (*e*.*g*., T-cell responses), humoral responses (B-cell/antibody responses), innate responses and combinations thereof.

By "immunogenic composition" is meant a composition comprising an antigen, antigen sequence, or immunogen, wherein the composition elicits an immune response in an immunized subj ect.

The term "immunize" (or immunization) refers to rendering a subject protected from a disease or pathology, or the symptoms thereof, caused by oncogenic ALK gene fusions, rearrangements, duplications or mutations (*e*.*g*., ALK-positive cancers), such as by vaccination.

The terms "isolated," "purified," or "biologically pure" refer to material that is free to varying degrees from components which normally accompany it as found in its native state. "Isolate" denotes a degree of separation from original source or surroundings. "Purify" denotes a degree of separation that is higher than isolation. A "purified" or "biologically pure" protein is sufficiently free of other materials such that any impurities do not materially affect the biological properties of the protein or cause other adverse consequences. That is, a nucleic acid, protein, or peptide is purified if it is substantially free of cellular material, debris, non-relevant viral material, or culture medium when produced by recombinant DNA techniques, or of chemical precursors or other chemicals when chemically synthesized. Purity and homogeneity are typically determined using standard purification methods and analytical chemistry techniques, for example, polyacrylamide gel electrophoresis or high-performance liquid chromatography. The term "purified" can denote that a nucleic acid or protein gives rise to essentially one band in an electrophoretic gel. For a protein that can be subjected to modifications, for example, phosphorylation or glycosylation, different modifications may give rise to different isolated proteins, which can be separately purified. The term "isolated" also embraces recombinant nucleic acids or proteins, as well as chemically synthesized nucleic acids or peptides.

By "isolated polynucleotide" is meant a nucleic acid (*e.g.,* a DNA molecule) that is free of the genes which flank the gene, in the naturally-occurring genome of the organism from which the nucleic acid molecule of the invention is derived. The term includes, for example, a recombinant DNA that is incorporated into a vector; into an autonomously replicating plasmid or virus; into the genomic DNA of a prokaryote or eukaryote; or that exists as a separate molecule independent of other sequences (for example, a cDNA or a genomic or cDNA fragment produced by PCR or restriction endonuclease digestion). In addition, the term includes an RNA molecule that is transcribed from a DNA molecule, as well as a recombinant DNA that is part of a hybrid gene encoding additional polypeptide sequence.

By an "isolated polypeptide" is meant a polypeptide of the invention that has been separated from components that naturally accompany it. Typically, the polypeptide is isolated when it is at least 40%, by weight, at least 50%, by weight, at least 60%, by weight, free from the proteins and naturally-occurring organic molecules with which it is naturally associated. Preferably, an isolated polypeptide preparation is at least 75%, more preferably at least 90%, and most preferably at least 99%, by weight, free from the proteins and naturally-occurring organic molecules with which it is naturally associated. An isolated polypeptide may be obtained, for example, by extraction from a natural source; by expression of a recombinant nucleic acid encoding such a polypeptide; or by chemically synthesizing the protein. Purity can be measured by any standard, appropriate method, for example, column chromatography, polyacrylamide gel electrophoresis, or by HPLC analysis. An isolated polypeptide can refer to an ALK antigen or immunogen polypeptide generated by the methods described herein.

By "linker" is meant one or more amino acids that serve as a spacer between two polypeptides or peptides of a fusion protein.

By "marker" is meant any protein or polynucleotide having an alteration in expression level or activity that is associated with a disease, condition, pathology, or disorder.

As used herein, "obtaining" as in "obtaining an agent" includes synthesizing, isolating, purchasing, or otherwise acquiring the agent.

The term "operably linked" refers to nucleic acid sequences as used herein. By way of example, a first nucleic acid sequence is operably linked to a second nucleic acid sequence when the first nucleic acid sequence is placed in a functional relationship with the second nucleic acid sequence. For instance, a promoter is operably linked to a coding sequence if the promoter affects (allows) the transcription or expression of the coding sequence. Generally, operably linked DNA sequences are contiguous and, where necessary to join two protein-coding regions, are in the same open reading frame.

The nucleic acid sequence encoding an ALK protein (antigen protein) generated by the described methods can be optimized for expression in mammalian cells via codon-optimization and RNA optimization (such as to increase RNA stability) using procedures and techniques practiced in the art.

By "open reading frame (ORF)" is meant a series of nucleotide triplets (codons) that code for amino acids without any termination codons. These sequences are usually translatable into a peptide or polypeptide.

The term "pharmaceutically acceptable vehicle" refers to conventional carriers (vehicles) and excipients that are physiologically and pharmaceutically acceptable for use, particularly in mammalian, *e*.*g*., human, subjects. Such pharmaceutically acceptable vehicles are known to the skilled practitioner in the pertinent art and can be readily found in Remington's Pharmaceutical Sciences, by E. W. Martin, Mack Publishing Co., Easton, Pa., 15th Edition (1975) and its updated editions, which describes compositions and formulations suitable for pharmaceutical delivery of one or more therapeutic or immunogenic compositions, such as one or more vaccines, and additional pharmaceutical agents. In general, the nature of a pharmaceutically acceptable carrier depends on the particular mode of administration being employed. For instance, parenteral formulations usually comprise injectable fluids/liquids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers may include, for example, pharmaceutical grades of mannitol, lactose, starch, or magnesium stearate, which typically stabilize and/or increase the half-life of a composition or drug. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

By "plasmid" is meant a circular nucleic acid molecule capable of autonomous replication in a host cell.

The terms "protein," "peptide," "polypeptide," and their grammatical equivalents are used interchangeably herein, and refer to a polymer of amino acid residues linked together by peptide (amide) bonds. The terms refer to a protein, peptide, or polypeptide of any size, structure, or function. Typically, a protein, peptide, or polypeptide will be at least three (3) amino acids long. A protein, peptide, or polypeptide can refer to an individual protein or a collection of proteins. One or more of the amino acids in a protein, peptide, or polypeptide can be modified, such as glycoproteins, for example, by the addition of a chemical entity such as a carbohydrate group, a hydroxyl group, a phosphate group, a farnesyl group, an isofarnesyl group, a fatty acid group, a linker for conjugation, functionalization, or other modifications, etc. A protein, peptide, or polypeptide can also be a single molecule or can be a multi-molecular complex. A protein, peptide, or polypeptide can be just a fragment of a naturally occurring protein or peptide. A protein, peptide, or polypeptide can be naturally occurring, recombinant, or synthetic, or any combination thereof.

In some embodiments, a protein comprises a proteinaceous part, *e*.*g*., an amino acid sequence constituting a nucleic acid binding domain, and an organic compound, *e*.*g*., a compound that can act as a nucleic acid cleavage agent. In some embodiments, a protein is in a complex with, or is in association with, a nucleic acid, e.g., RNA or DNA. Any of the proteins provided herein can be produced by any method known in the art. For example, the proteins provided herein can be produced via recombinant protein expression and purification, which is especially suited for fusion proteins comprising a peptide linker. Methods for recombinant protein expression and purification are well known, and include those described by Green and Sambrook, Molecular Cloning: A Laboratory Manual (4th ed., Cold Spring Harbor Laboratory Press, Cold Spring Harbor, N.Y. (2012)), the entire contents of which are incorporated herein by reference.

Conservative amino acid substitutions are those substitutions that, when made, least interfere with the properties of the original protein, that is, the structure and especially the function of the protein is conserved and is not significantly changed by such substitutions. Examples of conservative amino acid substitutions are known in the art, *e*.*g*., as set forth in, for example, U.S. Publication No. 2015/0030628. Conservative substitutions generally maintain (a) the structure of the polypeptide backbone in the area of the substitution, for example, as a sheet or helical conformation; (b) the charge or hydrophobicity of the molecule at the target site; and/or (c) the bulk of the side chain

The substitutions that are generally expected to produce the greatest changes in protein properties are non-conservative, for instance, changes in which (a) a hydrophilic residue, for example, seryl or threonyl, is substituted for (or by) a hydrophobic residue, for example, leucyl, isoleucyl, phenylalanyl, valyl or alanyl; (b) a cysteine or proline is substituted for (or by) any other residue; (c) a residue having an electropositive side chain, for example, lysyl, arginyl, or histadyl, is substituted for (or by) an electronegative residue, for example, glutamyl or aspartyl; or (d) a residue having a bulky side chain, for example, phenylalanine, is substituted for (or by) one not having a side chain, for example, glycine.

By "promoter" is meant an array of nucleic acid control sequences, which direct transcription of a nucleic acid. A promoter includes necessary nucleic acid sequences near the start site of transcription. A promoter also optionally includes distal enhancer or repressor sequence elements. A "constitutive promoter" is a promoter that is continuously active and is not subject to regulation by external signals or molecules. In contrast, the activity of an "inducible promoter" is regulated by an external signal or molecule (for example, a transcription factor). By way of example, a promoter may be a CMV promoter.

As will be appreciated by the skilled practitioner in the art, the term "purified" does not require absolute purity; rather, it is intended as a relative term. Thus, for example, a purified peptide, protein, or other active compound is one that is isolated in whole or in part from naturally associated proteins and other contaminants. In certain embodiments, the term "substantially purified" refers to a peptide, protein, or other active compound that has been isolated from a cell, cell culture medium, or other crude preparation and subjected to routine methods, such as fractionation, chromatography, or electrophoresis, to remove various components of the initial preparation, such as proteins, cellular debris, and other components.

A "recombinant" nucleic acid or protein is one that has a sequence that is not naturally occurring or that has a sequence that is made by an artificial combination of two otherwise separated segments of sequence. Such an artificial combination is often accomplished by chemical synthesis or by the artificial manipulation of isolated segments of nucleic acids, for example, by genetic engineering techniques. A "non-naturally occurring" nucleic acid or protein is one that may be made via recombinant technology, artificial manipulation, or genetic or molecular biological engineering procedures and techniques, such as those commonly practiced in the art.

By "reduces" is meant a negative alteration of at least 5%, 10%, 25%, 30%, 40%, 50%, 75%, 80%, 85%, 90%, 95%, 98%, or 100%.

By "reference" is meant a standard or control condition.

A "reference sequence" is a defined sequence used as a basis for sequence comparison. A reference sequence may be a subset of or the entirety of a specified sequence; for example, a segment of a full-length cDNA or gene sequence, or the complete cDNA or gene sequence. For polypeptides, the length of the reference polypeptide sequence will generally be at least about 16 amino acids, preferably at least about 20 amino acids, more preferably at least about 25 amino acids, and even more preferably about 35 amino acids, about 50 amino acids, or about 100 amino acids. For nucleic acids, the length of the reference nucleic acid sequence will generally be at least about 50 nucleotides, preferably at least about 60 nucleotides, more preferably at least about 75 nucleotides, and even more preferably about 100 nucleotides or about 300 nucleotides or any integer thereabout or therebetween.

By "specifically binds" is meant a compound or antibody that recognizes and binds a polypeptide, such as an ALK polypeptide, peptide, or vaccine product, but which does not substantially recognize and bind other molecules in a sample, for example, a biological sample, which naturally includes a polypeptide of the invention, such as an ALK polypeptide or peptide.

Nucleic acid molecules useful in the methods described herein include any nucleic acid molecule that encodes a polypeptide as described, or a fragment thereof. Such nucleic acid molecules need not be 100% identical with an endogenous nucleic acid sequence, but will typically exhibit substantial identity. Polynucleotides having "substantial identity" to an endogenous sequence are typically capable of hybridizing with at least one strand of a double-stranded nucleic acid molecule.

By "substantially identical" is meant a polypeptide or nucleic acid molecule exhibiting at least 50% identity to a reference amino acid sequence (for example, any one of the amino acid sequences described herein) or nucleic acid sequence (for example, any one of the nucleic acid sequences described herein). Preferably, such a sequence is at least 60%, or at least 80% or 85%, or at least or equal to 90%, 95%, 98% or even 99% identical at the amino acid level or nucleic acid to the sequence used for comparison.

"Sequence identity" refers to the similarity between amino acid or nucleic acid sequences that is expressed in terms of the similarity between the sequences. Sequence identity is frequently measured in terms of percentage identity (or similarity or homology); the higher the percentage, the more similar the sequences are. Homologs or variants of a given gene or protein will possess a relatively high degree of sequence identity when aligned using standard methods. Sequence identity is typically measured using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical or similar sequences by assigning degrees of homology to various substitutions, deletions, and/or other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine. In an exemplary approach to determining the degree of identity, a BLAST program may be used, with a probability score between e⁻³ and e⁻¹⁰⁰ indicating a closely related sequence. In addition, other programs and alignment algorithms are described in, for example, Smith and Waterman, 1981, Adv. Appl. Math. 2:482; Needleman and Wunsch, 1970, J. Mol. Biol. 48:443; Pearson and Lipman, 1988, Proc. Natl. Acad. Sci. U.S.A. 85:2444; Higgins and Sharp, 1988, Gene 73:237-244; Higgins and Sharp, 1989, CABIOS 5:151-153; Corpet et al., 1988, Nucleic Acids Research 16:10881-10890; Pearson and Lipman, 1988, Proc. Natl. Acad. Sci. U.S.A. 85:2444; and Altschul et al., 1994, Nature Genet. 6:119-129. The NCBI Basic Local Alignment Search Tool (BLAST^{™}) (Altschul et al. 1990, J. Mol. Biol. 215:403-410) is readily available from several sources, including the National Center for Biotechnology Information (NCBI, Bethesda, Md.) and on the Internet, for use in connection with the sequence analysis programs blastp, blastn, blastx, tblastn and tblastx.

By "subject" is meant an animal, *e.g.,* a mammal, including, but not limited to, a human, a non-human primate, or a non-human mammal, such as a bovine, equine, canine, ovine, or feline mammal, or a sheep, goat, llama, camel, or a rodent (rat, mouse), gerbil, or hamster. In a nonlimiting example, a subject is one who has, is at risk of developing, or who is susceptible to a disease caused by oncogenic ALK gene fusions, rearrangements, duplications or mutations (*e*.*g*., ALK-positive cancers). In particular aspects as described herein, the subject is a human subject, such as a patient.

Ranges provided herein are understood to be shorthand for all of the values within the range, inclusive of the first and last stated values. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or greater, consecutively, such as to 100 or greater.

As used herein, the terms "treat," "treating," "treatment," and the like refer to reducing, diminishing, decreasing, delaying, abrogating, ameliorating, or eliminating, a disease, condition, disorder, or pathology, and/or symptoms associated therewith. While not intending to be limiting, "treating" typically relates to a therapeutic intervention that occurs after a disease, condition, disorder, or pathology, and/or symptoms associated therewith, have begun to develop to reduce the severity of the disease, etc., and the associated signs and symptoms. It will be appreciated that, although not precluded, treating a disorder or condition does not require that the disease, condition, disorder, pathology, or the symptoms associated therewith, be completely eliminated.

As used herein, the terms "prevent," "preventing," "prevention," "prophylactic treatment" and the like, refer to inhibiting or blocking a disease state, or the full development of a disease in a subject, or reducing the probability of developing a disease, disorder or condition in a subject, who does not have, but is at risk of developing, or is susceptible to developing, a disease, disorder, or condition.

As referred to herein, a "transformed" or "transfected" cell is a cell into which a nucleic acid molecule or polynucleotide sequence has been introduced by molecular biology techniques. As used herein, the term "transfection" encompasses all techniques by which a nucleic acid molecule or polynucleotide may be introduced into such a cell, including transfection with viral vectors, transformation with plasmid vectors, and introduction of naked nucleic acid (DNA or RNA) by electroporation, lipofection, and particle gun acceleration.

By "vaccine" is meant a preparation of immunogenic material (*e*.*g*., protein or nucleic acid; vaccine) capable of stimulating (eliciting) an immune response, administered to a subject to treat a disease, condition, or pathology, or to prevent a disease, condition, or pathology caused by oncogenic *ALK* gene fusions, rearrangements, duplications or mutations (*e*.*g*., ALK-positive cancers). The immunogenic material may include, for example, antigenic proteins, peptides or DNA derived from ALK-expressing tumors or cell lines. Vaccines may elicit a prophylactic (preventative) immune response in the subject; they may also elicit a therapeutic response immune response in a subject. As mentioned above, methods of vaccine administration vary according to the vaccine, and can include routes or means, such as inoculation (intravenous or subcutaneous injection), ingestion, inhalation, or other forms of administration. Inoculations can be delivered by any number of routes, including parenteral, such as intravenous, subcutaneous or intramuscular. Vaccines may also be administered with an adjuvant to boost the immune response.

As used herein, a "vector" refers to a nucleic acid (polynucleotide) molecule into which foreign nucleic acid can be inserted without disrupting the ability of the vector to replicate in and/or integrate into a host cell. A vector can include nucleic acid sequences that permit it to replicate in a host cell, such as an origin of replication. An insertional vector is capable of inserting itself into a host nucleic acid. A vector can also include one or more selectable marker genes and other genetic elements. An expression vector is a vector that contains the necessary regulatory sequences to allow transcription and translation of inserted gene or genes in a host cell. In some embodiments of the present disclosure, the vector encodes an ALK protein. In some embodiments, the vector is the pTR600 expression vector (U.S. Patent Application Publication No. 2002/0106798; Ross et al., 2000, Nat Immunol. 1(2): 102-103; and Green et al., 2001, Vaccine 20:242-248).

Unless specifically stated or obvious from context, as used herein, the term "or" is understood to be inclusive. Unless specifically stated or obvious from context, as used herein, the terms "a," "an," and "the" are understood to be singular or plural. Similarly, the word "or" is intended to include "and" unless the context clearly indicates otherwise. Hence "comprising A or B" means including A, or B, or A and B. It is further to be understood that all base sizes or amino acid sizes, and all molecular weight or molecular mass values, given for nucleic acids or polypeptides are approximate, and are provided for description.

Unless specifically stated or obvious from context, as used herein, the term "about" is understood as within a range of normal tolerance in the art, for example within two (2) standard deviations (SD) of the mean. About may be understood as being within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

The recitation of a listing of chemical groups in any definition of a variable herein includes definitions of that variable as any single group or combination of listed groups. The recitation of some embodiments for a variable or aspect herein includes that embodiment as any single embodiment or in combination with any other embodiments or portions thereof.

Any compositions or methods provided herein can be combined with one or more of any of the other compositions and methods provided herein.

### BRIEF DESCRIPTION OF THE DRAWINGS

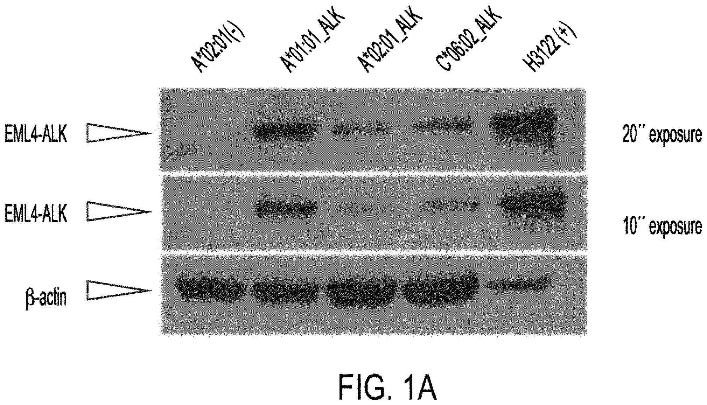

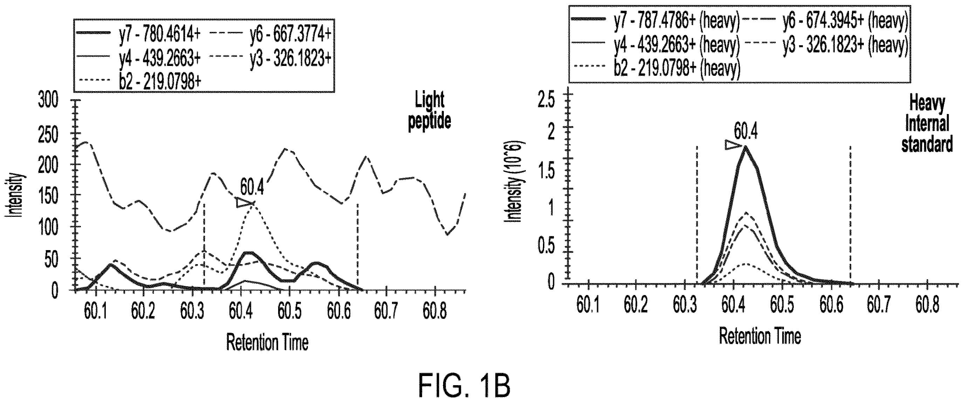

**FIGS. 1A** and **1B** depict the identification of ALK-specific peptides in B721.221/HLA monoallelic cells. **FIG. 1A** shows a western blot of transduced B721.221/A*02:01, B721.221/A*01:01 and B721.221/C*06:02 cells with a construct encoding the EMI,4-ALK variant 1 and GFP. Cells were sorted based on the GFP positivity and expanded. The untransduced B721.221/A*02:01 cell line was used as a negative control and the H3122 ALK+ NSCLC cell line was used as a positive control. **FIG. 1B** provides graphical depictions of targeted mass spectrometry in HLA*02:01 elute from B721.221/A*02:01 cells measuring endogenous un-labeled peptides (light peptides) and two algorithm predicted isotope-labeled ALK-specific peptides, AMI,DLLHVA and SLAMLDLLHV (heavy standard).

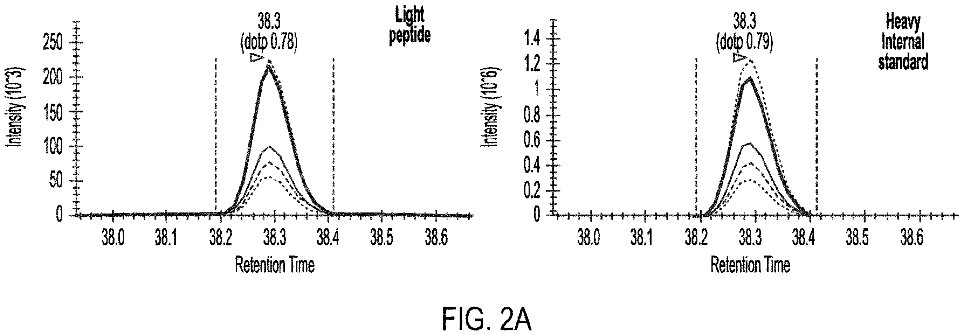

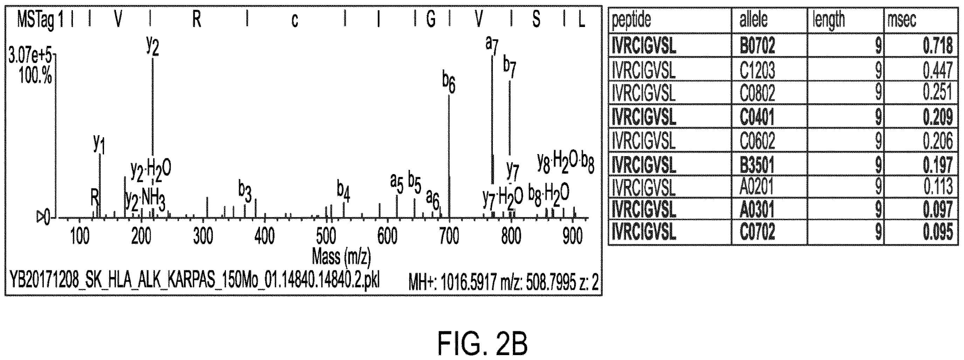

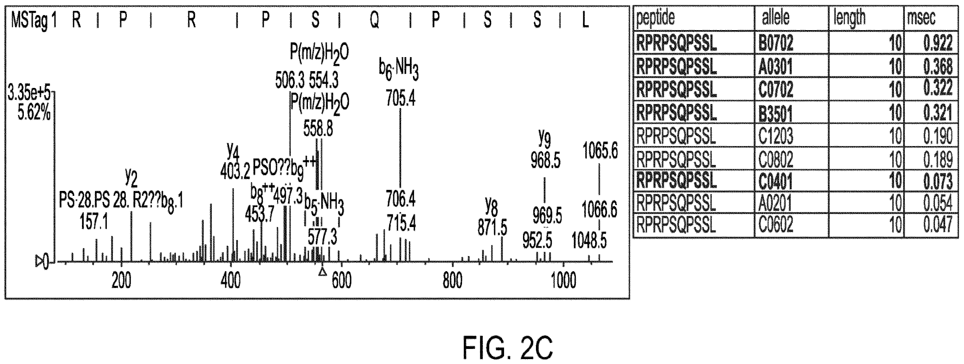

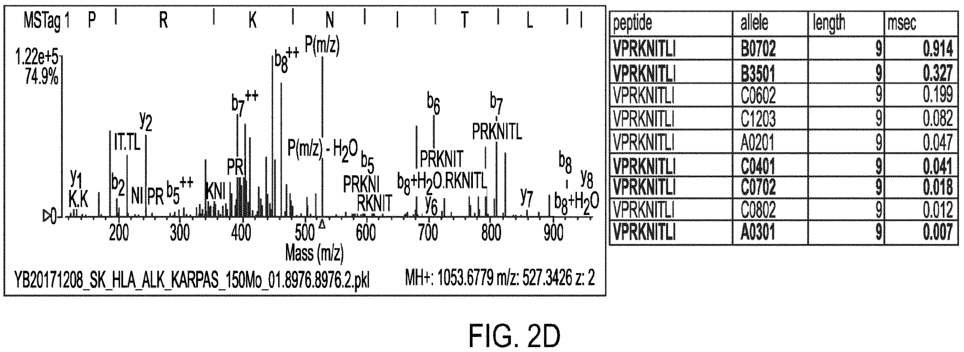

**FIGS. 2A** - **2D** depict the identification of ALK-specific peptides in ALK-positive ALCL cell lines. **FIG. 2A** provides graphical depictions of targeted mass spectrometry in HLA*02:01 elute from DEL cells measuring endogenous un-labeled peptides (light peptides) and two algorithm predicted isotope-labeled ALK-specific peptides, AMI,DLLHVA and SLAMLDLLHV (heavy standard). **FIG. 2B** provides a graphical depiction of the IVRCIGVSL ALK-peptide identified by discovery mass spectrometry in the pan-HLA elute from the Karpas-299 cell line and a table depicting the assignment of the ALK-peptide to an HLA allele. Karpas-299 alleles are shown in bold font. **FIG. 2C** provides a graphical depiction of the RPRPSQPSSL ALK-peptide identified by discovery mass spectrometry in the pan-HLA elute from the Karpas-299 cell line and a table depicting the assignment of the ALK-peptide to an HLA allele. Karpas-299 alleles are shown in bold font. **FIG. 2D** provides a graphical depiction of the VPRKNITLI ALK-peptide identified by discovery mass spectrometry in the pan-HLA elute from the Karpas-299 cell line and a table depicting the assignment of the ALK-peptide to an HLA allele. Karpas-299 alleles are shown in bold font.

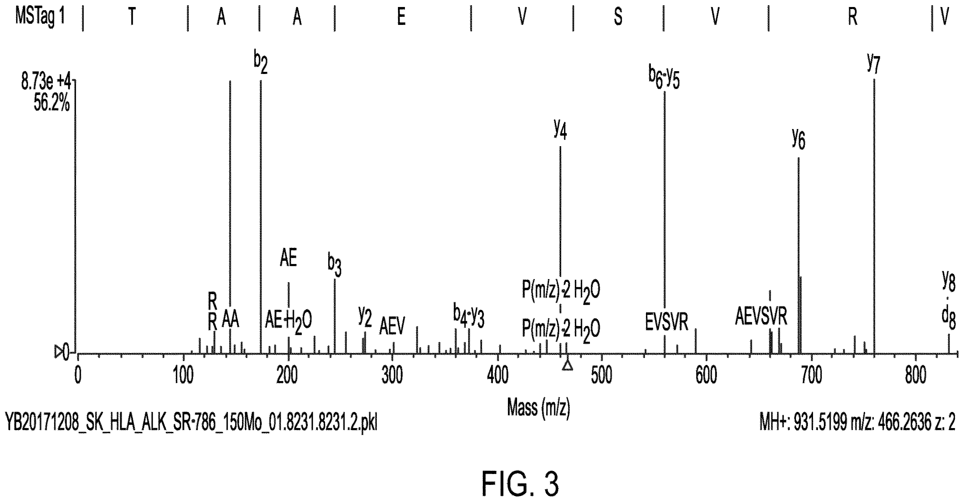

**FIG. 3** provides a graphical depiction of discovery mass spectrometry of the TAAEVSVRV ALK-peptide identified in HLA elute from the SR-786 cell line.

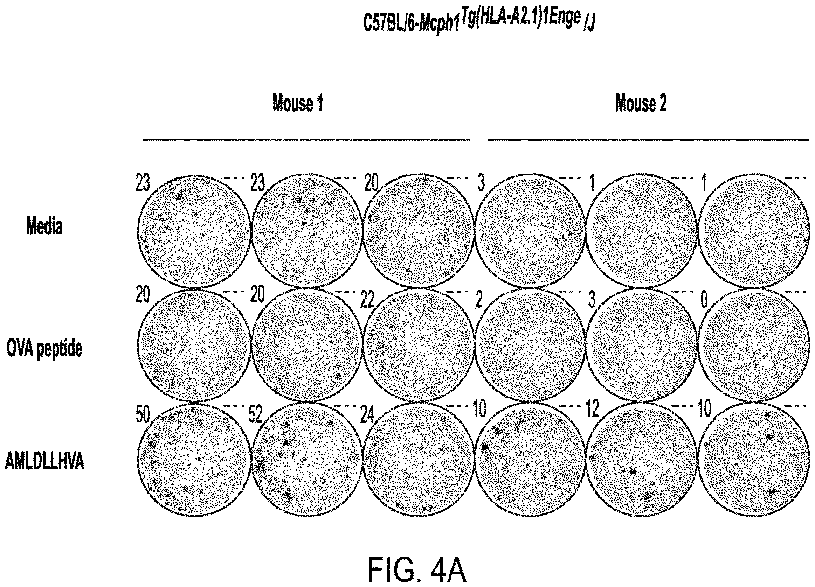

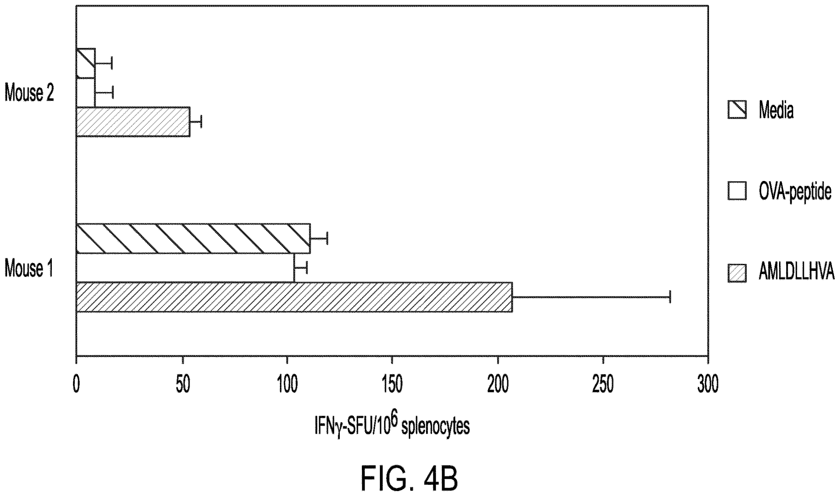

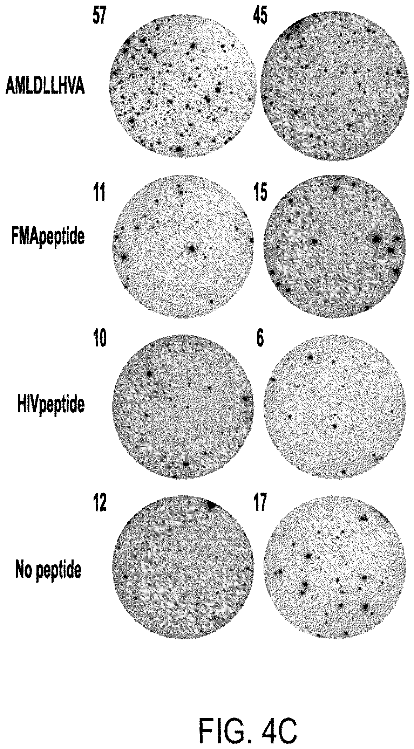

**FIGS. 4A** - **4C** depict an immune response against the HLA A*02:01-binding peptide AMLDLLHVA. **FIG. 4A** shows images of an interferon-gamma (IFN-γ) ELISPOT assay challenged with splenocytes from two (2) C57BL/*6-Mcphl^{Tg(HLA-A2.1)1Enge}*/J transgenic mice vaccinated with AMLDLLHVA together with Freund's complete adjuvant. Splenocytes were challenged with either the ALK peptide, OVA peptide (negative control), or media (negative control) in an IFN-γ-ELISPOT assay. **FIG. 4B** is a graphical depiction measuring the INF-γ spot forming units (SFU) between wells with splenocytes challenged with AMLDLLHVA and those wells challenged with OVA-peptide from the two (2) C57BL/6-*Mcph1^{Tg(HLA-A2.1)1Enge}*/J mice vaccinated with AMLDLLHVA. Error bars mean SD. **FIG. 4C** shows images of a direct IFN-γ ELISPOT assay challenged with PBMCs from ALK-positive NSCLC HLA*02:01 patients with AMLDLLHVA. HIV peptide and no peptide stimulation were used as negative controls. HLA*02:01 -specific Flu matrix peptide was used as a positive control.

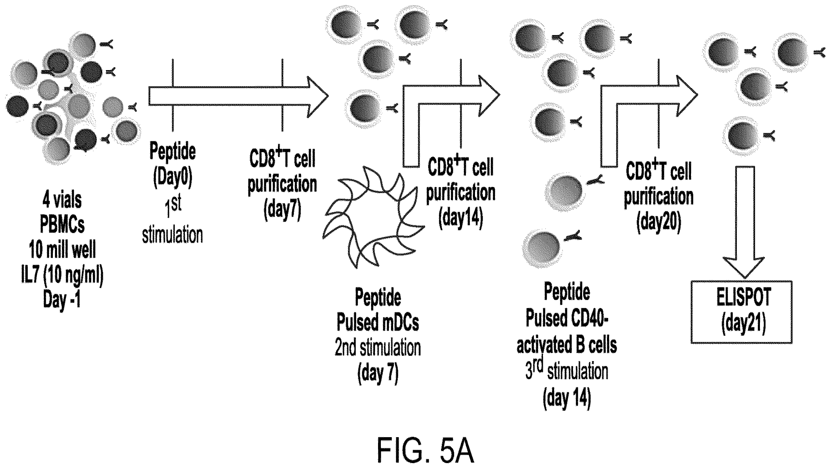

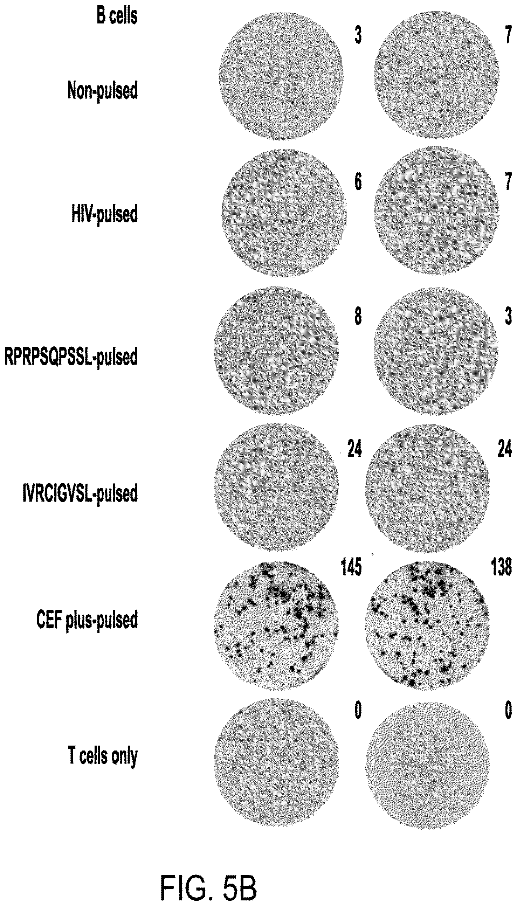

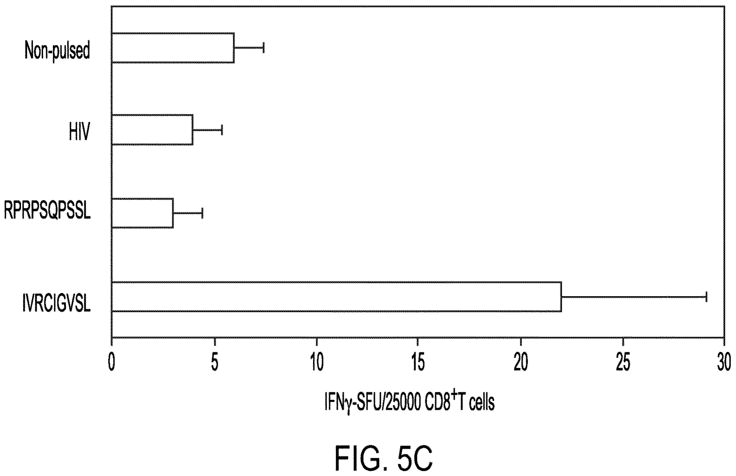

**FIGS. 5A** - **5C** depict peptide-specific CD8⁺ T-cell expansion for ALK B*07:02 binding peptides. **FIG. 5A** depicts an illustrative scheme of the protocol followed for peptide-specific CD8⁺ T-cell expansion. PBMCs from ALK-positive NSCLC HLA*07:02 patients were thawed and rested overnight in the presence of rh-II,-7 at day-1. At day 0, PBMCs were stimulated alternatively with IVRCIGVSL or RPRPSQPSSL. From day 3, and every 3-4 days, fresh media containing rh-IL-2 and rh-IL-7 was added. Second and third stimulation was done by incubating purified CD8⁺ T-cells with peptide-pulsed mature DCs (mDCs) or CD40-activated B-cells, respectively. IFN-γ ELISPOT was performed as a read out of the expansion at day 21. **FIG 5B** shows images of an IFN-γ ELISPOT assay of IVRCIGVSL-expanded CD8⁺ T-cells challenged with B-cells pulsed with different peptides. HIV-pulsed and non-pulsed B-cells were used as negative controls. CEF plus-pulsed B-cells were used as positive control. **FIG 5C** is a graphical depiction measuring the INF-γ spot forming units (SFU) between wells challenged with IVRCIGVSL-pulsed B-cells and those wells with B-cells pulsed with control peptides. HIV-pulsed and non-pulsed B-cells were used as negative controls. Error bars mean SD.

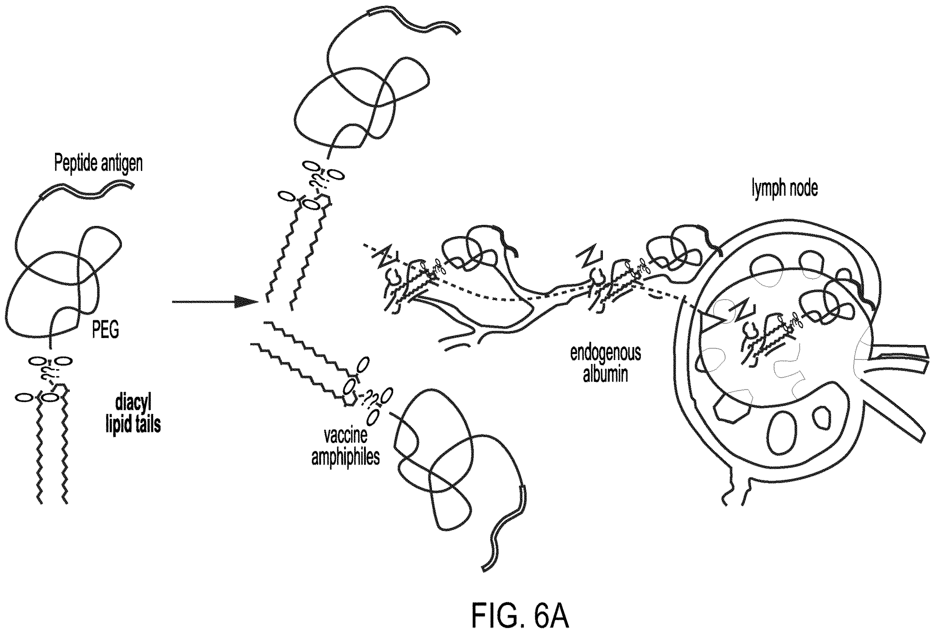

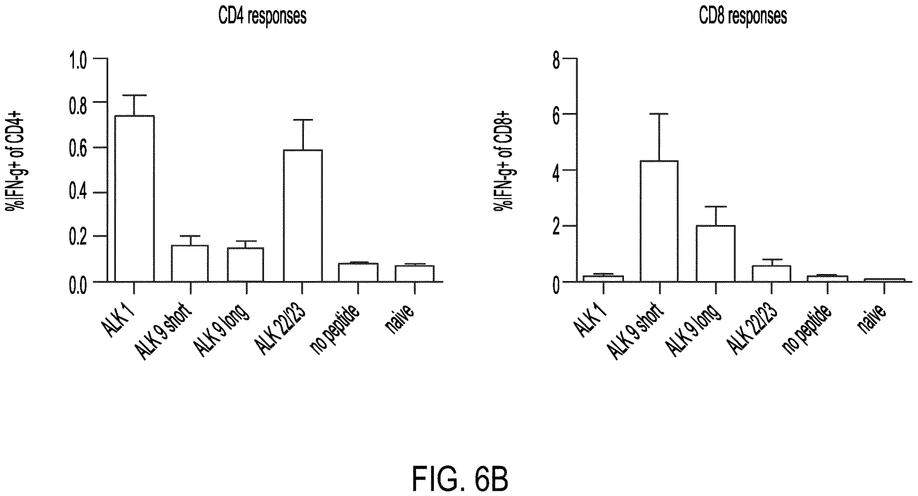

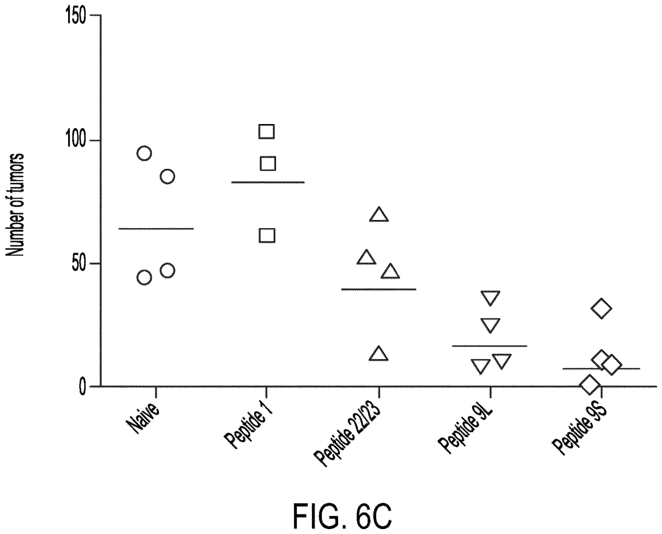

**FIGS. 6A** - **6C** depict amphiphile vaccines eliciting protective T-cell responses in mice. **FIG. 6A** depicts a schematic illustration of amphiphile-peptide vaccine function by binding to endogenous albumin present in interstitial fluid following injection, with subsequent albumin-mediated transport to lymph nodes for efficient capture by dendritic cells. **FIG. 6B** is a graphical depiction of CD4⁺ and CD8⁺ T-cell responses in Balb/c mice immunized with a series of different ALK peptides and then assayed for *ex vivo* antigen-specific cytokine production. No peptide and naive peptide were used as negative controls. **FIG. 6C** is a graphical depiction of the number of tumors assessed 14 days after Balb/c mice were immunized with a series of different ALK peptides. Naive peptide was used as a negative control.

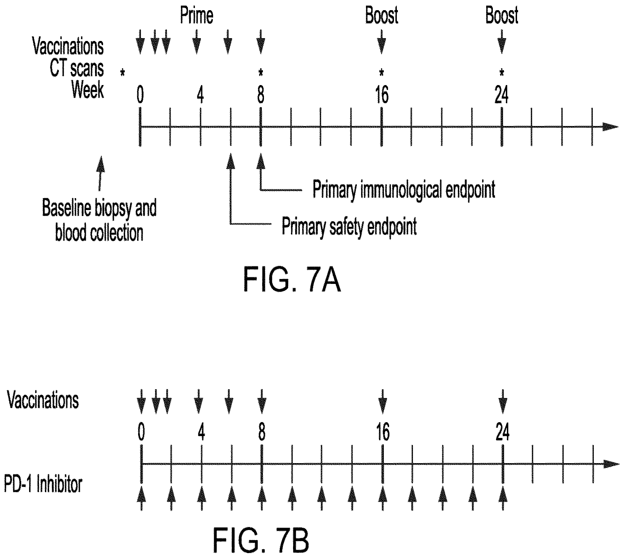

**FIGS. 7A** and 7B depict schematic illustrations of ALK-peptide vaccination administration schedules. **FIG. 7A** depicts a schematic illustration of ALK-peptide vaccination administration for either naked ALK-peptide or amphiphilic-conjugated ALK-peptide monotherapy. **FIG. 7B** depicts a schematic illustration of ALK-peptide vaccination administration for ALK-peptide (naked or amphiphilic-conjugated) in combination therapy with a PD-1 inhibitor.

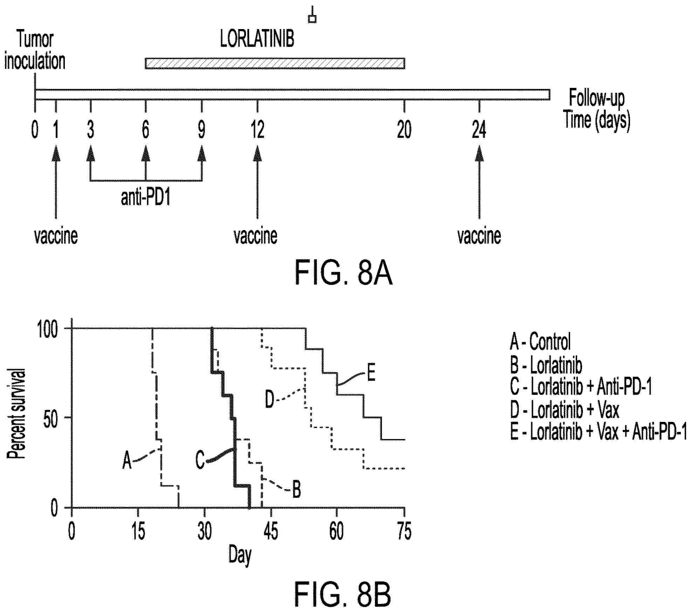

**FIGS. 8A** and **8B** depict schematic illustrations of ALK-peptide vaccination administration in combination with ALK inhibitor therapy (lorlatinib) and immune checkpoint therapy (anti-PD-1 antibody). **FIG. 8A** depicts a schematic illustration of a vaccination administration schedule for mice with NSCLC cell induced lung tumors treated with an ALK-peptide in combination with lorlatinib and anti-PD-1 antibodies. **FIG. 8B** is a graphical depiction of Kaplan-Meyer survival curves of mice with NSCLC cell induced lung tumors treated with either control, lorlatinib, lorlatinib with anti-PD-1 antibodies, lorlatinib with ALK-peptide vaccine, or lorlatinib with ALK-peptide vaccine and anti-PD-1 antibodies.

### DETAILED DESCRIPTION OF THE INVENTION

Lung cancer is the most common cause of cancer-related death worldwide, and the annual incidence of anaplastic lymphoma kinase (ALK) expressing non-small cell lung cancer (NSCLC) in the U.S. is about 8,000 cases. In these patients, treatment with ALK tyrosine kinase inhibitors (TKIs) fails to induce durable remissions. In this context, a successful ALK vaccine could lead to durable responses and greatly improve survival and quality of life for NSCLC patients. ALK represents an attractive target for vaccine development because of its oncogenicity, its immunogenicity, and its restricted expression to tumor tissue rather than healthy adult tissue. Importantly, use of a therapeutic ALK peptide vaccine could potentially be extended to many other cancer types which are driven by ALK rearrangements or activating mutations (*i*.*e*., ALK-positive cancers), such as anaplastic large cell lymphoma (ALCL), neuroblastoma, B-cell lymphoma, thyroid cancer, colon cancer, breast cancer, inflammatory myofibroblastic tumors (IMT), renal carcinoma, esophageal cancer, and melanoma. Therefore, a vaccine as described herein generated against the rearranged portion of ALK can both prevent the development of ALK-positive tumors and more effectively treat patients diagnosed with ALK-positive tumors.

As described below, the present invention features isolated ALK-specific immunogenic antigens, *e*.*g*., peptide antigens, derived from ALK-positive cell lines and immune cells from patients with ALK-positive cancers. Such immunogenic antigens are also referred to as "immunogens" herein. The ALK-specific immunogenic antigens elicit a potent immune response, *e*.*g*., in the form of reactive T-lymphocytes, following administration or delivery to, or introduction into, a subject, particularly, a human subject. The isolated ALK-specific immunogenic antigens may be used in methods to treat and/or reduce disease caused by oncogenic ALK gene fusions, rearrangements, duplications or mutations. The isolated ALK-specific immunogenic antigens may be conjugated to an amphiphilic tail in order to significantly increase T-cell expansion and greatly enhance anti-tumor efficacy.

The immunogenic ALK antigens described herein may be used in immunogenic compositions (*e*.*g*., ALK-specific vaccines) that treat ALK-positive cancers caused by oncogenic ALK gene fusions, rearrangements, duplications or mutations in a subject, particularly a human subject, to whom the immunogenic composition or vaccine, is administered. The vaccine elicits a potent ALK protein-specific T cell response that treats and/or protects against ALK-positive cancers in a subject. The antigens, immunogens, immunogenic compositions and vaccines, and pharmaceutical compositions thereof, of the invention provide an additional treatment option for patients that have either become resistant to or have failed to respond to prior and traditional therapies for ALK-positive cancers.

### Identification of ALK Proteins

The use of computational algorithms has been successfully applied in recent studies to identify T-cell neoantigens in both human and mice. (Carreno BM, et al. Cancer immunotherapy. A dendritic cell vaccine increases the breadth and diversity of melanoma neoantigen-specific T-cells. Science. 2015;348(6236):803-808; Gubin MM, et al. Checkpoint blockade cancer immunotherapy targets tumour-specific tumor antigens. Nature. 2014;515(7528):577-581). However, these algorithms are almost exclusively based on the affinities of synthetic peptides, and do not necessarily consider other parameters such as antigen expression levels, intracellular processing, and the transport of the peptides prior to HLA binding. Altogether, these factors can significantly limit the accuracy of the current algorithms in predicting genuine T-cell epitopes.

By using a liquid chromatography-tandem mass spectrometry (LC-MS/MS) analysis of the peptides presented on the surface of HLA monoallelic cells, only 26% of the peptides that actually bind common HLA alleles were predicted by Immune Epitope Database (IEDB) algorithms, one of the most commonly used algorithms (www.iedb.org). (Abelin JG, et al. Mass Spectrometry Profiling of HLA-Associated Peptidomes in Mono-allelic Cells Enables More Accurate Epitope Prediction. Immunity. 2017;46(2):315-326). The level of accuracy drops down to 0% for rare HLA alleles. Thus, current algorithms used to predict antigenic peptides are generally inaccurate and misleading as to which antigens are actually presented on tumor cell surfaces. (*See* Abelin JG, et al. Mass Spectrometry Profiling of HLA-Associated Peptidomes in Mono-allelic Cells Enables More Accurate Epitope Prediction. Immunity. 2017;46(2):315-326).

For direct identification of ALK antigenic peptides effectively presented on the cell surface of tumor cells in the most common HLA haplotypes, the HLA monoallelic cell system and algorithms as provided in Abelin JG, et al. (Mass Spectrometry Profiling of HLA-Associated Peptidomes in Mono-allelic Cells Enables More Accurate Epitope Prediction. Immunity. 2017;46(2):315-326), which is incorporated herein in its entirety, were adapted. To directly identify ALK peptides actually presented on the surface of ALK-expressing tumor cells, HLA-peptide complexes were pulled from ALK-expressing cell lines lysates and the HLA-bound peptides were analyzed using liquid chromatography-tandem mass spectrometry (LC-MS/MS).

The invention provides for a method for identifying the ALK-specific peptides provided herein as described in **Example 1**. In some embodiments, the ALK-expressing cell lines for use in identifying the ALK antigenic peptides provided herein encode specific HLA-alleles (*e*.*g*., HLA class I alleles) and may express or may be transduced with a construct to express an ALK fusion protein. In some embodiments, the ALK-expressing cells lines are generated as described in Abelin JG, et al. (Mass Spectrometry Profiling of HLA-Associated Peptidomes in Mono-allelic Cells Enables More Accurate Epitope Prediction. Immunity. 2017;46(2):315-326), which is incorporated herein in its entirety. In some embodiments, the HLA is encoded by a HLA class I allele. Nonlimiting examples of HLA class I alleles expressed by ALK-expressing cell lines are provided in **Table 1, Table 2** or **Table 3**. In some embodiments, the HLA class I allele is HLA A*02:01, HLA A*68:02, HLA B*07:02, or HLA C*07:02.

In some embodiments, the fusion protein is an ALK protein fused to an echinoderm microtubule-associated protein-like 4 (EML4) protein (ELM4-ALK). In some embodiments, the ELM4-ALK fusion protein is at least 85%, at least 90%, at least 95%, at least 98%, at least 99%, or 100% identical to a ELM4-ALK fusion protein in *Homo Sapiens* or a variant thereof. In some embodiments, the ELM4-ALK fusion protein is at least 85%, at least 90%, at least 95%, at least 98%, at least 99%, or 100% identical to an exemplary ELM4-ALK fusion protein amino acid sequence from *Homo Sapiens* (GenBank: BAM37627.1) as provided below:

1 
61 
121 
181 
241 
301 
361 
421 
481 
541 
601 
661 
721 
781 
841 
901 

961 

1021 

1081 

In some embodiments, the ELM4-ALK fusion protein is encoded by a nucleic acid sequence from *Homo Sapiens* that is at least 85%, at least 90%, at least 95%, at least 98%, at least 99%, or 100% identical to GenBank: AB274722.1 as provided below:

1 ggcggcgcgg cgcggcgctc gcggctgctg cctgggaggg aggccgggca ggcggctgag

61 cggcgcggct ctcaacgtga cggggaagtg gttcgggcgg ccgcggctta ctaccccagg

121 gcgaacggac ggacgacgga ggcgggagcc ggtagccgag ccgggcgacc tagagaacga

181 gcgggtcagg ctcagcgtcg gccactctgt cggtccgctg aatgaagtgc ccgcccctct

241 gagcccggag cccggcgctt tccccgcaag atggacggtt tcgccggcag tctcgatgat

301 agtatttctg ctgcaagtac ttctgatgtt caagatcgcc tgtcagctct tgagtcacga

361 gttcagcaac aagaagatga aatcactgtg ctaaaggcgg ctttggctga tgttttgagg

421 cgtcttgcaa tctctgaaga tcatgtggcc tcagtgaaaa aatcagtctc aagtaaaggc

481 caaccaagcc ctcgagcagt tattcccatg tcctgtataa ccaatggaag tggtgcaaac

541 agaaaaccaa gtcataccag tgctgtctca attgcaggaa aagaaactct ttcatctgct

601 gctaaaagtg gtacagaaaa aaagaaagaa aaaccacaag gacagagaga aaaaaaagag

661 gaatctcatt ctaatgatca aagtccacaa attcgagcat caccttctcc ccagccctct

721 tcacaacctc tccaaataca cagacaaact ccagaaagca agaatgctac tcccaccaaa

781 agcataaaac gaccatcacc agctgaaaag tcacataatt cttgggaaaa ttcagatgat

841 agccgtaata aattgtcgaa aataccttca acacccaaat taataccaaa agttaccaaa

901 actgcagaca agcataaaga tgtcatcatc aaccaagaag gagaatatat taaaatgttt

961 atgcgcggtc ggccaattac catgttcatt ccttccgatg ttgacaacta tgatgacatc

1021 agaacggaac tgcctcctga gaagctcaaa ctggagtggg catatggtta tcgaggaaag

1081 gactgtagag ctaatgttta ccttcttccg accgggaaaa tagtttattt cattgcatca

1141 gtagtagtac tatttaatta tgaggagaga actcagcgac actacctggg ccatacagac

1201 tgtgtgaaat gccttgctat acatcctgac aaaattagga ttgcaactgg acagatagct

1261 ggcgtggata aagatggaag gcctctacaa ccccacgtca gagtgtggga ttctgttact

1321 ctatccacac tgcagattat tggacttggc acttttgagc gtggagtagg atgcctggat

1381 ttttcaaaag cagattcagg tgttcattta tgtgttattg atgactccaa tgagcatatg

1441 cttactgtat gggactggca gaagaaagca aaaggagcag aaataaagac aacaaatgaa

1501 gttgttttgg ctgtggagtt tcacccaaca gatgcaaata ccataattac atgcggtaaa

1561 tctcatattt tcttctggac ctggagcggc aattcactaa caagaaaaca gggaattttt

1621 gggaaatatg aaaagccaaa atttgtgcag tgtttagcat tcttggggaa tggagatgtt

1681 cttactggag actcaggtgg agtcatgctt atatggagca aaactactgt agagcccaca

1741 cctgggaaag gacctaaagt gtaccgccgg aagcaccagg agctgcaagc catgcagatg

1801 gagctgcaga gccctgagta caagctgagc aagctccgca cctcgaccat catgaccgac

1861 tacaacccca actactgctt tgctggcaag acctcctcca tcagtgacct gaaggaggtg

1921 ccgcggaaaa acatcaccct cattcggggt ctgggccatg gagcctttgg ggaggtgtat

1981 gaaggccagg tgtccggaat gcccaacgac ccaagccccc tgcaagtggc tgtgaagacg

2041 ctgcctgaag tgtgctctga acaggacgaa ctggatttcc tcatggaagc cctgatcatc

2101 agcaaattca accaccagaa cattgttcgc tgcattgggg tgagcctgca atccctgccc

2161 cggttcatcc tgctggagct catggcgggg ggagacctca agtccttcct ccgagagacc

2221 cgccctcgcc cgagccagcc ctcctccctg gccatgctgg accttctgca cgtggctcgg

2281 gacattgcct gtggctgtca gtatttggag gaaaaccact tcatccaccg agacattgct

2341 gccagaaact gcctcttgac ctgtccaggc cctggaagag tggccaagat tggagacttc

2401 gggatggccc gagacatcta cagggcgagc tactatagaa agggaggctg tgccatgctg

2461 ccagttaagt ggatgccccc agaggccttc atggaaggaa tattcacttc taaaacagac

2521 acatggtcct ttggagtgct gctatgggaa atcttttctc ttggatatat gccatacccc

2581 agcaaaagca accaggaagt tctggagttt gtcaccagtg gaggccggat ggacccaccc

2641 aagaactgcc ctgggcctgt ataccggata atgactcagt gctggcaaca tcagcctgaa

2701 gacaggccca actttgccat cattttggag aggattgaat actgcaccca ggacccggat

2761 gtaatcaaca ccgctttgcc gatagaatat ggtccacttg tggaagagga agagaaagtg

2821 cctgtgaggc ccaaggaccc tgagggggtt cctcctctcc tggtctctca acaggcaaaa

2881 cgggaggagg agcgcagccc agctgcccca ccacctctgc ctaccacctc ctctggcaag

2941 gctgcaaaga aacccacagc tgcagaggtc tctgttcgag tccctagagg gccggccgtg

3001 gaagggggac acgtgaatat ggcattctct cagtccaacc ctccttcgga gttgcacagg

3061 gtccacggat ccagaaacaa gcccaccagc ttgtggaacc caacgtacgg ctcctggttt

3121 acagagaaac ccaccaaaaa gaataatcct atagcaaaga aggagccaca cgagaggggt

3181 aacctggggc tggagggaag ctgtactgtc ccacctaacg ttgcaactgg gagacttccg

3241 ggggcctcac tgctcctaga gccctcttcg ctgactgcca atatgaagga ggtacctctg

3301 ttcaggctac gtcacttccc ttgtgggaat gtcaattacg gctaccagca acagggcttg

3361 cccttagaag ccgctactgc ccctggagct ggtcattacg aggataccat tctgaaaagc

3421 aagaatagca tgaaccagcc tgggccctga gctcggtcac acactcactt ctcttccttg

3481 ggatccctaa gaccgtggag gagagagagg caatcaatgg ctccttcaca aaccagagac

3541 caaatgtcac gttttgtttt gtgccaacct attttgaagt accaccaaaa aagctgtatt

3601 ttgaaaatgc tttagaaagg ttttgagcat gggttcatcc tattctttcg aaagaagaaa

3661 atatcataaa aatgagtgat aaatacaagg cccagatgtg gttgcataag gtttttatgc

3721 atgtttgttg tatacttcct tatgcttctt ttaaattgtg tgtgctctgc ttcaatgtag

3781 tcagaattag ctgcttctat gtttcatagt tggggtcata gatgtttcct tgccttgttg

3841 atgtggacat gagccatttg aggggagagg gaacggaaat aaaggagtta tttgtaatga

3901 aaaaaaaaaa aaaaaaaaaa aaaaaa

In some embodiments, the ALK-expressing cell line may include the B721.221 human lymphoblastic cell line, which does not express endogenous HLA class I (A, B and C) due to gamma-ray-induced mutations in the HLA complex. (Shimizu Y, DeMars R. Production of human cells expressing individual transferred HLA-A, -B, -C genes using an HLA-A, -B, -C null human cell line. J. Immunol. 1989;142(9):3320-3328). In some embodiments, the B721.221 cell line is transduced with a construct encoding an EML4-ALK fusion protein. In some embodiments, the construct encodes EMI,4-ALK variant 1, the most frequent EMI,4-ALK fusion protein (Lin JJ, et al. Impact of EMI,4-ALK Variant on Resistance Mechanisms and Clinical Outcomes in ALK-Positive Lung Cancer. J. Clin. Oncol. 2018:JCO2017762294). In one embodiment, the ELM4-ALK variant 1 fusion protein has an amino acid sequence from *Homo Sapiens* that is at least 85%, at least 90%, at least 95%, at least 98%, at least 99%, or 100% identical to GenBank: BAF73611.1 as provided below:

1 
61 
121 
181 
241 
301 
361 
421 
481 

541 
601 
661 
721 
781 
841 
901 
961 
1021 

In some embodiments, the fusion protein is an ALK protein fused to a nucleophosmin (NPM) protein (NPM-ALK). In some embodiments, ALK-expressing cell lines may include anaplastic large cell lymphoma (ALCL) cell lines encoding frequent HLA-alleles (*e*.*g*., Karpas-299, DEL, and SR-786). These ALCL cell lines express high levels of the NPM-ALK fusion protein. In some embodiments, the NPM-ALK fusion protein is at least 85%, at least 90%, at least 95%, at least 98%, at least 99%, or 100% identical to a NPM-ALK fusion protein in *Homo Sapiens.* In some embodiments, the NPM-ALK fusion protein is at least 85%, at least 90%, at least 95%, at least 98%, at least 99%, or 100% identical to an exemplary NPM-ALK fusion protein amino acid sequence from *Homo Sapiens* as provided below (ALK cytoplasmic portion in bold font):

In some embodiments, the NPM-ALK fusion protein is at least 85%, at least 90%, at least 95%, at least 98%, at least 99%, or 100% identical to an exemplary NPM-ALK fusion protein amino acid sequence from *Homo Sapiens* (GenBank: AAA58698.1) as provided below:

1 medsmdmdms plrpqnylfg celkadkdyh fkvdndeneh qlslrtvslg agakdelhiv
61 eaeamnyegs pikvtlatlk msvqptvslg gfeitppvvl rlkcgsgpvh isgqhlvvyr
121 rkhqelqamq melqspeykl sklrtstimt dynpnycfag ktssisdlke vprknitlir
181 glghgafgev yegqvsgmpn dpsplqvavk tlpevcseqd eldflmeali iskfnhqniv
241 rcigvslqsl prfillelma ggdlksflre trprpsqpss lamldllhva rdiacgcqyl
301 eenhfihrdi aarnclltcp gpgrvakigd fgmardiyra syyrkggcam lpvkwmppea
361 fmegiftskt dtwsfgvllw eifslgympy psksnqevle fvtsggrmdp pkncpgpvyr
421 imtqcwqhqp edrpnfaiil erieyctqdp dvintalpie ygplveeeek vpvrpkdpeg
481 vppllvsqqa kreeerspaa ppplpttssg kaakkptaae vsvrvprgpa veggh vnmaf
541 sqsnppselh kvhgsrnkpt slwnptygsw ftekptkknn piakkephdr gnlglegsct
601 vppnvatgrl pgasllleps sltanmkevp lfrlrhfpcg nvnygyqqqg lpleaatapg
661 aghyedtilk sknsmnqpgp

In some embodiments, the NPM-ALK fusion protein is at least 85%, at least 90%, at least 95%, at least 98%, at least 99%, or 100% identical to an exemplary NPM-ALK fusion protein amino acid sequence from *Homo Sapiens* as provided below:

M E D S M D M D M S P L R P Q N Y L F G
C E L K A D K D Y H F K V D N D E N E H
Q L S L R T V S L G A G A K D E L H I V
E A E A M N Y E G S P I K V T L A T L K
M S V Q P T V S L G G F E I T P P V V L
R L K C G S G P V H I S G Q H L V V Y R
R K H Q E L Q A M Q M E L Q S P E Y K L
S K L R T S T I M T D Y N P N Y C F A G
K T S S I S D L K E V P R K N I T L I R
G L G H G A F G E V Y E G Q V S G M P N
D P S P L Q V A V K T L P E V C S E Q D
E L D F L M E A L I I S K F N H Q N I V
R C I G V S L Q S L P R F I L L E L M A
G G D L K S F L R E T R P R P S Q P S S
L A M L D L L H V A R D I A C G C Q Y L
E E N H F I H R D I A A R N C L L T C P
G P G R V A K I G D F G M A R D I Y R A
S Y Y R K G G C A M L P V K W M P P E A
F M E G I F T S K T D T W S F G V L L W
E I F S L G Y M P Y P S K S N Q E V L E
F V T S G G R M D P P K N C P G P V Y R
I M T Q C W Q H Q P E D R P N F A I I L
E R I E Y C T Q D P D V I N T A L P I E
Y G P L V E E E E K V P V R P K D P E G
V P P L L V S Q Q A K R E E E R S P A A
P P P L P T T S S G K A A K K P T A A E
V S V R V P R G P A V E G G H V N M A F
S Q S N P P S E L H K V H G S R N K P T
S L W N P T Y G S W F T E K P T K K N N
P I A K K E P H D R G N L G L E G S C T
V P P N V A T G R L P G A S L L L E P S
S L T A N M K E V P L F R L R H F P C G
N V N Y G Y Q Q Q G L P L E A A T A P G

A G H Y E D T I L K S K N S M N Q P G P
*

In some embodiments, an exemplary NPM-ALK fusion protein is encoded by a nucleic acid sequence from *Homo Sapiens* that is at least 85%, at least 90%, at least 95%, at least 98%, at least 99%, or 100% identical to the sequence provided below:

1 atggaagattcgatggacatggacatgagccccctgaggccccagaactatcttttcggt 60
61 tgtgaactaaaggccgacaaagattatcactttaaggtggataatgatgaaaatgagcac 120
121 cagttatctttaagaacggtcagtttaggggctggtgcaaaggatgagttgcacattgtt 180
181 gaagcagaggcaatgaattacgaaggcagtccaattaaagtaacactggcaactttgaaa 240
241 atgtctgtacagccaacggtttcccttgggggctttgaaataacaccaccagtggtctta 300
301 aggttgaagtgtggttcagggccagtgcatattagtggacagcacttagtagtgtaccgc 360
361 cggaagcaccaggagctgcaagccatgcagatggagctgcagagccctgagtacaagctg 420
421 agcaagctccgcacctcgaccatcatgaccgactacaaccccaactactgctttgctggc 480
481 aagacctcctccatcagtgacctgaaggaggtgccgcggaaaaacatcaccctcattcgg 540
541 ggtctgggccatggcgcctttggggaggtgtatgaaggccaggtgtccggaatgcccaac 600
601 gacccaagccccctgcaagtggctgtgaagacgctgcctgaagtgtgctctgaacaggac 660
661 gaactggatttcctcatggaagccctgatcatcagcaaattcaaccaccagaacattgtt 720
721 cgctgcattggggtgagcctgcaatccctgccccggttcatcctgctggagctcatggcg 780
781 gggggagacctcaagtccttcctccgagagacccgccctcgcccgagccagccctcctcc 840
841 ctggccatgctggaccttctgcacgtggctcgggacattgcctgtggctgtcagtatttg 900
901 gaggaaaaccacttcatccaccgagacattgctgccagaaactgcctcttgacctgtcca 960
961 ggccctggaagagtggccaagattggagacttcgggatggcccgagacatctacagggcg 1020
1021 agctactatagaaagggaggctgtgccatgctgccagttaagtggatgcccccagaggcc 1080
1081 ttcatggaaggaatattcacttctaaaacagacacatggtcctttggagtgctgctatgg 1140
1141 gaaatcttttctcttggatatatgccataccccagcaaaagcaaccaggaagttctggag 1200
1201 tttgtcaccagtggaggccggatggacccacccaagaactgccctgggcctgtataccgg 1260
1261 ataatgactcagtgctggcaacatcagcctgaagacaggcccaactttgccatcattttg 1320
1321 gagaggattgaatactgcacccaggacccggatgtaatcaacaccgctttgccgatagaa 1380
1381 tatggtccacttgtggaagaggaagagaaagtgcctgtgaggcccaaggaccctgagggg 1440
1441 gttcctcctctcctggtctctcaacaggcaaaacgggaggaggagcgcagcccagctgcc 1500
1501 ccaccacctctgcctaccacctcctctggcaaggctgcaaagaaacccacagctgcagag 1560
1561 gtctctgttcgagtccctagagggccggccgtggaagggggacacgtgaatatggcattc 1620
1621 tctcagtccaaccctccttcggagttgcacaaggtccacggatccagaaacaagcccacc 1680
1681 agcttgtggaacccaacgtacggctcctggtttacagagaaacccaccaaaaagaataat 1740
1741 cctatagcaaagaaggagccacacgacaggggtaacctggggctggagggaagctgtact 1800
1801 gtcccacctaacgttgcaactgggagacttccgggggcctcactgctcctagagccctct 1860
1861 tcgctgactgccaatatgaaggaggtacctctgttcaggctacgtcacttcccttgtggg 1920
1921 aatgtcaattacggctaccagcaacagggcttgcccttagaagccgctactgcccctgga 1980
1981 gctggtcattacgaggataccattctgaaaagcaagaatagcatgaaccagcctgggccc 2040
2041 tga 2043

### ALK Immunogenic Polypeptides

The present invention features the identification of ALK antigens and immunogenic polypeptides (immunogens) with the ability to generate an immune response so as to treat a disease and its symptoms, either prophylactically or therapeutically, caused by oncogenic *ALK* gene fusions, rearrangements, duplications or mutations (*e*.*g*., ALK-positive cancers) following administration and delivery to a susceptible subject. It will also be appreciated that the isolated ALK antigen proteins as described herein and used as immunogens elicit an immune response, *e*.*g*., producing T-lymphocytes, in a subject. The ALK antigens and immunogens of the invention may be incorporated into a pharmaceutical composition, immunogenic composition, or vaccine as provided herein.

In some embodiments, the isolated ALK antigen protein elicits a protective immune response against at least one, more than one, or all types of ALK-positive cancers.

In some embodiments, the disease caused by oncogenic *ALK* gene fusions, rearrangements, duplications or mutations is an ALK-positive cancer. Nonlimiting examples of ALK-positive cancers include non-small cell lung cancer (NSCLC), anaplastic large cell lymphoma (ALCL), neuroblastoma, B-cell lymphoma, thyroid cancer, colon cancer, breast cancer, inflammatory myofibroblastic tumors (IMT), renal carcinoma, esophageal cancer, melanoma, or a combination thereof. In some embodiments, the ALK-positive cancer is non-small cell lung cancer (NSCLC). In some embodiments, the ALK-positive cancer is anaplastic large cell lymphoma (ALCL).

### ALK Antigens and Immunogens

The present invention provides herein ALK antigens and immunogens capable of generating an immune response against one or more diseases caused by oncogenic *ALK* gene fusions, rearrangements, duplications or mutations (*e*.*g*., ALK-positive cancers). An ALK antigen or immunogen as described herein is a polypeptide, peptide, or antibody-binding portion thereof. In some embodiments, the ALK antigen or immunogen is a protein or fragment thereof having an amino acid sequence that is at least 85%, at least 90%, at least 95%, at least 98%, at least 99%, or 100% identical to an anaplastic lymphoma kinase (ALK) capable of inducing an immune response in an immunized subject. In some embodiments, the ALK antigen or immunogen is at least 85%, at least 90%, at least 95%, at least 98%, at least 99%, or 100% identical to the ALK protein in *Homo Sapiens.* In some embodiments, the ALK antigen or immunogen is at least 85%, at least 90%, at least 95%, at least 98%, at least 99%, or 100% identical to the full-length amino acid sequence from *Homo Sapiens* as provided below (ALK cytoplasmic portion in bold font):

TASSGGMGAIGLLWLLPLLLSTAAVGSGMGTGQRAGSPAAGPPLQPREPLSYSRLQRKSLAVDF
VVPSLFRVYARDLLLPPSSSELKAGRPEARGSLALDCAPLLRLLGPAPGVSWTAGSPAPAEART
LSRVLKGGSVRKLRRAKQLVLELGEEAILEGCVGPPGEAAVGLLQFNLSELFSWWIRQGEGRLR
IRLMPEKKASEVGREGRLSAAIRASQPRLLFQIFGTGHSSLESPTNMPSPSPDYFTWNLTWIMK
DSFPFLSHRSRYGLECSFDFPCELEYSPPLHDLRNQSWSWRRIPSEEASQMDLLDGPGAERSKE
MPRGSFLLLNTSADSKHTILSPWMRSSSEHCTLAVSVHRHLQPSGRYIAQLLPHNEAAREILLM
PTPGKHGWTVLQGRIGRPDNPFRVALEYISSGNRSLSAVDFFALKNCSEGTSPGSKMALQSSFT
CWNGTVLQLGQACDFHQDCAQGEDESQMCRKLPVGFYCNFEDGFCGWTQGTLSPHTPQWQVRTL
KDARFQDHQDHALLLSTTDVPASESATVTSATFPAPIKSSPCELRMSWLIRGVLRGNVSLVLVE
NKTGKEQGRMVWHVAAYEGLSLWQWMVLPLLDVSDRFWLQMVAWWGQGSRAIVAFDNISISLDC
YLTISGEDKILQNTAPKSRNLFERNPNKELKPGENSPRQTPIFDPTVHWLFTTCGASGPHGPTQ
AQCNNAYQNSNLSVEVGSEGPLKGIQIWKVPATDTYSISGYGAAGGKGGKNTMMRSHGVSVLGI
FNLEKDDMLYILVGQQGEDACPSTNQLIQKVCIGENNVIEEEIRVNRSVHEWAGGGGGGGATY
VFKMKDGVPVPLIIAAGGGGRAYGAKTDTFHPERLENNSSVLGLNGNSGAAGGGGGWNDNTSLL
WAGKSLQEGATGGHSCPQAMKKWGWETRGGFGGGGGGCSSGGGGGGYIGGNAASNNDPEMDGED
GVSFISPLGILYTPALKVMEGHGEVNIKHYLNCSHCEVDECHMDPESHKVICFCDHGTVLAEDG
VSCIVSPTPEPHLPLSLILSVVTSALVAALVLAFSGIMIVYRRKHQELQAMQMELQSPEYKLSK
LRTSTIMTDYNPNYCFAGKTSSISDLKEVPRKNITLIRGLGHGAFGEVYEGQVSGMPNDPSPLQ
VAVKTLPEVCSEQDELDFLMEALIISKFNHQNIVRCIGVSLQSLPRFILLELMAGGDLKSFLRE
TRPRPSQPSSLAMLDLLHVARDIACGCQYLEENHFIHRDIAARNCLLTCPGPGRVAKIGDFGMA
RDIYRASYYRKGGCAMLPVKWMPPEAFMEGIFTSKTDTWSFGVLLWEIFSLGYMPYPSKSNQEV
LEFVTSGGRMDPPKNCPGPVYRIMTQCWQHQPEDRPNFAIILERIEYCTQDPDVINTALPIEYG
PLVEEEEKVPVRPKDPEGVPPLLVSQQAKREEERSPAAPPPLPTTSSGKAAKKPTAAEISVRVP
RGPAVEGGHVNMAFSQSNPPSELHKVHGSRNKPTSLWNPTYGSWFTEKPTKKNNPIAKKEPHDR
GNLGLEGSCTVPPNVATGRLPGASLLLEPSSLTANMKEVPLFRLRHFPCGNVNYGYQQQGLPLE
AATAPGAGHYEDTILKSKNSMNQPGP

In some embodiments, the ALK antigen or immunogen is at least 85%, at least 90%, at least 95%, at least 98%, at least 99%, or 100% identical to the full-length ALK amino acid sequence from *Homo Sapiens* as provided below:

G A A A V V A A G T S R R L C S E G R G
A P R C F P A A L W S A T Q S R G R * *

W V R R G R Q D F G R P C P E R P Q L L
P P G P L Q C L R T L R S R G A G E S K
D A A N L R S A G A G I H A Q K F S R Q
T V R S L P A A E R * L E G A Q D G S L
R P R F P P R P G R R A W R S Q K E R K
R R P G Q R A A A G S R R S Q P * K L Q
R L E A A P R G D R P Q L R L R G A G E
D G T Q L P P P F N H S S S S V P S A A
S Y R R G R G T R R G E R E A Q G P S Q
* A Q C A * V S L D S P L S F Q V C F I
* T P A R L R A V G G K Q E T C A H A Q
S S G D Q V E G A A G Y Q G L F R A S S
H L G E S E G * G W A R R A V * T A S S
G G M G A I G L L W L L P L L L S T A A
V G S G M G T G Q R A G S P A A G P P L
Q P R E P L S Y S R L Q R K S L A V D F
V V P S L F R V Y A R D L L L P P S S S
E L K A G R P E A R G S L A L D C A P L
L R L L G P A P G V S W T A G S P A P A
E A R T L S R V L K G G S V R K L R R A
K Q L V L E L G E E A I L E G C V G P P
G E A A V G L L Q F N L S E L F S W W I
R Q G E G R L R I R L M P E K K A S E V
G R E G R L S A A I R A S Q P R L L F Q
I F G T G H S S L E S P T N M P S P S P
D Y F T W N L T W I M K D S F P F L S H
R S R Y G L E C S F D F P C E L E Y S P
P L H D L R N Q S W S W R R I P S E E A
S Q M D L L D G P G A E R S K E M P R G
S F L L L N T S A D S K H T I L S P W M
R S S S E H C T L A V S V H R H L Q P S
G R Y I A Q L L P H N E A A R E I L L M
P T P G K H G W T V L Q G R I G R P D N
P F R V A L E Y I S S G N R S L S A V D
F F A L K N C S E G T S P G S K M A L Q
S S F T C W N G T V L Q L G Q A C D F H
Q D C A Q G E D E S Q M C R K L P V G F
Y C N F E D G F C G W T Q G T L S P H T
P Q W Q V R T L K D A R F Q D H Q D H A
L L L S T T D V P A S E S A T V T S A T
F P A P I K S S P C E L R M S W L I R G

V L R G N V S L V L V E N K T G K E Q G
R M V W H V A A Y E G L S L W Q W M V L
P L L D V S D R F W L Q M V A W W G Q G
S R A I V A F D N I S I S L D C Y L T I
S G E D K I L Q N T A P K S R N L F E R
N P N K E L K P G E N S P R Q T P I F D
P T V H W L F T T C G A S G P H G P T Q
A Q C N N A Y Q N S N L S V E V G S E G
P L K G I Q I W K V P A T D T Y S I S G
Y G A A G G K G G K N T M M R S H G V S
V L G I F N L E K D D M L Y I L V G Q Q
G E D A C P S T N Q L I Q K V C I G E N
N V I E E E I R V N R S V H E W A G G G
G G G G G A T Y V F K M K D G V P V P L
I I A A G G G G R A Y G A K T D T F H P
E R L E N N S S V L G L N G N S G A A G
G G G G W N D N T S L L W A G K S L Q E
G A T G G H S C P Q A M K K W G W E T R
G G F G G G G G G C S S G G G G G G Y I
G G N A A S N N D P E M D G E D G V S F
I S P L G I L Y T P A L K V M E G H G E
V N I K H Y L N C S H C E V D E C H M D
P E S H K V I C F C D H G T V L A E D G
V S C I V S P T P E P H L P L S L I L S
V V T S A L V A A L V L A F S G I M I V
Y R R K H Q E L Q A M Q M E L Q S P E Y
K L S K L R T S T I M T D Y N P N Y C F
A G K T S S I S D L K E V P R K N I T L
I R G L G H G A F G E V Y E G Q V S G M
P N D P S P L Q V A V K T L P E V C S E
Q D E L D F L M E A L I I S K F N H Q N
I V R C I G V S L Q S L P R F I L L E L
M A G G D L K S F L R E T R P R P S Q P
S S L A M L D L L H V A R D I A C G C Q
Y L E E N H F I H R D I A A R N C L L T
C P G P G R V A K I G D F G M A R D I Y
R A S Y Y R K G G C A M L P V K W M P P
E A F M E G I F T S K T D T W S F G V L
L W E I F S L G Y M P Y P S K S N Q E V
L E F V T S G G R M D P P K N C P G P V
Y R I M T Q C W Q H Q P E D R P N F A I

I L E R I E Y C T Q D P D V I N T A L P
I E Y G P L V E E E E K V P V R P K D P
E G V P P L L V S Q Q A K R E E E R S P
A A P P P L P T T S S G K A A K K P T A
A E I S V R V P R G P A V E G G H V N M
A F S Q S N P P S E L H K V H G S R N K
P T S L W N P T Y G S W F T E K P T K K
N N P I A K K E P H D R G N L G L E G S
C T V P P N V A T G R L P G A S L L L E
P S S L T A N M K E V P L F R L R H F P
C G N V N Y G Y Q Q Q G L P L E A A T A
P G A G H Y E D T I L K S K N S M N Q P
G P * A R S H T H F S S L G S L R P W R
R E R Q W L L H K P E T K C H V L F C A
N L F * S T T K K A V F * K C F R K V L
S M G S S Y S F E R R K Y H K N E * * I
Q G P D V V A * G F Y A C L L Y T S L C
F F Q I V C A L L Q C S Q N * L L L C F
I V G V I D V S L P C * C G H E P F E G
R G N G N K G V I C N D *

In some embodiments, ALK antigen or immunogen amino acid sequence comprises an amino acid sequence that is at least 95%, at least 98%, at least 99%, or 100% identical to a sequence provided in **Table 3, Table 4,** or **Table 5**. In some embodiments, the ALK antigen or immunogen comprises an amino acid sequence that is at least 95%, at least 98%, at least 99%, or 100% identical to: RPRPSQPSSL. In some embodiments, the ALK antigen or immunogen comprises an amino acid sequence that is at least 95%, at least 98%, at least 99%, or 100% identical to: IVRCIGVSL. In some embodiments, the ALK antigen or immunogen comprises an amino acid sequence that is at least 95%, at least 98%, at least 99%, or 100% identical to: VPRKNITLI. In some embodiments, the ALK antigen or immunogen comprises an amino acid sequence that is at least 95%, at least 98%, at least 99%, or 100% identical to: TAAEVSVRV. In some embodiments, the ALK antigen or immunogen comprises an amino acid sequence that is at least 95%, at least 98%, at least 99%, or 100% identical to: AMLDLLHVA.

In some embodiments, the ALK antigen or immunogen is conjugated to an amphiphile or amphiphilic tail (*see, e.g.,* **FIG. 6A****).** In some embodiments, the amphiphile is N-hydroxy succinimidyl ester-end-functionalized poly(ethylene glycol)-lipid (NHS-PEG2KDa-DSPE). ALK amph-peptides may significantly increase T-cell expansion and greatly enhance anti-tumor efficacy (*see* **Example 2).** ALK amph-peptides may be generated as taught in H. Liu et al., Structure-based programming of lymph-node targeting in molecular vaccines. Nature 507, 5199522 (2014), which is incorporated herein in its entirety.

In some embodiments, the ALK antigen or immunogen conjugated to an amphiphile or amphiphilic tail comprises an amino acid sequence from **Table 3.** In some embodiments, the ALK antigen or immunogen conjugated to an amphiphile or amphiphilic tail comprises an amino acid sequence from **Table 4.** In some embodiments, the ALK antigen or immunogen conjugated to an amphiphile or amphiphilic tail comprises an amino acid sequence from **Table 5.** In some embodiments, the ALK antigen or immunogen conjugated to an amphiphile or amphiphilic tail comprises flanking amino acid sequences. In some embodiments, the flanking amino acid sequences are on either side or on both sides of the ALK antigen or immunogen sequence. In some embodiments, the ALK antigen or immunogen conjugated to an amphiphile or amphiphilic tail comprises a central core amino acid sequence with flanking amino acid sequences on both sides of the core. In some embodiments, the core amino acid sequence is about 9 to 10 amino acids in length. In some embodiments, the flanking amino acid sequences are between 5 to 15 amino acids. In some embodiments, the ALK antigen or immunogen conjugated to an amphiphile or amphiphilic tail comprises an amino acid sequence that is about 9 to about 30 amino acids in length. In some embodiments, the ALK antigen or immunogen conjugated to an amphiphile or amphiphilic tail comprises an amino acid sequence from **Table 3** with flanking amino acid sequences.

In some embodiments, the ALK antigen or immunogen is a polynucleotide sequence. In some embodiments, the ALK antigen or immunogen is a polynucleotide sequence that encodes a polypeptide or peptide antigen or fragment thereof as described herein. In some embodiments, the nucleic acid molecule encoding an ALK polypeptide or fragment thereof (antigen or antigen protein or immunogen) is at least 85%, at least 90%, at least 95%, at least 98%, at least 99%, or 100% identical to the full-length ALK nucleic acid sequence from *Homo Sapiens* provided below:

2 gggcggcagcggtggtagcagctggtacctcccgccgcctctgttcggagggtcgcggg 61

62 gcaccgaggtgctttccggccgccctctggtcggccacccaaagccgcgggcgctgatga 121

122 tgggtgaggagggggcggcaagatttcgggcgcccctgccctgaacgccctcagctgctg 181

182 ccgccggggccgctccagtgcctgcgaactctgaggagccgaggcgccggtgagagcaag 241

242 gacgctgcaaacttgcgcagcgcgggggctgggattcacgcccagaagttcagcaggcag 301

302 acagtccgaagccttcccgcagcggagagatagcttgagggtgcgcaagacggcagcctc 361

362 cgccctcggttcccgcccagaccgggcagaagagcttggaggagccaaaaggaacgcaaa 421

422 aggcggccaggacagcgtgcagcagctgggagccgccgttctcagccttaaaagttgcag 481
482 agattggaggctgccccgagaggggacagaccccagctccgactgcggggggcaggagag 541
542 gacggtacccaactgccacctcccttcaaccatagtagttcctctgtaccgagcgcagcg 601
602 agctacagacgggggcgcggcactcggcgcggagagcgggaggctcaaggtcccagccag 661
662 tgagcccagtgtgcttgagtgtctctggactcgcccctgagcttccaggtctgtttcatt 721
722 tagactcctgctcgcctccgtgcagttgggggaaagcaagagacttgcgcgcacgcacag 781
782 tcctctggagatcaggtggaaggagccgctgggtaccaaggactgttcagagcctcttcc 841
842 catctcggggagagcgaagggtgaggctgggcccggagagcagtgtaaacggcctcctcc 901
902 ggcgggatgggagccatcgggctcctgtggctcctgccgctgctgctttccacggcagct 961
962 gtgggctccgggatggggaccggccagcgcgcgggctccccagctgcggggccgccgctg 1021
1022 cagccccgggagccactcagctactcgcgcctgcagaggaagagtctggcagttgacttc 1081
1082 gtggtgccctcgctcttccgtgtctacgcccgggacctactgctgccaccatcctcctcg 1141
1142 gagctgaaggctggcaggcccgaggcccgcggctcgctagctctggactgcgccccgctg 1201
1202 ctcaggttgctggggccggcgccgggggtctcctggaccgccggttcaccagccccggca 1261
1262 gaggcccggacgctgtccagggtgctgaagggcggctccgtgcgcaagctccggcgtgcc 1321
1322 aagcagttggtgctggagctgggcgaggaggcgatcttggagggttgcgtcgggcccccc 1381
1382 ggggaggcggctgtggggctgctccagttcaatctcagcgagctgttcagttggtggatt 1441
1442 cgccaaggcgaagggcgactgaggatccgcctgatgcccgagaagaaggcgtcggaagtg 1501
1502 ggcagagagggaaggctgtccgcggcaattcgcgcctcccagccccgccttctcttccag 1561
1562 atcttcgggactggtcatagctccttggaatcaccaacaaacatgccttctccttctcct 1621
1622 gattattttacatggaatctcacctggataatgaaagactccttccctttcctgtctcat 1681
1682 cgcagccgatatggtctggagtgcagctttgacttcccctgtgagctggagtattcccct 1741
1742 ccactgcatgacctcaggaaccagagctggtcctggcgccgcatcccctccgaggaggcc 1801
1802 tcccagatggacttgctggatgggcctggggcagagcgttctaaggagatgcccagaggc 1861
1862 tcctttctccttctcaacacctcagctgactccaagcacaccatcctgagtccgtggatg 1921
1922 aggagcagcagtgagcactgcacactggccgtctcggtgcacaggcacctgcagccctct 1981
1982 ggaaggtacattgcccagctgctgccccacaacgaggctgcaagagagatcctcctgatg 2041
2042 cccactccagggaagcatggttggacagtgctccagggaagaatcgggcgtccagacaac 2101
2102 ccatttcgagtggccctggaatacatctccagtggaaaccgcagcttgtctgcagtggac 2161
2162 ttctttgccctgaagaactgcagtgaaggaacatccccaggctccaagatggccctgcag 2221
2222 agctccttcacttgttggaatgggacagtcctccagcttgggcaggcctgtgacttccac 2281
2282 caggactgtgcccagggagaagatgagagccagatgtgccggaaactgcctgtgggtttt 2341
2342 tactgcaactttgaagatggcttctgtggctggacccaaggcacactgtcaccccacact 2401
2402 cctcaatggcaggtcaggaccctaaaggatgcccggttccaggaccaccaagaccatgct 2461
2462 ctattgctcagtaccactgatgtccccgcttctgaaagtgctacagtgaccagtgctacg 2521
2522 tttcctgcaccgatcaagagctctccatgtgagctccgaatgtcctggctcattcgtgga 2581
2582 gtcttgaggggaaacgtgtccttggtgctagtggagaacaaaaccgggaaggagcaaggc 2641
2642 aggatggtctggcatgtcgccgcctatgaaggcttgagcctgtggcagtggatggtgttg 2701
2702 cctctcctcgatgtgtctgacaggttctggctgcagatggtcgcatggtggggacaagga 2761
2762 tccagagccatcgtggcttttgacaatatctccatcagcctggactgctacctcaccatt 2821
2822 agcggagaggacaagatcctgcagaatacagcacccaaatcaagaaacctgtttgagaga 2881

2882 aacccaaacaaggagctgaaacccggggaaaattcaccaagacagacccccatctttgac 2941
2942 cctacagttcattggctgttcaccacatgtggggccagcgggccccatggccccacccag 3001
3002 gcacagtgcaacaacgcctaccagaactccaacctgagcgtggaggtggggagcgagggc 3061
3062 cccctgaaaggcatccagatctggaaggtgccagccaccgacacctacagcatctcgggc 3121
3122 tacggagctgctggcgggaaaggcgggaagaacaccatgatgcggtcccacggcgtgtct 3181
3182 gtgctgggcatcttcaacctggagaaggatgacatgctgtacatcctggttgggcagcag 3241
3242 ggagaggacgcctgccccagtacaaaccagttaatccagaaagtctgcattggagagaac 3301
3302 aatgtgatagaagaagaaatccgtgtgaacagaagcgtgcatgagtgggcaggaggcgga 3361
3362 ggaggagggggtggagccacctacgtatttaagatgaaggatggagtgccggtgcccctg 3421
3422 atcattgcagccggaggtggtggcagggcctacggggccaagacagacacgttccaccca 3481
3482 gagagactggagaataactcctcggttctagggctaaacggcaattccggagccgcaggt 3541
3542 ggtggaggtggctggaatgataacacttccttgctctgggccggaaaatctttgcaggag 3601
3602 ggtgccaccggaggacattcctgcccccaggccatgaagaagtggggggtggagacaaga 3661
3662 gggggtttcggagggggtggaggggggtgctcctcaggtggaggaggcggaggatatata 3721
3722 ggcggcaatgcagcctcaaacaatgaccccgaaatggatggggaagatggggtttccttc 3781
3782 atcagtccactgggcatcctgtacaccccagctttaaaagtgatggaaggccacggggaa 3841
3842 gtgaatattaagcattatctaaactgcagtcactgtgaggtagacgaatgtcacatggac 3901
3902 cctgaaagccacaaggtcatctgcttctgtgaccacgggacggtgctggctgaggatggc 3961
3962 gtctcctgcattgtgtcacccaccccggagccacacctgccactctcgctgatcctctct 4021
4022 gtggtgacctctgccctcgtggccgccctggtcctggctttctccggcatcatgattgtg 4081
4082 taccgccggaagcaccaggagctgcaagccatgcagatggagctgcagagccctgagtac 4141
4142 aagctgagcaagctccgcacctcgaccatcatgaccgactacaaccccaactactgcttt 4201
4202 gctggcaagacctcctccatcagtgacctgaaggaggtgccgcggaaaaacatcaccctc 4261
4262 attcggggtctgggccatggcgcctttggggaggtgtatgaaggccaggtgtccggaatg 4321
4322 cccaacgacccaagccccctgcaagtggctgtgaagacgctgcctgaagtgtgctctgaa 4381
4382 caggacgaactggatttcctcatggaagccctgatcatcagcaaattcaaccaccagaac 4441
4442 attgttcgctgcattggggtgagcctgcaatccctgccccggttcatcctgctggagctc 4501
4502 atggcggggggagacctcaagtccttcctccgagagacccgccctcgcccgagccagccc 4561
4562 tcctccctggccatgctggaccttctgcacgtggctcgggacattgcctgtggctgtcag 4621
4622 tatttggaggaaaaccacttcatccaccgagacattgctgccagaaactgcctcttgacc 4681
4682 tgtccaggccctggaagagtggccaagattggagacttcgggatggcccgagacatctac 4741
4742 agggcgagctactatagaaagggaggctgtgccatgctgccagttaagtggatgccccca 4801
4802 gaggccttcatggaaggaatattcacttctaaaacagacacatggtcctttggagtgctg 4861
4862 ctatgggaaatcttttctcttggatatatgccataccccagcaaaagcaaccaggaagtt 4921
4922 ctggagtttgtcaccagtggaggccggatggacccacccaagaactgccctgggcctgta 4981
4982 taccggataatgactcagtgctggcaacatcagcctgaagacaggcccaactttgccatc 5041
5042 attttggagaggattgaatactgcacccaggacccggatgtaatcaacaccgctttgccg 5101
5102 atagaatatggtccacttgtggaagaggaagagaaagtgcctgtgaggcccaaggaccct 5161
5162 gagggggttcctcctctcctggtctctcaacaggcaaaacgggaggaggagcgcagccca 5221
5222 gctgccccaccacctctgcctaccacctcctctggcaaggctgcaaagaaacccacagct 5281
5282 gcagagatctctgttcgagtccctagagggccggccgtggaagggggacacgtgaatatg 5341

5342 gcattctctcagtccaaccctccttcggagttgcacaaggtccacggatccagaaacaag 5401

5402 cccaccagcttgtggaacccaacgtacggctcctggtttacagagaaacccaccaaaaag 5461

5462 aataatcctatagcaaagaaggagccacacgacaggggtaacctggggctggagggaagc 5521

5522 tgtactgtcccacctaacgttgcaactgggagacttccgggggcctcactgctcctagag 5581

5582 ccctcttcgctgactgccaatatgaaggaggtacctctgttcaggctacgtcacttccct 5641

5642 tgtgggaatgtcaattacggctaccagcaacagggcttgcccttagaagccgctactgcc 5701

5702 cctggagctggtcattacgaggataccattctgaaaagcaagaatagcatgaaccagcct 5761

5762 gggccctgagctcggtcgcacactcacttctcttccttgggatccctaagaccgtggagg 5821

5822 agagagaggcaatggctccttcacaaaccagagaccaaatgtcacgttttgttttgtgcc 5881

5882 aacctattttgaagtaccaccaaaaaagctgtattttgaaaatgctttagaaaggttttg 5941

5942 agcatgggttcatcctattctttcgaaagaagaaaatatcataaaaatgagtgataaata 6001

6002 caaggcccagatgtggttgcataaggtttttatgcatgtttgttgtatacttccttatgc 6061

6062 ttctttcaaattgtgtgtgctctgcttcaatgtagtcagaattagctgcttctatgtttc 6121

6122 atagttggggtcatagatgtttccttgccttgttgatgtggacatgagccatttgagggg 6181

6182 agagggaacggaaataaaggagttatttgtaatgactaaaa 6222

In some embodiments, ALK polynucleotide sequences encode ALK antigen or immunogen amino acid sequences that are at least 95%, at least 98%, at least 99%, or 100% identical to the sequences provided in **Table 3, Table 4,** and **Table 5**. In some embodiments, the ALK polynucleotide sequence encodes the ALK antigen or immunogen amino acid sequence that is at least 95%, at least 98%, at least 99%, or 100% identical to: RPRPSQPSSL. In some embodiments, the ALK polynucleotide sequence encodes the ALK antigen or immunogen amino acid sequence that is at least 95%, at least 98%, at least 99%, or 100% identical to: IVRCIGVSL. In some embodiments, the ALK polynucleotide sequence encodes the ALK antigen or immunogen amino acid sequence that is at least 95%, at least 98%, at least 99%, or 100% identical to: VPRKNITLI. In some embodiments, the ALK polynucleotide sequence encodes the ALK antigen or immunogen amino acid sequence that is at least 95%, at least 98%, at least 99%, or 100% identical to: TAAEVSVRV. In some embodiments, the ALK polynucleotide sequence encodes the ALK antigen or immunogen amino acid sequence that is at least 95%, at least 98%, at least 99%, or 100% identical to: AMLDLLHVA.

In some embodiments, the amino acid sequence of the antigen or immunogen, e.g., the ALK protein, is reverse translated and optimized for expression in mammalian cells. As will be appreciated by a skilled practitioner in the art, optimization of the nucleic acid sequence includes optimization of the codons for expression of a sequence in mammalian cells and RNA optimization (such as RNA stability).

In some embodiments, the ALK antigen or immunogen is isolated and/or purified. In some embodiments, the antigen or immunogen is formulated for administration to a subject in need. In some embodiments, the antigen or immunogen is administered to a subject in need thereof in an effective amount to elicit an immune response (*e.g.,* a T-cell response) in the subject. In some embodiments, the immune response produces T-lymphocytes. In some embodiments, the immune response is prophylactic or therapeutic.

In some embodiments, fusion proteins comprising the ALK antigen polypeptides are described herein. In some embodiments, the ALK polypeptide can be fused to any heterologous amino acid sequence to form the fusion protein. By way of example, peptide components of ALK polypeptides may be generated independently and then fused together to produce an intact ALK polypeptide antigen, for use as an immunogen.

### ALK Immunogenic Compositions and Vaccines

The ALK antigens or immunogens may be used in immunogenic compositions or vaccines to elicit an immune response, *e.g.,* a T-cell response, against disease caused by oncogenic *ALK* gene fusions, rearrangements, duplications or mutations *(e.g.,* ALK-positive cancers). In some embodiments, the immune response includes producing T-lymphocytes.

In particular embodiments, the ALK polypeptides of the immunogenic compositions or vaccines contain antigenic determinants that serve to elicit an immune response in a subject (e.g., the production of activated T-cells) that can treat and/or protect a subject against disease caused by oncogenic *ALK* gene fusions, rearrangements, duplications or mutations *(e.g.,* ALK-positive cancers) and symptoms thereof.

In some embodiments, such immunogenic compositions or vaccines as described herein contain at least one ALK antigen or immunogen and are effective in treating, reducing, delaying, or preventing at least one disease caused by oncogenic ALK gene fusions, rearrangements, duplications or mutations (*e*.*g*., ALK-positive cancers). In some embodiments, such immunogenic compositions or vaccines as described herein contain two or more ALK antigens or immunogens and are effective in treating, reducing, or preventing at least one disease caused by oncogenic ALK gene fusions, rearrangements, duplications or mutations (*e*.*g*., ALK-positive cancers). In some embodiments, the two or more ALK antigens or immunogens comprise two or more amino acid sequences selected from the following: AMLDLLHVA; RPRPSQPSSL; IVRCIGVSL; VPRKNITLI; and/or TAAEVSVRV. In some embodiments, the two or more ALK antigens or immunogens comprise two or more amino acid sequences selected from **Table 3, Table 4** and/or **Table 5**. In some embodiments, the immunogenic compositions or vaccines contain at least one ALK antigen or immunogen conjugated to an amphiphile or amphiphilic tail (*see, e.g.* **FIG. 6A**). In some embodiments, at least one of the two or more ALK antigens or immunogens in an immunogenic composition or vaccine is conjugated to an amphiphile or amphiphilic tail. In some embodiments, the amphiphile is N-hydroxy succinimidyl ester-end-functionalized poly(ethylene glycol)-lipid (NHS-PEG2KDa-DSPE). In some embodiments, the two or more ALK antigens or immunogens are provided in equal concentration ratios in an immunogenic composition or vaccine.

Because the ALK antigens or immunogens and the sequences thereof as described herein and used as immunogenic compositions or vaccines, elicit an immune response in an immunocompetent subject, they provide a superior vaccine against which an immune response (*e*.*g*., producing T-lymphocytes) is generated.

In some embodiments, an immunogenic composition or a vaccine is provided that elicits an immune response (*e*.*g*., producing T-lymphocytes) in a subject following introduction, administration, or delivery of the antigen or immunogen to the subject. The route of introduction, administration, or delivery is not limited and may include, for example, intravenous, subcutaneous, intramuscular, oral, or other routes. The immunogenic composition or vaccine may be therapeutic (*e*.*g*., administered to a subject following a symptom of disease caused by oncogenic *ALK* gene fusions, rearrangements, duplications or mutations (*e.g.,* ALK-positive cancers)) or prophylactic (*e*.*g*., administered to a subject prior to the subject having or expressing a symptom of disease, or full-blown disease, caused by oncogenic *ALK* gene fusions, rearrangements, duplications or mutations (*e*.*g*., ALK-positive cancers)).

### Vectors

Vectors containing a nucleotide sequence encoding an isolated ALK polypeptide or peptide antigen are provided. In some embodiments, the vectors comprise a nucleotide sequence encoding the full-length ALK polypeptide or peptide antigen that is at least 85%, at least 90%, at least 95%, at least 96%, at least 97%, at least 98%, at least 99%, or 100% identical to a *Homo Sapiens* full-length ALK polypeptide as provided herein. In some embodiments, the vectors comprise a nucleotide sequence encoding the ALK polypeptide or peptide antigen that is at least 95%, at least 96%, at least 97%, at least 98%, at least 99%, or 100% identical to a polynucleotide encoding at least one ALK polypeptide sequence provided in **Table 3, Table 4** or **Table 5.** In some embodiments, the vector further includes a promoter operably linked to the nucleotide sequence encoding the ALK polypeptide. In a particular embodiment, the promoter is a cytomegalovirus (CMV) promoter. In some embodiments, the nucleotide sequence of the vector is at least 85%, at least 90%, at least 95%, at least 98%, at least 99%, or 100% identical to the polynucleotide sequence provided below. In some embodiments, the nucleotide sequence of the vector is at least 85%, at least 90%, at least 95%, at least 98%, at least 99%, or 100% identical to the *Homo Sapiens* full-length ALK nucleic acid sequence provided below.

2 ggggcggcagcggtggtagcagctggtacctcccgccgcctctgttcggagggtcgcggg 61
62 gcaccgaggtgctttccggccgccctctggtcggccacccaaagccgcgggcgctgatga 121
122 tgggtgaggagggggcggcaagatttcgggcgcccctgccctgaacgccctcagctgctg 181
182 ccgccggggccgctccagtgcctgcgaactctgaggagccgaggcgccggtgagagcaag 241
242 gacgctgcaaacttgcgcagcgcgggggctgggattcacgcccagaagttcagcaggcag 301
302 acagtccgaagccttcccgcagcggagagatagcttgagggtgcgcaagacggcagcctc 361
362 cgccctcggttcccgcccagaccgggcagaagagcttggaggagccaaaaggaacgcaaa 421
422 aggcggccaggacagcgtgcagcagctgggagccgccgttctcagccttaaaagttgcag 481
482 agattggaggctgccccgagaggggacagaccccagctccgactgcggggggcaggagag 541
542 gacggtacccaactgccacctcccttcaaccatagtagttcctctgtaccgagcgcagcg 601
602 agctacagacgggggcgcggcactcggcgcggagagcgggaggctcaaggtcccagccag 661
662 tgagcccagtgtgcttgagtgtctctggactcgcccctgagcttccaggtctgtttcatt 721
722 tagactcctgctcgcctccgtgcagttgggggaaagcaagagacttgcgcgcacgcacag 781
782 tcctctggagatcaggtggaaggagccgctgggtaccaaggactgttcagagcctcttcc 841
842 catctcggggagagcgaagggtgaggctgggcccggagagcagtgtaaacggcctcctcc 901
902 ggcgggatgggagccatcgggctcctgtggctcctgccgctgctgctttccacggcagct 961
962 gtgggctccgggatggggaccggccagcgcgcgggctccccagctgcggggccgccgctg 1021
1022 cagccccgggagccactcagctactcgcgcctgcagaggaagagtctggcagttgacttc 1081
1082 gtggtgccctcgctcttccgtgtctacgcccgggacctactgctgccaccatcctcctcg 1141
1142 gagctgaaggctggcaggcccgaggcccgcggctcgctagctctggactgcgccccgctg 1201
1202 ctcaggttgctggggccggcgccgggggtctcctggaccgccggttcaccagccccggca 1261
1262 gaggcccggacgctgtccagggtgctgaagggcggctccgtgcgcaagctccggcgtgcc 1321
1322 aagcagttggtgctggagctgggcgaggaggcgatcttggagggttgcgtcgggcccccc 1381
1382 ggggaggcggctgtggggctgctccagttcaatctcagcgagctgttcagttggtggatt 1441
1442 cgccaaggcgaagggcgactgaggatccgcctgatgcccgagaagaaggcgtcggaagtg 1501
1502 ggcagagagggaaggctgtccgcggcaattcgcgcctcccagccccgccttctcttccag 1561
1562 atcttcgggactggtcatagctccttggaatcaccaacaaacatgccttctccttctcct 1621
1622 gattattttacatggaatctcacctggataatgaaagactccttccctttcctgtctcat 1681
1682 cgcagccgatatggtctggagtgcagctttgacttcccctgtgagctggagtattcccct 1741
1742 ccactgcatgacctcaggaaccagagctggtcctggcgccgcatcccctccgaggaggcc 1801
1802 tcccagatggacttgctggatgggcctggggcagagcgttctaaggagatgcccagaggc 1861
1862 tcctttctccttctcaacacctcagctgactccaagcacaccatcctgagtccgtggatg 1921
1922 aggagcagcagtgagcactgcacactggccgtctcggtgcacaggcacctgcagccctct 1981
1982 ggaaggtacattgcccagctgctgccccacaacgaggctgcaagagagatcctcctgatg 2041
2042 cccactccagggaagcatggttggacagtgctccagggaagaatcgggcgtccagacaac 2101
2102 ccatttcgagtggccctggaatacatctccagtggaaaccgcagcttgtctgcagtggac 2161
2162 ttctttgccctgaagaactgcagtgaaggaacatccccaggctccaagatggccctgcag 2221
2222 agctccttcacttgttggaatgggacagtcctccagcttgggcaggcctgtgacttccac 2281

2282 caggactgtgcccagggagaagatgagagccagatgtgccggaaactgcctgtgggtttt 2341
2342 tactgcaactttgaagatggcttctgtggctggacccaaggcacactgtcaccccacact 2401
2402 cctcaatggcaggtcaggaccctaaaggatgcccggttccaggaccaccaagaccatgct 2461
2462 ctattgctcagtaccactgatgtccccgcttctgaaagtgctacagtgaccagtgctacg 2521
2522 tttcctgcaccgatcaagagctctccatgtgagctccgaatgtcctggctcattcgtgga 2581
2582 gtcttgaggggaaacgtgtccttggtgctagtggagaacaaaaccgggaaggagcaaggc 2641
2642 aggatggtctggcatgtcgccgcctatgaaggcttgagcctgtggcagtggatggtgttg 2701
2702 cctctcctcgatgtgtctgacaggttctggctgcagatggtcgcatggtggggacaagga 2761
2762 tccagagccatcgtggcttttgacaatatctccatcagcctggactgctacctcaccatt 2821
2822 agcggagaggacaagatcctgcagaatacagcacccaaatcaagaaacctgtttgagaga 2881
2882 aacccaaacaaggagctgaaacccggggaaaattcaccaagacagacccccatctttgac 2941
2942 cctacagttcattggctgttcaccacatgtggggccagcgggccccatggccccacccag 3001
3002 gcacagtgcaacaacgcctaccagaactccaacctgagcgtggaggtggggagcgagggc 3061
3062 cccctgaaaggcatccagatctggaaggtgccagccaccgacacctacagcatctcgggc 3121
3122 tacggagctgctggcgggaaaggcgggaagaacaccatgatgcggtcccacggcgtgtct 3181
3182 gtgctgggcatcttcaacctggagaaggatgacatgctgtacatcctggttgggcagcag 3241
3242 ggagaggacgcctgccccagtacaaaccagttaatccagaaagtctgcattggagagaac 3301
3302 aatgtgatagaagaagaaatccgtgtgaacagaagcgtgcatgagtgggcaggaggcgga 3361
3362 ggaggagggggtggagccacctacgtatttaagatgaaggatggagtgccggtgcccctg 3421
3422 atcattgcagccggaggtggtggcagggcctacggggccaagacagacacgttccaccca 3481
3482 gagagactggagaataactcctcggttctagggctaaacggcaattccggagccgcaggt 3541
3542 ggtggaggtggctggaatgataacacttccttgctctgggccggaaaatctttgcaggag 3601
3602 ggtgccaccggaggacattcctgcccccaggccatgaagaagtgggggtgggagacaaga 3661
3662 gggggtttcggagggggtggaggggggtgctcctcaggtggaggaggcggaggatatata 3721
3722 ggcggcaatgcagcctcaaacaatgaccccgaaatggatggggaagatggggtttccttc 3781
3782 atcagtccactgggcatcctgtacaccccagctttaaaagtgatggaaggccacggggaa 3841
3842 gtgaatattaagcattatctaaactgcagtcactgtgaggtagacgaatgtcacatggac 3901
3902 cctgaaagccacaaggtcatctgcttctgtgaccacgggacggtgctggctgaggatggc 3961
3962 gtctcctgcattgtgtcacccaccccggagccacacctgccactctcgctgatcctctct 4021
4022 gtggtgacctctgccctcgtggccgccctggtcctggctttctccggcatcatgattgtg 4081
4082 taccgccggaagcaccaggagctgcaagccatgcagatggagctgcagagccctgagtac 4141
4142 aagctgagcaagctccgcacctcgaccatcatgaccgactacaaccccaactactgcttt 4201
4202 gctggcaagacctcctccatcagtgacctgaaggaggtgccgcggaaaaacatcaccctc 4261
4262 attcggggtctgggccatggcgcctttggggaggtgtatgaaggccaggtgtccggaatg 4321
4322 cccaacgacccaagccccctgcaagtggctgtgaagacgctgcctgaagtgtgctctgaa 4381
4382 caggacgaactggatttcctcatggaagccctgatcatcagcaaattcaaccaccagaac 4441
4442 attgttcgctgcattggggtgagcctgcaatccctgccccggttcatcctgctggagctc 4501
4502 atggcggggggagacctcaagtccttcctccgagagacccgccctcgcccgagccagccc 4561
4562 tcctccctggccatgctggaccttctgcacgtggctcgggacattgcctgtggctgtcag 4621
4622 tatttggaggaaaaccacttcatccaccgagacattgctgccagaaactgcctcttgacc 4681
4682 tgtccaggccctggaagagtggccaagattggagacttcgggatggcccgagacatctac 4741

4742 agggcgagctactatagaaagggaggctgtgccatgctgccagttaagtggatgccccca 4801
4802 gaggccttcatggaaggaatattcacttctaaaacagacacatggtcctttggagtgctg 4861
4862 ctatgggaaatcttttctcttggatatatgccataccccagcaaaagcaaccaggaagtt 4921
4922 ctggagtttgtcaccagtggaggccggatggacccacccaagaactgccctgggcctgta 4981
4982 taccggataatgactcagtgctggcaacatcagcctgaagacaggcccaactttgccatc 5041
5042 attttggagaggattgaatactgcacccaggacccggatgtaatcaacaccgctttgccg 5101
5102 atagaatatggtccacttgtggaagaggaagagaaagtgcctgtgaggcccaaggaccct 5161
5162 gagggggttcctcctctcctggtctctcaacaggcaaaacgggaggaggagcgcagccca 5221
5222 gctgccccaccacctctgcctaccacctcctctggcaaggctgcaaagaaacccacagct 5281
5282 gcagagatctctgttcgagtccctagagggccggccgtggaagggggacacgtgaatatg 5341
5342 gcattctctcagtccaaccctccttcggagttgcacaaggtccacggatccagaaacaag 5401
5402 cccaccagcttgtggaacccaacgtacggctcctggtttacagagaaacccaccaaaaag 5461
5462 aataatcctatagcaaagaaggagccacacgacaggggtaacctggggctggagggaagc 5521
5522 tgtactgtcccacctaacgttgcaactgggagacttccgggggcctcactgctcctagag 5581
5582 ccctcttcgctgactgccaatatgaaggaggtacctctgttcaggctacgtcacttccct 5641
5642 tgtgggaatgtcaattacggctaccagcaacagggcttgcccttagaagccgctactgcc 5701
5702 cctggagctggtcattacgaggataccattctgaaaagcaagaatagcatgaaccagcct 5761
5762 gggccctgagctcggtcgcacactcacttctcttccttgggatccctaagaccgtggagg 5821
5822 agagagaggcaatggctccttcacaaaccagagaccaaatgtcacgttttgttttgtgcc 5881
5882 aacctattttgaagtaccaccaaaaaagctgtattttgaaaatgctttagaaaggttttg 5941
5942 agcatgggttcatcctattctttcgaaagaagaaaatatcataaaaatgagtgataaata 6001
6002 caaggcccagatgtggttgcataaggtttttatgcatgtttgttgtatacttccttatgc 6061
6062 ttctttcaaattgtgtgtgctctgcttcaatgtagtcagaattagctgcttctatgtttc 6121
6122 atagttggggtcatagatgtttccttgccttgttgatgtggacatgagccatttgagggg 6181
6182 agagggaacggaaataaaggagttatttgtaatgactaaaa 6222

The vectors used to express an ALK antigen as described herein may be any suitable expression vector known and used in the art. In some embodiments, the vector is a prokaryotic or eukaryotic vector. In some embodiments, the vector is an expression vector, such as a eukaryotic (*e*.*g*., mammalian) expression vector. In another embodiment, the vector is a plasmid (prokaryotic or bacterial) vector. In another embodiment, the vector is a viral vector.

Provided are isolated, non-naturally occurring polypeptide antigens, *e.g*., ALK polypeptide antigens, produced by transfecting a host cell with an expression vector as known and used in the art under conditions sufficient to allow for expression of the polypeptide, *e*.*g*., an ALK polypeptide, in the cell. Isolated cells containing the vectors are also provided.

Also provided is an ALK polypeptide, as described herein, produced by transfecting a host cell with a vector containing a polynucleotide encoding the ALK polypeptide. Also provided in some embodiments is an ALK polypeptide, as described herein, produced by transfecting a host cell with a vector encoding the ALK polypeptide under conditions sufficient to allow for expression of the ALK protein. Collections of plasmids (vectors) are also contemplated. In certain embodiments, the collection of plasmids includes plasmid encoding an ALK protein as described herein.

### Compositions and Pharmaceutical Compositions for Administration

Compositions comprising at least one ALK protein, or a polynucleotide encoding at least one ALK protein, as described herein are provided. In some embodiments, the compositions further comprise a pharmaceutically acceptable carrier, diluent, excipient, or vehicle. In some embodiments, an adjuvant (a pharmacological or immunological agent that modifies or boosts an immune response, *e*.*g*., to produce more antibodies that are longer-lasting) is also employed. For example, without limitation, the adjuvant can be an inorganic compound, such as alum, aluminum hydroxide, or aluminum phosphate; mineral or paraffin oil; squalene; detergents such as Quil A; plant saponins; Freund's complete or incomplete adjuvant, a biological adjuvant (*e.g.,* cytokines such as IL-1, IL-2, or IL-12); bacterial products such as killed *Bordetella pertussis,* or toxoids; or immunostimulatory oligonucleotides (such as CpG oligonucleotides). In some embodiments, the adjuvant is conjugated to an amphiphile as previously described (H. Liu et al., Structure-based programming of lymph-node targeting in molecular vaccines. Nature 507, 5199522 (2014)). In some embodiments, the amphiphile is N-hydroxy succinimidyl ester-end-functionalized poly(ethylene glycol)-lipid (NHS-PEG2KDa-DSPE)

Compositions and preparations (*e.g.,* physiologically or pharmaceutically acceptable compositions) containing ALK polypeptides or polynucleotides for parenteral administration include, without limitation, sterile aqueous or non-aqueous solutions, suspensions, and emulsions. Nonlimiting examples of non-aqueous solvents include propylene glycol, polyethylene glycol, vegetable oils, such as olive oil and canola oil, and injectable organic esters, such as ethyl oleate. Aqueous carriers include water, alcoholic/aqueous solutions, emulsions or suspensions, including saline and buffered media. Parenteral vehicles include, for example, sodium chloride solution, Ringer's dextrose, dextrose and sodium chloride, lactated Ringer's, or fixed oils. Intravenous vehicles include, for example, fluid and nutrient replenishers, electrolyte replenishers (such as those based on Ringer's dextrose), and the like. Preservatives and other additives may also be present in such compositions and preparations, such as, for example, antimicrobials, antioxidants, chelating agents, colorants, stabilizers, inert gases and the like.

Some of the compositions may potentially be administered as a pharmaceutically acceptable acid- or base-addition salt, formed by reaction with inorganic acids, such as hydrochloric acid, hydrobromic acid, perchloric acid, nitric acid, thiocyanic acid, sulfuric acid, and phosphoric acid, and organic acids, such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, tri-alkyl and aryl amines and substituted ethanolamines.

Provided herein are pharmaceutical compositions which include a therapeutically effective amount of an isolated ALK polypeptide or polynucleotide antigen, alone, or in combination with a pharmaceutically acceptable carrier. Pharmaceutically acceptable carriers include, but are not limited to, saline, buffered saline, dextrose, water, glycerol, ethanol, and combinations thereof. The carrier and composition can be sterile, and the formulation suits the mode of administration. The composition can also contain minor amounts of wetting or emulsifying agents, or pH buffering agents. The composition can be a liquid or aqueous solution, suspension, emulsion, dispersion, tablet, pill, capsule, powder, or sustained release formulation. A liquid or aqueous composition can be lyophilized and reconstituted with a solution or buffer prior to use. The composition can be formulated as a suppository, with traditional binders and carriers such as triglycerides. Oral formulations can include standard carriers, such as pharmaceutical grades of mannitol, lactose, starch, magnesium stearate, sodium saccharine, cellulose, and magnesium carbonate. Any of the commonly known pharmaceutical carriers, such as sterile saline solution or sesame oil, can be used. The medium can also contain conventional pharmaceutical adjunct materials such as, for example, pharmaceutically acceptable salts to adjust the osmotic pressure, buffers, preservatives and the like. Other media that can be used in the compositions and administration methods as described are normal saline and sesame oil.

### Methods of Treatment, Administration and Delivery

Methods of treating a disease, or symptoms thereof, caused by the oncogenic *ALK* gene fusions, rearrangements, duplications or mutations (*e.g.,* ALK-positive cancers) are provided. The methods comprise administering a therapeutically effective amount of an antigen, immunogen, immunogenic composition, or vaccine, as described herein, or a pharmaceutical composition comprising the immunogen or a vaccine, as described herein, to a subject (*e.g.,* a mammal), in particular, a human subject. The invention provides methods of treating a subject suffering from, or at risk of, or susceptible to disease, or a symptom thereof, or delaying the progression of a disease caused by oncogenic *ALK* gene fusions, rearrangements, duplications or mutations (*e.g.,* ALK-positive cancers). In some embodiments, the method includes administering to the subject *(e.g.,* a mammalian subject), an amount or a therapeutic amount of an immunogenic composition or a vaccine comprising at least one ALK antigen polypeptide, sufficient to treat the disease, delay the growth of, or treat the symptoms thereof, caused by the oncogenic *ALK* gene under conditions in which the disease and/or the symptoms thereof are treated.

In some embodiments, the methods herein include administering to the subject (including a human subject identified as in need of such treatment) an effective amount of an isolated, ALK antigen or immunogen polypeptide, or an immunogenic composition or vaccine, or a pharmaceutical composition thereof, as described herein to produce such effect. The treatment methods are suitably administered to subjects, particularly humans, suffering from, are susceptible to, or at risk of having a disease, or symptoms thereof, caused by oncogenic *ALK* gene fusions, rearrangements, duplications or mutations, namely, ALK-positive cancers. Nonlimiting examples of ALK-positive cancers include non-small cell lung cancer (NSCLC), anaplastic large cell lymphoma (ALCL), neuroblastoma, B-cell lymphoma, thyroid cancer, colon cancer, breast cancer, inflammatory myofibroblastic tumors (IMT), renal carcinoma, esophageal cancer, melanoma, or a combination thereof.

Identifying a subject in need of such treatment can be based on the judgment of the subject or of a health care professional and can be subjective *(e.g.,* opinion) or objective (*e.g.,* measurable by a test or diagnostic method). Briefly, the determination of those subjects who are in need of treatment or who are "at risk" or "susceptible" can be made by any objective or subjective determination by a diagnostic test (*e.g.,* blood sample, biopsy, genetic test, enzyme or protein marker assay), marker analysis, family history, and the like, including an opinion of the subject or a health care provider. In some embodiments, the subject in need of treatment can be identified by measuring ALK specific autoantibodies and ALK-specific T-cell responses in a patient sample (*e.g.,* blood sample) or by assessing infiltrating immune cell subsets from a tumor core biopsy from a subject *(see* **Example 3**). The ALK antigens and immunogens, such as ALK polypeptides, immunogenic compositions and vaccines as described herein, may also be used in the treatment of any other disorders in which disease caused by oncogenic *ALK* gene fusions, rearrangements, duplications or mutations may be implicated. A subject undergoing treatment can be a non-human mammal, such as a veterinary subject, or a human subject (also referred to as a "patient").

In addition, prophylactic methods of preventing or protecting against a disease, or symptoms thereof, caused by oncogenic *ALK* gene fusions, rearrangements, duplications or mutations are provided. Such methods comprise administering a therapeutically effective amount of a pharmaceutical composition comprising an ALK immunogenic composition or vaccine as described herein to a subject *(e.g.,* a mammal, such as a human), in particular, prior to development or onset of a disease, such as ALK-positive tumors or cancers.

In another embodiment, a method of monitoring the progress of a disease caused by oncogenic *ALK* gene fusions, rearrangements, duplications or mutations (*e.g.,* ALK-positive cancers), or monitoring treatment of the disease is provided. The method includes a diagnostic measurement (*e.g.,* CT scan, screening assay or detection assay) in a subject suffering from or susceptible to disease or symptoms thereof associated with oncogenic *ALK* gene fusions, rearrangements, duplications or mutations (*e.g.,* ALK-positive cancers), in which the subject has been administered an amount *(*e.g., a therapeutic amount) of an isolated ALK protein, as described herein, or an immunogenic composition or vaccine as described herein, sufficient to treat the disease or symptoms thereof. The diagnostic measurement in the method can be compared to samples from healthy, normal controls; in a pre-disease sample of the subject; or in other afflicted/diseased patients to establish the treated subject's disease status. For monitoring, a second diagnostic measurement may be obtained from the subject at a time point later than the determination of the first diagnostic measurement, and the two measurements can be compared to monitor the course of disease or the efficacy of the therapy/treatment. In certain embodiments, a pre-treatment measurement in the subject (*e.g.,* in a sample or biopsy obtained from the subject or CT scan) is determined prior to beginning treatment as described; this measurement can then be compared to a measurement in the subject after the treatment commences and/or during the course of treatment to determine the efficacy of (monitor the efficacy of) the disease treatment. In some embodiments, efficacy of the disease treatment can be performed with antibody marker analysis and/or interferon-gamma (IFN-γ) ELISPOT assays.

The isolated ALK antigen polypeptide, or compositions thereof, can be administered to a subject by any of the routes normally used for introducing a recombinant protein or composition containing the recombinant protein into a subject. Routes and methods of administration include, without limitation, intradermal, intramuscular, intraperitoneal, intrathecal, parenteral, such as intravenous (IV) or subcutaneous (SC), vaginal, rectal, intranasal, inhalation, intraocular, intracranial, or oral. Parenteral administration, such as subcutaneous, intravenous or intramuscular administration, is generally achieved by injection (immunization). Injectables can be prepared in conventional forms and formulations, either as liquid solutions or suspensions, solid forms (*e.g.,* lyophilized forms) suitable for solution or suspension in liquid prior to injection, or as emulsions. Injection solutions and suspensions can be prepared from sterile powders, granules, and tablets. Administration can be systemic or local.

The isolated ALK polypeptides or compositions thereof, can be administered in any suitable manner, such as with pharmaceutically acceptable carriers, diluents, or excipients as described *supra.* Pharmaceutically acceptable carriers are determined in part by the particular immunogen or composition being administered, as well as by the particular method used to administer the composition. Accordingly, a pharmaceutical composition comprising the isolated ALK antigen polypeptides or compositions thereof, can be prepared using a wide variety of suitable and physiologically and pharmaceutically acceptable formulations.

Further provided is a method of eliciting or generating an immune response in a subject with a disease caused by oncogenic *ALK* gene fusions, rearrangements, duplications or mutations (*e.g.,* ALK-positive cancers) by administering to the subject an isolated ALK protein antigen or immunogen, or immunogenic composition or vaccine thereof, as described herein. In some embodiments, the ALK protein can be administered using any suitable route of administration, such as, for example, by intramuscular injection. In some embodiments, the ALK protein is administered as a composition comprising a pharmaceutically acceptable carrier. In some embodiments, the composition comprises an adjuvant selected from, for example, alum, Freund's complete or incomplete adjuvant, a biological adjuvant or immunostimulatory oligonucleotides (such as CpG oligonucleotides). In some embodiments, the adjuvant is conjugated to an amphiphile. In other embodiments, the composition may be administered in combination with one or more therapeutic agents or molecules.

Also provided is a method of immunizing a subject against disease or the symptoms thereof caused by oncogenic *ALK* gene fusions, rearrangements, duplications or mutations (*e.g.,* ALK-positive cancers), in which the method involves administering to the subject an isolated ALK protein as described herein, or administering an immunogenic composition or vaccine thereof. In some embodiments of the method, the composition further comprises a pharmaceutically acceptable carrier, diluent, excipient, and/or an adjuvant. For example, the adjuvant can be alum, Freund's complete or incomplete adjuvant, a biological adjuvant or immunostimulatory oligonucleotides (such as CpG oligonucleotides). In some embodiments, the adjuvant is conjugated to an amphiphile. In some embodiments, the ALK peptides (or compositions thereof) are administered intramuscularly.

An advantage of the immunogens and immunogenic compositions comprising ALK antigens described herein is that an immune response is elicited against not only the ALK-expressing tumor or cell line from which the antigen was derived, but also against one or more, or all, ALK-positive cancers, *e*.*g*., non-small cell lung cancer (NSCLC), anaplastic large cell lymphoma (ALCL), neuroblastoma, B-cell lymphoma, thyroid cancer, colon cancer, breast cancer, inflammatory myofibroblastic tumors (IMT), renal carcinoma, esophageal cancer, melanoma, or a combination thereof. In some embodiments, the immunogens and immunogenic compositions described herein elicit immune responses against NSCLC. In some embodiments, the immunogens and immunogenic compositions described herein elicit immune responses against ALCL. Thus, the ALK immunogens are more cost effective to produce, and beneficially elicit an immune response, thus, obviating a need to make and administer a poly- or multivalent immunogenic composition or vaccine.

Administration of the isolated ALK antigen polypeptides or compositions thereof, can be accomplished by single or multiple doses. In some embodiments, the doses can be administered according to the vaccination schedule described in **Example 3** and **FIG. 7A**. The dose administered to a subject should be sufficient to induce a beneficial therapeutic response in a subject over time, such as to inhibit, block, reduce, ameliorate, protect against, or prevent disease caused by oncogenic *ALK* gene fusions, rearrangements, duplications or mutations (*e.g.,* ALK-positive cancers). The dose required will vary from subject to subject depending on the species, age, weight and general condition of the subject, by the severity of the cancer being treated, by the particular composition being used and by the mode of administration. An appropriate dose can be determined by a person skilled in the art, such as a clinician or medical practitioner, using only routine experimentation. One of skill in the art is capable of determining therapeutically effective amounts of ALK antigen or immunogen, or immunogenic compositions or vaccines thereof, that provide a therapeutic effect or protection against diseases caused by *ALK* gene fusions, rearrangements, duplications or mutations (*e.g.,* ALK-positive cancers) suitable for administering to a subject in need of treatment or protection.

### Adjuvants and Combination Therapies

The ALK immunogens or immunogenic compositions or vaccines containing an ALK-specific peptide antigen can be administered alone or in combination with other therapeutic agents to enhance antigenicity or immunogenicity, *i.e.,* to increase an immune response, such as the elicitation of specific or neutralizing antibodies, in a subject. For example, the ALK-specific peptide can be administered with an adjuvant, such as alum, Freund's incomplete adjuvant, Freund's complete adjuvant, biological adjuvant, or immunostimulatory oligonucleotides (such as CpG oligonucleotides). The adjuvant may be conjugated to an amphiphile as previously described (H. Liu et al., Structure-based programming of lymph-node targeting in molecular vaccines. Nature 507, 5199522 (2014)). In some embodiments, the amphiphile conjugated to the adjuvant is N-hydroxy succinimidyl ester-end-functionalized poly(ethylene glycol)-lipid (NHS-PEG2KDa-DSPE).

One or more cytokines, such as interleukin-1 (IL-2), interleukin-6 (IL-6), interleukin-12 (IL-12), the protein memory T-cell attractant "Regulated on Activation, Normal T Expressed and Secreted" (RANTES), granulocyte-macrophage-colony stimulating factor (GM-CSF), tumor necrosis factor-alpha (TNF-α), or interferon-gamma (IFN-γ); one or more growth factors, such as GM-CSF or granulocyte-colony stimulation factor (G-CSF); one or more molecules such as the TNF ligand superfamily member 4 ligand (OX40L) or the type 2 transmembrane glycoprotein receptor belonging to the TNF superfamily (4-1BBL), or combinations of these molecules, can be used as biological adjuvants, if desired or warranted *(see, e.g.,* Salgaller et al., 1998, J. Surg. Oncol. 68(2):122-38; Lotze et al., 2000, Cancer J. Sci. Am. 6(Suppl 1):S61-6; Cao et al., 1998, Stem Cells 16(Suppl 1):251-60; Kuiper et al., 2000, Adv. Exp. Med. Biol. 465:381-90). These molecules can be administered systemically (or locally) to a subject.

Several ways of inducing cellular responses, both *in vitro* and *in vivo,* are known and practiced in the art. Lipids have been identified as agents capable of assisting in priming cytotoxic lymphocytes (CTL) *in vivo* against various antigens. For example, palmitic acid residues can be attached to the alpha and epsilon amino groups of a lysine residue and then linked (for example, via one or more linking residues, such as glycine, glycine-glycine, serine, serine-serine, or the like) to an immunogenic peptide (U.S. Patent No. 5,662,907). The lipidated peptide can then be injected directly in a micellar form, incorporated in a liposome, or emulsified in an adjuvant. As another example, *E. coli* lipoproteins, such as tripalmitoyl-S-glycerylcysteinlyseryl-serine can be used to prime tumor-specific CTL when covalently attached to an appropriate peptide (*see, e.g.,* Deres et al., 1989, Nature 342:561). Moreover, the induction of neutralizing antibodies can also be primed with the same molecule conjugated to a peptide which displays an appropriate epitope, and two compositions can be combined to elicit both humoral and cell-mediated responses where such a combination is deemed desirable.

The ALK-specific peptides can also be administered as a combination therapy with one or more other therapeutic agents, such as ALK inhibitors, tyrosine kinase inhibitors (TKIs), and/or immune checkpoint inhibitors. Non-limiting examples of ALK inhibitors include lorlatinib (Lobrena^{®}). Non-limiting examples of checkpoint inhibitors include programmed cell death protein 1 (PD-1) inhibitors, programmed death-ligand 1 (PD-L1), and cytotoxic T-lymphocyte-associated antigen-4 (CTLA-4) inhibitors. Nonlimiting examples of PD-1 inhibitors include pembrolizumab (Keytruda^{®}) and nivolumab (Opdivo^{®}). Nonlimiting examples of CTLA-4 inhibitors include ipilimumab (Yervoy^{®}). Non-limiting examples of TKI inhibitors include crizotinib, ceritinib, alectinib, brigatinib, and lorlatinib.

In some embodiments, one or more ALK inhibitors, immune checkpoint inhibitors, and/or TKI inhibitors is administered simultaneously or sequentially with ALK-specific peptide antigens, immunogens, or immunogenic compositions or vaccines containing an ALK-specific peptide antigen or immunogen. In some embodiments, the ALK-specific peptide antigen, immunogen, or immunogenic composition or vaccine containing an ALK-specific peptide antigen or immunogen is administered with a PD-1 inhibitor. In some embodiments, the PD-1 inhibitor is an anti-PD-1 antibody. In some embodiments, the ALK-specific peptide antigen, immunogen, or immunogenic composition or vaccine containing an ALK-specific peptide antigen or immunogen is administered with a PD-1 inhibitor according to the schedule described in **Example 3** and/or **FIG. 7B****.** In some embodiments, the ALK-specific peptide antigen, immunogen, or immunogenic composition or vaccine containing an ALK-specific peptide antigen or immunogen is administered with a PD-L1 inhibitor.

In some embodiments, the ALK-specific peptide antigen, immunogen, or immunogenic composition or vaccine containing an ALK-specific peptide antigen or immunogen is administered with a TKI inhibitor. In some embodiments, the ALK-specific peptide antigen, immunogen, or immunogenic composition or vaccine containing an ALK-specific peptide antigen or immunogen is administered with an ALK inhibitor. In some embodiments, the ALK-specific peptide antigen, immunogen, or immunogenic composition or vaccine containing an ALK-specific peptide antigen or immunogen is administered with lorlatinib.

In some embodiments, the ALK-specific peptide antigen, immunogen, or immunogenic composition or vaccine containing an ALK-specific peptide antigen or immunogen is administered with a PD-1 inhibitor in combination with an ALK inhibitor. In some embodiments, the ALK-specific peptide antigen, immunogen, or immunogenic composition or vaccine containing an ALK-specific peptide antigen or immunogen is administered with an anti-PD-1 antibody in combination with lorlatinib. In some embodiments, the ALK-specific peptide antigen, immunogen, or immunogenic composition or vaccine containing an ALK-specific peptide antigen or immunogen is administered with an anti-PD-1 antibody in combination with lorlatinib according to the schedule described in **Example 4** and/or **FIG. 8A**.

While treatment methods may involve the administration of a vaccine containing a ALK immunogenic protein as described herein, one skilled in the art will appreciate that the ALK protein itself, as a component of a pharmaceutically acceptable composition or as a fusion protein, can be administered to a subject in need thereof to elicit an immune response against an ALK-positive cancer in the subject.

### Kits

Also provided are kits containing the ALK antigen or immunogen as described, or an immunogenic composition, or a vaccine, or a pharmaceutically acceptable composition containing the antigen or immunogen and a pharmaceutically acceptable carrier, diluent, or excipient, for administering to a subject, for example. The antigen or immunogen may be in the form of an ALK protein (polypeptide) or a polynucleotide (a polynucleotide encoding an ALK polypeptide), as described herein. Kits containing one or more of the plasmids, or a collection of plasmids as described herein, are also provided. As will be appreciated by the skilled practitioner in the art, such a kit may contain one or more containers that house the antigen, immunogen, vaccine, or composition, carriers, diluents or excipients, as necessary, and instructions for use.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", second edition (Sambrook, 1989); "Oligonucleotide Synthesis" (Gait, 1984); "Animal Cell Culture" (Freshney, 1987); "Methods in Enzymology" "Handbook of Experimental Immunology" (Weir, 1996); "Gene Transfer Vectors for Mammalian Cells" (Miller and Calos, 1987); "Current Protocols in Molecular Biology" (Ausubel, 1987); "PCR: The Polymerase Chain Reaction", (Mullis, 1994); "Current Protocols in Immunology" (Coligan, 1991). These techniques are applicable to the production of the polynucleotides and polypeptides of the invention, and, as such, may be considered in making and practicing the invention. Useful techniques for particular embodiments will be discussed in the sections that follow.

The following examples are put forth to provide those of ordinary skill in the art with a complete disclosure and description of how to make and use the assay, screening, and therapeutic methods of the invention, and are not intended to limit the scope of what the inventors regard as their invention.

### EXAMPLES

The following examples are provided to illustrate certain particular features and/or embodiments. The examples should not be construed to limit the disclosure to the particular features or embodiments described.

### EXAMPLE 1. IDENTIFICATION OF ALK-SPECIFIC PEPTIDES FOR AN ALK-POSITIVE CANCER VACCINE

The direct identification of ALK peptides that are effectively presented on the surface of ALK-expressing cancer cells in the context of the most frequent patient haplotypes will provide a powerful resource to design the most effective vaccine to elicit ALK-specific T-cell responses.

### Example 1.1 Identification of ALK-Specific Peptides in Different HLA-Haplotypes

The B721.221 human lymphoblastic cell line does not express endogenous HLA class I (A, B and C) due to gamma-ray-induced mutations in the HLA complex. (Shimizu Y, DeMars R. Production of human cells expressing individual transferred HLA-A, -B, -C genes using an HLA-A, -B, -C null human cell line. J. Immunol. 1989;142(9):3320-3328). Fifty (50) cell lines each expressing a single HLA class I allele (monoallelic cell lines) were generated by transducing B721.221 cells with retroviruses encoding HLA class I genes. This tool allows the unequivocal assignation of peptides identified by mass spectrometry to a specific HLA class I allele. The HLA-peptide complexes were immunoprecipitated with an anti-HLA A/B/C antibody (clone W6/32, Santa Cruz) and the peptides released were analyzed by liquid chromatography-tandem mass spectrometry (LC-MS/MS). Using retroviral transduction, the EML4-ALK variant 1, the most frequent EML4-ALK fusion protein (Lin JJ, et al. Impact of EML4-ALK Variant on Resistance Mechanisms and Clinical Outcomes in ALK-Positive Lung Cancer. J. Clin. Oncol. 2018:JCO2017762294), was expressed into different HLA-monoallelic B721.221 cell lines **(****FIG. 1A****).** The selection of the different cell lines to be transduced was based on the frequency of HLA class I alleles among patients with ALK-positive NSCLC **(Table 1).** B721.221/A*02:01, B721.221/A*01:01 and B721.221/C*06:02 cells were transduced with a construct encoding the EML4-ALK variant 1 and GFP. Cells were sorted based on the GFP positivity and expanded. A western blot was performed using an untransduced B721.221/A*02:01 cell line as a negative control and the H3122 ALK+ NSCLC cell line as a positive control.

Using the most sensitive targeted mass spectrometry, two (2) peptides predicted by the algorithms from the immune epitope database (IEDB), AMLDLLHVA and SLAMLDLLHV, were isotope-labelled (heavy standard) and searched in HLA*02:01 elute from B721.221/A*02:01 cells. Only the 9-mer, AMLDLLHVA, ALK-specific peptide binding the HLA A*02:01 allele was identified **(****FIG. 1B****).** ALK-specific peptides were not identified in the HLA A*01:01, HLA A*02:01 or HLA C*06:02 eluates using discovery mass spectrometry. Since protein overexpression can compensate for low binding affinity of the peptides for the HLA proteins (*see, e.g.,* Abelin JG, et al. Mass Spectrometry Profiling of HLA-Associated Peptidomes in Mono-allelic Cells Enables More Accurate Epitope Prediction. Immunity. 2017;46(2):315-326) and considering that the level of EMI,4-ALK expression in B721.221/HLA A*02:01 cells was at least one order of magnitude below the range of expression of the reference NSCLC H3122 cell line **(****FIG. 1A****),** the identified ALK peptide, AMLDLLHVA, was determined to be presented on the surface of tumor cells with a strong affinity for HLA A*02:01.

**Table 1. Percentage of different HLA class I alleles among 100 ALK-positive lung cancer patients.**

| **HLA A** | | **HLA B** | | **HLAC** | |
|---|---|---|---|---|---|
| **Allele** | **Frequency** | **Allele** | **Frequency** | **Allele** | **Frequency** |
| *02:01 | 43% | *07:02 | 18% | *07:01 | 27% |
| *01:01 | 26% | *08:01 | 15% | *04:01 | 26% |
| *24:02 | 24% | *35:01 | 12% | *06:02 | 24% |
| *03:01 | 22% | *18:01 | 10% | *07:02 | 22% |
| *11:01 | 12% | *44:02 | 10% | *03:04 | 15% |

### Example 1.2 Identification of ALK-Specific Peptides in Different Cell Lines

The low levels of EMI,4-ALK expression reached by transducing B721.221 cells make it difficult to identify ALK-specific peptides normally expressed on the surface of tumor cells. In order to avoid this technical challenge, different ALK-positive anaplastic large cell lymphoma (ALCL) cell lines encoding frequent HLA-alleles were selected (Karpas-299, DEL, and SR-786). ALCL cell lines express high levels of the NPM-ALK fusion protein, a fusion protein containing the exactly same ALK portion as the EML4-ALK fusion protein. The HLA genotype and HLA expression on the selected cell lines were confirmed before proceeding with mass spectrometry experiments **(Table 2).** The two HLA -A, -B and -C alleles were determined by Next Generation sequencing (NGS). Expression levels of HLA were determined by flow cytometry with a pan-HLA antibody. Positive or negative antibody staining for anti-HLA-A*02, which recognizes all the allelic variants of HLA-A*02, and anti-DR, which recognizes all the HLA class II DR (HLA-DR) subclasses, was determined by flow cytometry.

**Table 2. Characterization of ALK-positive ALCL cell lines.**

| | **Next Generation Sequencing** | | | | | | | **Antibody Staining** | | |
|---|---|---|---|---|---|---|---|---|---|---|
| | **HLA** | **A(1)** | **A(2)** | **B(1)** | **B(2)** | **C(1)** | **C(2)** | **Pan-HLA** | **A02** | **DR** |
| **ALCL Cell Lines** | **KARPAS** | *03:01 | *11:01 | *07:02 | *35:01 | *04:01 | *07:02 | (+++) | No | Yes |
| | **DEL** | *26:01 | *02:01 | *14:01 | *27:05 | *08:02 | *05:01 | (++++ ) | Yes | Yes |
| | **SR786** | *02:01 | *68:02 | *55:01 | *57:01 | *03:03 | *06:02 | (+++) | Yes | No |

Since the HLA A*02:01 allele can be immunoprecipitated by a specific antibody (BB7.2 clone), the peptide, AMLDLLHVA, previously identified in B721.221/HLA A*02:01 cells, was isotope-labeled (heavy standard) and its presence was confirmed by targeted mass spectrometry in the HLA A *02:01 elute of DEL cells **(****FIG. 2A****).** For cell lines expressing different HLAs than HLA A*02:01, the HLA-peptide complexes were pulled down with an anti-pan-HLA antibody. ALK-specific peptides in the HLAs elutes were then searched by mass spectrometry in discovery mode. The system and algorithm as provided in Abelin JG, et al. (Mass Spectrometry Profiling of HLA-Associated Peptidomes in Mono-allelic Cells Enables More Accurate Epitope Prediction. Immunity. 2017;46(2):315-326) was used to assign the discovered peptides to a specific HLA. This algorithm was trained with the endogenous peptides presented by several of the aforementioned HLA-monoallelic cell lines. Following this approach, three (3) ALK-specific peptides presented in the HLA elute from Karpas-299 cells were identified **(****FIGS. 2B-2D****).** Two independent injections were run in the mass spectrometer. IVRCIGVSL and RPRPSQPSSL were found in both injections **(****FIGS. 2B** and **2C****,** respectively). VPRKNITLI was only found in one of the two injections **(****FIG. 2D****).** All three (3) peptides were strongly predicted to bind the HLA B*07:02 allele **(****FIGS. 2B-2D****).**

This data showed the physiological processing and presentation of four (4) different ALK-peptides on the groove of two frequent HLA class I haplotypes, HLA A*02:01 and HLA B*07:02, by cancer cells **(Table 3).** The peptides described in Table 3 belong to the intracytoplasmic portion of ALK; therefore, are comprised in all the possible forms of ALK aberrant expression, such as overexpression (neuroblastoma) or the EML4-ALK (NSCLC) and NPM-ALK (ALCL) translocations. Based on the frequency of these HLA class I alleles among individuals with ALK-positive NSCLC, a therapeutic vaccine against these peptides would be applicable to about 54% of patients.

In addition, the 9-mer peptide, TAAEVSVRV, was identified in two independent experiments using pan-HLA elute from SR-786 cells **(****FIG. 3****).** According to the NetMHCpan and Artificial Neural Network (ANN) algorithms from IEDB, this peptide binds the HLA A*68:02 allele **(Table 3),** an allele found in about 5% of patients with ALK-positive tumors.

**Table 3. Peptides discovered by mass spectrometry.**

| **Sequence** | **Predicted Allele** |
|---|---|
| AMLDLLHVA | HLA A*02:01 |
| RPRPSQPSSL | HLA B*07:02 |
| IVRCIGVSL | HLA B*07:02 |
| VPRKNITLI | HLA B*07:02 |
| TAAEVSVRV | HLA A*68:02 |

### Example 1.3 Identified ALK-Specific Peptides Induce Immune Response In Vitro

The ability of the identified peptides to induce an immune response was assessed *in vitro.* Cryopreserved PBMCs from ALK-positive NSCLC patients or PBMCs from healthy donors were incubated *in vitro* with autologous antigen presenting cells (APC) as previously described (Cai A, et al. Mutated BCR-ABL generates immunogenic T-cell epitopes in Chronic myelogenous leukemia (CML) patients (Rajasagi et al., Clin. Cancer Res. 2012;18(20):5761-5772; Rajasagi M, et al. Blood. 2014;124(3):453-462). 10×10⁶ PBMCs were incubated in a 24-well plate with autologous dendritic cells (DCs) pulsed with 10µM of the corresponding peptide (ratio 100:1) in a media supplemented with 10% human AB serum, rhIL7 (10ng/ml) and rhIL2 (20 IU/ml). After 6 days, CD8⁺ T-cells were isolated from the bulk population by magnetic beads (StemCell Technologies) and incubated with irradiated B721.221-HLA monoallele pulsed with 10µM of the corresponding peptide (ratio 10:1). Cells were re-stimulated on days 7, 14, 21 and 28 by adding rhIL7 (10ng/ml) and rhIL2 (20IU/ml) on days 8, 15, 22 and 29. The specificity of the T-cells was evaluated one week after the last stimulation. T-cells were incubated with irradiated B721.221/corresponding HLA/peptide-pulsed or B721.221/corresponding HLA/EML4-ALK transduced (ratio 1:1) cells in an IFNγ-ELISPOT plate (Mabtech). As an alternative approach, T-cells were stained by dextramers that were synthesized based on the identified peptide in a complex with the corresponding HLA allele (Immunex).

The capability of these peptides in generating T-cells responses was also assessed. HLA-A*02:01 transgenic mice (C57BL/6-*Mcph1^{Tg(HLA-A2.1)1Enge}*/J) were vaccinated with the AMLDLLHVA peptide together with Freund's complete adjuvant. Three (3) weeks after vaccination, splenocytes from the vaccinated mice were challenged with AMLDLLHVA, OVA-peptide (negative control), and media (negative control) in an interferon-gamma (IFN-γ) ELISPOT assay **(****FIG. 4A****).** In the two (2) vaccinated mice, a 2- and 5-fold difference, respectively, was found in IFN -γ spot forming units (SFU) between wells with splenocytes challenged with AMI,DLLHVA and those wells challenged with OVA-peptide **(****FIG. 4B****).** Moreover, a direct IFN-γ-ELISPOT assay was performed by challenging PBMCs from ALK-positive NSCLC HLA A*02:01 patients with the AMLDLLHVA peptide, HIV peptide and no peptide stimulation negative controls, and flu matrix positive control **(****FIG. 4C****).** One (1) out of twenty (20) patients evaluated showed responses to this specific peptide, indicating an ALK-specific spontaneous T-cell response.

For the ALK-specific HLA B*07:02 binding peptides, IVRCIGVSL and RPRPSQPSSL, a significant response was not detected in the direct IFN-γ-ELISPOT performed with PBMCs from four (4) ALK-positive NSCLC patients. Thus, peptide-specific CD8⁺ T-cell expansion with IVRCIGVSL and RPRPSQPSSL was evaluated in ALK-positive NSCLC HLA B*07:02 patient PBMCs according to the schematic in **FIG. 5A****.** PBMCs from ALK-positive NSCLC HLA*07:02 patients were thawed and rested overnight in the presence of rh-II,-7 (10 ng/ml) at Day -1. At Day 0, PBMCs were stimulated alternatively with IVRCIGVSL or RPRPSQPSSL. From Day 3, and every 3-4 days, fresh media containing rh-IL-2 and rh-IL-7 was added. Second and third stimulation was done by incubating purified CD8⁺ T-cells with peptide-pulsed mature DCs (mDCs) or CD40-activated B-cells, respectively. IFN-γ ELISPOT was performed as a read out of the expansion at Day 21. HIV-pulsed and non-pulsed B-cells were used as negative controls. CEF plus-pulsed B-cells were used as positive control.

When performing an IFN-γ-ELISPOT using the IVRCIGVSL-expanded CD8+ T-cells, about four (4) times more spot forming units in wells challenged with IVRCIGVSL-pulsed B-cells was detected compared to those wells challenged with B-cells pulsed with control peptides **(****FIG. 5B** **and** **5C****).** The ELISPOT was repeated twice with similar results. The same approach with the RPRPSQPSSL peptide in PBMCs from four (4) ALK-positive NSCLC HLA B*07:02 patients did not produce significant responses.

### EXAMPLE 2. ALK AMPHIPHILE PEPTIDES

Without wishing to be bound by theory, *in vitro* and *in vivo* data suggest that amphiphile-peptide (amph-peptide) vaccines function by binding to endogenous albumin present in interstitial fluid following injection, with subsequent albumin-mediated transport to lymph nodes for efficient capture by dendritic cells (DCs) (H. Liu et al., Structure-based programming of lymph-node targeting in molecular vaccines. Nature 507, 5199522 (2014)). A schematic of this amph-peptide vaccine "albumin hitchhiking" concept is shown in **Fig. 6A****.**

### Example 2.1 ALK Peptide Selection for Amphiphile-Peptide Conjugation

*In silico* prediction of peptide-MHC binding is considered a reliable strategy to rank the probability of shared or neoantigens to be effectively presented on human HLA subtypes (E. F. Fritsch et al., HLA-binding properties of tumor neoepitopes in humans. Cancer Immunol Res 2, 5229529 (2014)). To determine whether conjugation with an amphiphilic tail can be applied to an ALK-specific peptide vaccine, *in silico* prediction and ELISpot-based screening techniques were used to identify immunogenic ALK peptides that induce strong immune responses in mouse models.

Out of twenty-eight (28) patients analyzed for HLA haplotypes, thirteen (13) patients were HLA A*02:01, eight (8) patients were HLA B*07:02, and eight (8) patients were HLA C*07:02. NetMHCpan and IEDB algorithms were used to predict which ALK peptides were most likely to be presented on HLA A*02:01, HLA B*07:02, and HLA C*07:02. The predicted ALK peptides for these three HLA haplotypes are shown in **Table 4.**

**Table 4. Selection of ALK peptides for synthesis of an ALK vaccine**

| **HLA A*02:01** | | | |
|---|---|---|---|
| **NetMHCpan** | | **IEDB** | |
| **9mer** | **nM** | **9mer** | **Percentile Rank** |
| VLLWEIFSL | 5 | VLLWEIFSL | 0.3 |
| AMLDLLHVA | 8 | FLMEALIIS | 0.5 |
| KTDTWSFGV | 11 | AMLDLLHVA | 0.5 |
| FLMEALIIS | 16 | KTDTWSFGV | 1.1 |
| GMARDIYRA | 77 | GMARDIYRA | 1.2 |
| | | | |

| **10mer** | **nM** | **10mer** | **Percentile Rank** |
|---|---|---|---|
| SLAMLDLLHV | 16 | SLAMLDLLHV | 0.4 |
| FLMEALIISK | 35 | FLMEALIISK | 0.55 |
| SLPRFILLEL | 53 | SLPRFILLEL | 1.45 |
| LLLEPSSLTA | 71 | GVLLWEIFSL | 1.55 |
| GVLLWEIFSL | 93 | LLLEPSSLTA | 1.6 |
| | | | |

| **HLA B*07:02** | | | |
|---|---|---|---|
| **NetMHCpan** | | **IEDB** | |
| **9mer** | **nM** | **9mer** | **Percentile Rank** |
| KPTKKNNPI | 14 | RPSQPSSLA | 0.3 |
| RPSQPSSLA | 16 | LPRFELLEL | 0.5 |
| LPRFELLEL | 18 | VPRKNITLI | 0.5 |
| IVRCIGVSL | 20 | KPTKKNNPI | 1.1 |
| VPRKNITLI | 27 | RPRPSQPSS | 1.2 |
| | | | |

| **10mer** | **nM** | **10mer** | **Percentile Rank** |
|---|---|---|---|
| RPRPSQPSSL | 7 | RPRPSQPSSL | 0.1 |
| RPSQPSSLAM | 14 | RPSQPSSLAM | 0.25 |
| YPSKSNQEVL | 55 | LPRFELLELM | 0.3 |
| LPRFELLELM | 60 | YPSKSNQEVL | 0.65 |
| SPLQVAVKTL | 85 | SPLQVAVKTL | 0.85 |
| | | | |

| **HLA C*07:02** | | | |
|---|---|---|---|
| **NetMHCpan** | | **IEDB** | |
| **9mer** | **nM** | **9mer** | **Percentile Rank** |
| YYRKGGCAM | 62 | VYEGQVSGM | 0.7 |
| YRKGGCAMI | 194 | YRKGGCAMI | 0.75 |
| VYRRKHQEL | 225 | VYRRKHQEL | 0.85 |
| KWMPPEAFM | 257 | VRVPRGPAV | 1.2 |
| GRLPGASLL | 316 | YYRKGGCAM | 1.35 |
| | | | |

| **10mer** | **nM** | **10mer** | **Percentile Rank** |
|---|---|---|---|
| YYRKGGCAMI | 64 | YYRKGGCAML | 0.2 |
| SYYRKGGCAM | 145 | SYYRKGGCAM | 0.5 |
| ERSPAAPPPL | 429 | RRKHQELQAM | 1.2 |
| RRKHQELQAM | 443 | ERSAPAAPPPL | 1.2 |
| GRLPGASLLL | 495 | SFGVLLWEIF | 1.3 |

Twenty-three (23) synthetic long peptides (SLP) were synthesized, each approximately thirty (30) amino acids in length **(Table 5).** The SLPs were synthesized to overlap with one another and span the entire region of ALK, which is translocated in human cancers. The peptides were synthesized by standard solid-phase GMP synthesis. Each peptide was purified by HPLC, and pooled and coupled through amino terminal residues with N-hydroxy succinimidyl ester-end-functionalized poly(ethylene glycol)-lipid (NHS-PEG2KDa-DSPE), forming the amphiphile-peptide conjugate, followed by size exclusion chromatography (SEC) purification.

**Table 5. ALK synthetic long peptides (SLPs)**

| | |
|---|---|
| 1 | VYRRKHQELQAMQMELQSPEYKLSKLRTSTIMTDYN |
| 2 | KLRTSTIMTDYNPNYCFAGKTSSISDLKEVPRKNIT |
| 3 | SDLKEVPRKNITLIRGLGHGAFGEVYEGQVSGMPND |
| 4 | VYEGQVSGMPNDPSPLQVAVKTLPEVCSEQDELDFL |
| 5 | EVCSEQDELDFLMEALIISKFNHQNIVRCIGVSLQS |
| 6 | NIVRCIGVSLQSLPRFILLELMAGGDLKSFLRETRP |
| 7 | GDLKSFLRETRPRPSQPSSLAMLDLLHVARDIACGC |
| 8 | LLHVARDIACGCQYLEENHFIHRDIAARNCLLTCPG |
| 9 | IAARNCLLTCPGPGRVAKIGDFGMARDIYRASYYRK |
| 10 | ARDIYRASYYRKGGCAMLPVKWMPPEAFMEGIFTSK |
| 11 | PEAFMEGIFTSKTDTWSFGVLLWEIFSLGYMPYPSK |
| 12 | IFSLGYMPYPSKSNQEVLEFVTSGGRMDPPKNCPGP |
| 13 | GRMDPPKNCPGPVYRIMTQCWQHQPEDRPNFAIILE |
| 14 | PEDRPNFAIILERIEYCTQDPDVINTALPIEYGPLV |
| 15 | NTALPIEYGPLVEEEEKVPVRPKDPEGVPPLLVSQQ |
| 16 | PEGVPPLLVSQQAKREEERSPAAPPPLPTTSSGKAA |
| 17 | PPLPTTSSGKAAKKPTAAEISVRVPRGPAVEGGHVN |
| 18 | PRGPAVEGGHVNMAFSQSNPPSELHKVHGSRNKPTS |
| 19 | HKVHGSRNKPTSLWNPTYGSWFTEKPTKKNNPIAKK |
| 20 | KPTKKNNPIAKKEPHDRGNLGLEGSCTVPPNVATGR |
| 21 | SCTVPPNVATGRLPGASLLLEPSSLTANMKEVPLFR |
| 22 | LTANMKEVPLFRLRHFPCGNVNYGYQQQGLPLEAAT |
| 23 | GYQQQGLPLEAATAPGAGHYEDTILKSKNSMNQPGP |

### Example 2.2 ALK Amph-Peptides Elicit Protective T-Cell Responses

To determine whether, ALK amph-peptides elicited a T-cell response, Balb/c mice (Charles River Laboratories) were immunized with a series of different ALK amph-peptides and then assayed for *ex vivo* antigen-specific cytokine production. ALK amph-peptides may be generated as provided in H. Liu et al., Structure-based programming of lymph-node targeting in molecular vaccines. Nature 507, 5199522 (2014). The ALK amph-peptides assayed include: VYRRKHQEL (ALK Peptide 1); PGPGRVAKI (ALK Peptide 9 short); PGPGRVAKIGDFGMARDIY (ALK Peptide 9 long); and CGNVNYGYQQQGLPLEAAT (ALK Peptide 22/23). **FIG. 6B** shows that the reactive ALK sequences identified by ELISPOT are immunogenic as amph-peptides, eliciting both CD4⁺ and CD8⁺ T-cell responses. Further, the number of tumors were assessed fourteen (14) days after the Balb/c mice were immunized with the different ALK peptides. Naive peptide was used as a negative control. Mice vaccinated with ALK amph-peptides and then challenged with ALK⁺ tumor cells were strongly resistant to the establishment of lung tumors **(****Fig. 6C****).** This data shows that compared to "naked" peptides, peptides modified with an amphiphilic conjugate significantly increased T-cell expansion and greatly enhanced anti-tumor efficacy.

### EXAMPLE 3. ALK VACCINATION SCHEDULE FOR NSCLC PATIENTS HARBORING ALK REARRANGEMENTS

In selecting patients with advanced ALK-positive NSCLC for administration of an ALK vaccine, patients were required to have progressive disease and at least one prior ALK tyrosine kinase inhibitor treatment or treatment with multiple ALK tyrosine kinase inhibitors. A washout period of three (3) weeks for standard chemotherapy or five (5) half-lives for oral tyrosine kinase inhibitors was required prior to vaccine administration. At baseline, all patients were required to undergo both blood collection to measure ALK specific autoantibodies and ALK-specific T-cell responses as well as a tumor core biopsy in order to assess for infiltrating immune cell subsets. The first portion of this study consisted of peptide administration alone. Six (6) patients (Cohort 1A) received "naked" ALK peptides, and six (6) patients (Cohort 1B) received amphiphilic-conjugated peptides. Twenty (20) peptides were synthesized and pools of five (5) peptides and the adjuvant poly-ICLC, which is a synthetic complex of carboxymethylcellulose, polyinosinic-polycytidylic acid, and poly-L-lysine double-stranded RNA, were injected into four (4) different anatomic sites that drain to separate lymph node basins in order to reduce potential antigenic competition between peptides binding to the same HLA molecule. ALK vaccine was administered on days 1, 8, 15, 29, 43, and 57 with a booster vaccine at 16 and 24 weeks according to the schedule shown in **FIG. 7A****.** Patients were assessed for dose-limiting toxicities (DLTs) for a period of six (6) weeks. Based on an analysis of safety, tolerability, efficacy, and ability to generate immunologic responses to ALK at eight (8) weeks, one of the two peptide formulations (naked or amphiphilic) was selected for use in patients for Cohort 2. In Cohort 2A, eight (8) patients received concurrent ALK peptide vaccine in combination with a commercially available PD-1 inhibitor according to the schedule as shown in **FIG. 7B****.** In Cohort 2B, eight (8) patients initially received ALK peptide vaccine alone, and then upon disease progression, the PD-1 inhibitor was added to the treatment regimen.

### EXAMPLE 4. EFFICACY OF ALK-PEPTIDE VACCINE IN COMBINATION WITH ALK INHIBITOR THERAPY AND IMMUNE CHECKPOINT THERAPY

To test the efficacy of an ALK-peptide vaccine in combination with ALK inhibitor therapy and immune checkpoint therapy, a non-small cell lung cancer (NSCLC) mouse model was utilized. To perform the experiments in immunocompetent mice, EML4-ALK rearranged NSCLC cell line syngeneic to the BALB/c mouse strain was generated. These cells were further edited by CRISPR/Cas9 technology to introduce in the ALK gene a CD8⁺ epitope specific for BALB/c mice. These NSCLC cell lines colonize the lung when injected into the tail vein and generate lung tumors. Once tumors formed in the lungs, mice were treated with either: i) the ALK-specific inhibitor, lorlatinib, for 15 days (n=9); ii) with a combination of lorlatinib and the check-point inhibitor, PD-1, (n=9); iii) with lorlatinib and the ALK vaccine (n=9); or iv) a combination of lorlatinib, anti-PD1 and ALK vaccine (n=8) **(****FIG. 8A****).** The control group did not receive any type of treatment (n=5). All mice injected with tumor cells that did not receive any treatment died before day 21 due to the development of multiple tumors. Lorlatinib treatment delayed tumor progression (median survival 36.5 days). Anti-PD-1 did not add any benefit to the treatment with lorlatinib (median survival 36.5 days). The addition of the ALK vaccine to lorlatinib significantly extended the survival of the mice (median survival 54 days). Adding anti-PD-1 to the ALK vaccine in combination with lorlatinib further extended survival (median survival 68 days) **(****FIG. 8B****).** Thus, the ALK vaccine, alone or in combination with anti-PD-1, significantly reduced tumor growth and extended survival of mice with ALK-positive lung tumors treated with an ALK inhibitor.

### Other Embodiments

From the foregoing description, it will be apparent that variations and modifications may be made to the invention described herein to adopt it to various usages and conditions. Such embodiments are also within the scope of the following claims.

The recitation of a listing of elements in any definition of a variable herein includes definitions of that variable as any single element or combination (or subcombination) of listed elements. The recitation of some embodiments herein includes that embodiment as any single embodiment or in combination with any other embodiments or portions thereof.

All patents and publications mentioned in this specification are herein incorporated by reference to the same extent as if each independent patent and publication was specifically and individually indicated to be incorporated by reference.

### Further Embodiment

1. An isolated anaplastic lymphoma kinase (ALK) peptide, wherein the peptide: i) is a fragment of the cytoplasmic portion of an ALK protein shared by cancers having an ALK rearrangement and cancers expressing the ALK protein; ii) is capable of binding a human leukocyte antigen (HLA); and iii) is capable of generating an immune response against one or more ALK-positive cancers, wherein the peptide does not include the amino acid sequence AMLDLLHVA.
2. An isolated anaplastic lymphoma kinase (ALK) peptide capable of generating an immune response against one or more ALK-positive cancers, wherein the ALK peptide comprises the amino acid sequence RPRPSQPSSL.
4. An isolated anaplastic lymphoma kinase (ALK) peptide capable of generating an immune response against one or more ALK-positive cancers, wherein the ALK peptide comprises the amino acid sequence IVRCIGVSL.
5. An isolated synthetic anaplastic lymphoma kinase (ALK) peptide capable of generating an immune response against one or more ALK-positive cancers, wherein the ALK peptide comprises the amino acid sequence VPRKNITLI.
6. An isolated synthetic anaplastic lymphoma kinase (ALK) peptide capable of generating an immune response against one or more ALK-positive cancers, wherein the ALK peptide comprises the amino acid sequence TAAEVSVRV.
7. The peptide of item 1, wherein the peptide comprises an amino acid sequence that has at least about 95% identity to a sequence listed in Table 3, Table 4, or Table 5.
8. The peptide of item 1, wherein the peptide comprises or consists of an amino acid sequence selected from those listed in Table 3, Table 4, or Table 5.
9. The peptide of item 1, wherein the peptide comprises an amino acid sequence that has at least about 95% identity to an amino acid sequence selected from the group consisting of RPRPSQPSSL; IVRCIGVSL; VPRKNITLI; and TAAEVSVRV.
10. The peptide of any one of items 1-9, wherein the peptide is conjugated to an amphiphile.
11. The peptide of item 10, wherein the amphiphile is N-hydroxy succinimidyl ester-end-functionalized poly(ethylene glycol)-lipid (NHS-PEG2KDa-DSPE).
12. The peptide of any one of items 1-11, wherein the one or more ALK-positive cancers is selected from the group consisting of non-small cell lung cancer (NSCLC), anaplastic large cell lymphoma (ALCL), neuroblastoma, B-cell lymphoma, thyroid cancer, colon cancer, breast cancer, inflammatory myofibroblastic tumors (IMT), renal carcinoma, esophageal cancer, and melanoma.
13. The peptide of item 12, wherein the one or more ALK-positive cancers is non-small cell lung cancer (NSCLC).
14. The peptide of item 12, wherein the one or more ALK-positive cancers is anaplastic large cell lymphoma (ALCL).
15. The peptide of item 1, wherein the HLA is encoded by a HLA class I allele.
16. The peptide of item 15, wherein the HLA class I allele is selected from those listed in Table 1, Table 2, or Table 3.
17. The peptide of any one of items 15 or 16, wherein the HLA class I allele is HLA A*02:01, HLA A*68:02, HLA B*07:02, or HLA C*07:02.
18. The peptide of item 1, wherein the ALK protein is a full-length human ALK protein.
19. The peptide of item 1, wherein the ALK rearrangement is a nucleophosmin-ALK rearrangement (NPM-ALK) or an echinoderm microtubule-associate protein-like 4-ALK rearrangement (EMI,4-ALK).
20. A polynucleotide encoding the ALK peptide of any one of items 1-19.
21. A vaccine comprising the polynucleotide of item 20.
22. A vaccine comprising the ALK peptide of any one of items 1-19.
23. A vaccine comprising two or more isolated synthetic anaplastic lymphoma kinase (ALK) peptides capable of generating an immune response against one or more ALK-positive cancers, wherein the two or more isolated ALK peptides are selected from those listed in Table 3, Table 4, or Table 5.
24. The vaccine of item 23, wherein the two or more ALK peptides comprise two or more amino acid sequences selected from the following: AMLDLLHVA; RPRPSQPSSL; IVRCIGVSL; VPRKNITLI; and/or TAAEVSVRV.
25. The vaccine of any one of items 23 or 24, wherein at least one of the two or more ALK peptides is conjugated to an amphiphile.
26. The vaccine of item 25, wherein the amphiphile is N-hydroxy succinimidyl ester-end-functionalized poly(ethylene glycol)-lipid (NHS-PEG2KDa-DSPE).
27. An immunogenic composition comprising the vaccine of any one of items 21-26 and a pharmaceutically acceptable carrier, diluent, or excipient.
28. The immunogenic composition of item 27, further comprising an adjuvant.
29. The immunogenic composition of item 28, wherein the adjuvant is a synthetic complex of carboxymethylcellulose, polyinosinic-polycytidylic acid, and poly-L-lysine double-stranded RNA (poly ICLC) or CpG oligonucleotides.
30. The immunogenic composition of item 28, wherein the adjuvant is conjugated to an amphiphile.
31. The immunogenic composition of item 30, wherein the amphiphile conjugated to the adjuvant is N-hydroxy succinimidyl ester-end-functionalized poly(ethylene glycol)-lipid (NHS-PEG2KDa-DSPE).
32. A method of treating an ALK-positive cancer in a subject, the method comprising administering to the subject an effective amount of the ALK peptide of any one of items 1-11, the vaccine of any one of items 21-26, or the immunogenic composition of any one of items 27-31.
33. A method of treating a subject with one or more ALK-positive cancers, the method comprising administering to the subject an effective amount of the ALK peptide of any one of items 1-11, the vaccine of any one of items 21-26, or the immunogenic composition of any one of items 27-31.
34. The method of any one of items 32 or 33, further comprising administering, simultaneously or sequentially, to the subject an effective amount of one or more of an ALK inhibitor, immune checkpoint inhibitor, and/or tyrosine kinase inhibitor (TKI).
35. The method of item 34, wherein the immune checkpoint inhibitor is selected from one or more of a programmed cell death protein 1 (PD-1) inhibitor, programmed death-ligand 1 (PD-L1) inhibitor, or cytotoxic T-lymphocyte-associated antigen-4 (CTLA-4) inhibitor.
36. The method of item 35, wherein the PD-1 inhibitor is an anti-PD1 antibody.
37. The method of item 34, wherein the ALK inhibitor is lorlatinib.
38. The method of item 35, wherein the anti-PD1 antibody is administered in combination with lorlatinib.
39. The method of any one of items 32-38, wherein an adjuvant is concomitantly administered to the subject.
40. The method of any one of items 32-39, wherein the ALK-positive cancer is selected from the group consisting of non-small cell lung cancer (NSCLC), anaplastic large cell lymphoma (ALCL), neuroblastoma, B-cell lymphoma, thyroid cancer, colon cancer, breast cancer, inflammatory myofibroblastic tumors (IMT), renal carcinoma, esophageal cancer, and melanoma.
41. The method of item 40, wherein the ALK-positive cancer is non-small cell lung cancer (NSCLC).
42. The method of item 40, wherein the ALK-positive cancer is anaplastic large cell lymphoma (ALCL).
43. The method of any one of items 32-42, wherein the subject had at least one prior treatment with at least one tyrosine kinase inhibitor (TKI).
44. The method of any one of items 32-43, wherein the step of administering comprises one or more doses of the ALK peptide, the vaccine, or the immunogenic composition.
45. The method of any one of items 32-44, wherein the step of administering comprises at least six (6) prime doses and at least two (2) booster doses of the ALK peptide, the vaccine, or the immunogenic composition.
46. A method of generating an immune response in a subject comprising administering to the subject an effective amount of the ALK peptide of any one of items 1-11, the vaccine of any one of items 21-26, or the immunogenic composition of any one of items 27-31.
47. The method of item 46, wherein the immune response comprises producing T-lymphocytes.
48. A kit comprising an agent for administration to a subject with one or more ALK-positive cancers, wherein the agent comprises the ALK peptide of any one of items 1-11, the vaccine of any one of items 21-26, or the immunogenic composition of any one of items 27-31.

## Claims

1. An isolated anaplastic lymphoma kinase (ALK) peptide, wherein the peptide: i) is a fragment of the cytoplasmic portion of an ALK protein shared by cancers having an ALK rearrangement and cancers expressing the ALK protein; ii) is capable of binding a human leukocyte antigen (HLA); and iii) is capable of generating an immune response against one or more ALK-positive cancers, wherein the peptide comprises amino acid sequence TAAEVSVRV.

2. The isolated anaplastic lymphoma kinase peptide of claim 1, wherein the peptide is conjugated to an amphiphile.

3. The isolated anaplastic lymphoma kinase peptide of claim 2, wherein the amphiphile is N-hydroxy succinimidyl ester-end-functionalized poly(ethylene glycol)-lipid (NHS-PEG2KDa-DSPE).

4. The isolated anaplastic lymphoma kinase peptide of any one of claims 1-3, wherein the one or more ALK-positive cancers is selected from the group consisting of non-small cell lung cancer (NSCLC), anaplastic large cell lymphoma (ALCL), neuroblastoma, B-cell lymphoma, thyroid cancer, colon cancer, breast cancer, inflammatory myofibroblastic tumors (IMT), renal carcinoma, esophageal cancer, and melanoma.

5. The isolated anaplastic lymphoma kinase peptide of claim 1, wherein the HLA is encoded by a HLA class I allele.

6. The isolated anaplastic lymphoma kinase peptide of claim 5, wherein the HLA class I allele is HLA A*02:01, HLA A*68:02, HLA B*07:02, or HLA C*07:02.

7. A polynucleotide encoding the ALK peptide of any one of claims 1-6.

8. A vaccine or immunogenic composition comprising the polynucleotide of claim 7 or one or more of the ALK peptide of any one of claims 1-6.

9. The vaccine or immunogenic composition of claim 8, further comprising an adjuvant, wherein the adjuvant is a synthetic complex of carboxymethylcellulose, polyinosinic-polycytidylic acid, and poly-L-lysine double-stranded RNA (poly ICLC) or CpG oligonucleotides.

10. The ALK peptide of any one of claims 1-6, or the vaccine or immunogenic composition of claim 8 or 9 for use in a method of treating an ALK-positive cancer in a subject, the method comprising administering to the subject an effective amount of the ALK peptide or the vaccine or immunogenic composition, optionally further comprising administering, simultaneously or sequentially, to the subject an effective amount of one or more of an ALK inhibitor, immune checkpoint inhibitor wherein the immune checkpoint inhibitor is selected from one or more of a programmed cell death protein 1 (PD-1) inhibitor, programmed death-ligand 1 (PD-L1) inhibitor, or cytotoxic T-lymphocyte-associated antigen-4 (CTLA-4) inhibitor, and/or tyrosine kinase inhibitor (TKI).

11. The ALK peptide or the vaccine or immunogenic composition for use of claim 10, wherein the ALK-positive cancer is selected from the group consisting of non-small cell lung cancer (NSCLC), anaplastic large cell lymphoma (ALCL), neuroblastoma, B-cell lymphoma, thyroid cancer, colon cancer, breast cancer, inflammatory myofibroblastic tumors (IMT), renal carcinoma, esophageal cancer, and melanoma.

12. The ALK peptide or the vaccine or immunogenic composition for use of claim 10 or 11, wherein the PD-1 inhibitor is an anti-PD1 antibody and the ALK inhibitor is lorlatinib.

13. The ALK peptide or the vaccine or immunogenic composition for use of claim 10 or 11, wherein an adjuvant is concomitantly administered to the subject.

14. The ALK peptide or the vaccine or immunogenic composition for use of claim 10 or 11, wherein the subject had at least one prior treatment with at least one tyrosine kinase inhibitor (TKI).

15. A kit for use in treating a subject with one or more ALK-positive cancers, wherein said kit comprises an agent for administration to said subject, wherein the agent comprises the ALK peptide of any one of claims 1-6, or the vaccine or immunogenic composition of claim 8 or 9.
